# EUROPEAN PATENT APPLICATION

(11) **EP 4 417 616 A1**
(43) Date of publication of application: **21.08.2024**
(21) Application number: 23156662.1
(22) Date of filing: 14.02.2023
(51) Int. Cl.: C07J 9/00, C07J 31/00, A61P 5/44, A61P 35/00, A61P 37/04, A61K 31/575, C07J 53/00

(54) **LXR ANTAGONISTS**

(71) Applicant: Ospedale San Raffaele S.r.l., 20132 Milano (IT); Università degli Studi di Perugia, 06123 Perugia (IT)
(72) Inventor: Russo, Vincenzo, 20132 Milano (MI) (IT); Raccosta, Laura, 20132 Milano (MI) (IT); Marinozzi, Maura, 06123 Perugia (PG) (IT); Pacciarini, Manuela, 06123 Perugia (PG) (IT); Pontini, Lorenzo, 06123 Perugia (PG) (IT)
(74) Representative: Pace Napoleone, Maria

(57) **Abstract**

The present invention relates to novel antagonists of the Liver X Receptors (LXRs) of formula (I) which can be used alone or in combination with other anti cancer therapies, such as the immune checkpoint blockers or cell adoptive cell therapy, preferably T cell adoptive cell therapy, to treat different cancers, including melanoma, Hodgkin lymphoma, renal, lung, bladder and head and neck cancers. 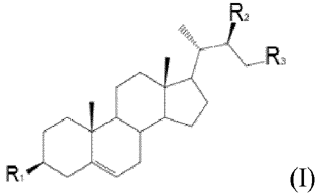 Wherein:
R₁ is OH or OSO₃X;
R₂ is OH or OSO₃X;
X at each occurrence is independently selected from H⁺, Na⁺, K⁺ , NH₄⁺ , pyridinium;
and wherein when R₁ is OSO₃X then R₃ is CH ₂CH(CH₃)₂, and
when R₁ is OH then R₃ is an aryl or heteroaryl ring each of said aryl or heteroaryl ring being optionally substituted with one or more substituents independently selected from C₁₋₆alkyl, C₁₋₆alkoxy, halogen, C₁₋₆haloalkyl, C₁₋₆haloalkoxy, C₃₋₆cycloalkyl, OH, halogen, NH₂, NH-C₁₋₆alkyl, N(C₁₋₆alkyl)2, NH-C(=O)C₁₋₃alkyl.

## Description

### FIELD OF THE INVENTION

The present invention relates to novel antagonists of the Liver X Receptors (LXRs) which can be used alone or in combination with other anti cancer therapies, such as the immune checkpoint blockers or cell adoptive cell therapy, preferably T cell adoptive cell therapy, to treat different cancers, including melanoma, Hodgkin lymphoma, renal, lung, bladder and head and neck cancers.

### BACKGROUND OF THE INVENTION

Immune checkpoint inhibitors (ICIs) counteracting inhibitory signals of T cell activation have revolutionized the antitumor treatments and improved the overall survival of patients affected by different tumor types¹. However, still many patients do not respond or progress after an initial benefit². ICI-resistant patients often display signatures of poor antigen presentation. Poor antigen presentation can be associated to the reduced density of dendritic cells (DCs) in the tumor microenvironment (TME)³, to the dampening of DC antigen presenting abilities⁴ or the inhibition of DC differentiation and maturation³. Restoring DC functions or recruiting alternative sources of antigen presenting cells remains crucial for non-responding patients with defects of antigen presenting cells. Many products from cholesterol and lipid metabolism exert immunosuppression by dampening the stimulatory functions of DCs⁵. Different mechanisms can be responsible for the accumulation of lipids in DCs, *i.e.* the up-regulation of receptors promoting lipid uptake or mechanisms involving the endoplasmic reticulum stress response via the transcription factor X-box-binding protein 1 (XBP1)⁶. Oxidized cholesterol metabolites, namely oxysterols, were shown to modulate DC functions by inhibiting the expression of the chemokine receptor CCR7, thus inhibiting their migration to draining lymphnodes and tumor immunity⁷. Oxysterols can be inactivated by the enzyme sulfotransferase 2B1b (SULT2B1b), which generates sulfated oxysterols⁸. SUTLT2B1b has been shown to impair LXRs signaling^{9,10} in response to dietary cholesterol¹¹, contribute to T cell proliferation after TCR engagement¹², generate intrinsic regulators of thymocyte development¹³, and restore antitumor immune responses when over-expressed by tumor cells, through the modulation of the migratory ability of tumor-infiltrating DCs⁷ and neutrophils¹⁴. Whether SULT2B1b and sulfated oxysterols regulate other cellular processes during tumor formation, such as myeloid cell differentiation remains unknown. Monocytes are circulating phagocytic mononuclear cells capable of differentiating into either tissue macrophages or in potent antigen presenting cells (*i.e.* monocyte-derived DCs)^{15,16}. In established tumors, classic Ly6C⁺ monocytes migrate to tumors in a C-C chemokine receptor-2 (CCR2)-dependent manner and mainly differentiate into tumor-associated macrophages, which contribute to tumor progression, metastasis formation and therapy resistance¹⁷. While this differentiation pathway is well established, very little is known on the route leading to the intratumor differentiation of Ly6C^{high} monocytes towards monocyte-DCs (Mono-DCs). Anthracycline-induced cell death was recently shown to promote the recruitment and differentiation/activation of Mono-DCs into the TME, and these cells cross-presented TAAs to CD8⁺ T cells¹⁸. Recently, the activation of the transcription factor p53 induced by inflammation, was shown to differentiate Ly6C⁺CD103⁺ monocytic antigen-presenting cells in tumors¹⁹. Whether other pathways, such as metabolic pathways, contribute to monocyte Mono-DC differentiation remains poorly known. WO2021133976 discloses a LXR antagonist, larsucosterol or DUR-928, a cholesterol derivative of formula: C₂₇H₄₆O₅S (CAS No. : 884905-07-1), developed by the company Durect corporation, currently in Phase 2 for Alcoholic Hepatitis and Phase 1 for Non-Alcoholic Steatohepatitis (NASH), Chronic Kidney Disease. WO2018/222975 discloses methods of treating cancer with a combination of an LXRβ agonist and GITR modulator. The disclosure also provides methods of treating cancer including combinations of LXRβ agonists and GITR modulators with additional immunotherapies such as PD1 inhibitors, PDL1 inhibitors, and adoptive T-cell transfer therapy. It describes also the compound RGX-104 (Formula: C34-H33-Cl-F3-N-O3), a LXRβ agonist currently tested in Phase 1/2 as antitumor agent. Recently specific subsets of oxysterols have been identified as endogenous ligands of LXRs and, given the action of LXRs (α and β isoforms) as whole-body cholesterol sensors and key regulators of lipogenesis, their potential to assume a key role in the modulation of lipid metabolism and glucose homeostasis has been further investigated. WO2011/077245 relates to the use of LXR ligand inactivators, or of LXR inhibitors/antagonists, for the treatment of cancer. WO2011/103175 relates to a group of synthetic oxysterols that are agonists or ligands of LXRs, and that can inhibit Hedghog (Hh) signaling. WO2019/021122 relates to novel steroidal stigmasterol and ergosterol derivatives, characterized by the presence of oxygenated functions at C-22 and C-23 positions, able to work as LXRs agonist for the treatment of diseases associated with LXRs, such as cancer, inflammation, metabolic and autoimmune diseases. For example, this patent application refers to compound PFM009 below as a potent and isoform unselective LXR agonist:

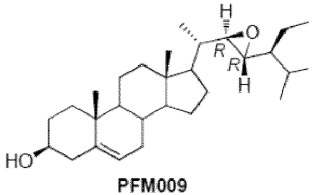

There is still the need of LXR antagonists to be used in therapy.

### SUMMARY OF THE INVENTION

The present invention provides novel oxysterol derivatives characterized by the following general formula (I) 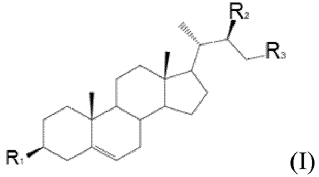 wherein:
R₁ is OH or OSO₃X;
R₂ is OH or OSO₃X;
X at each occurrence is independently selected from H⁺, Na⁺, K⁺, NH₄⁺ , pyridinium;
and wherein when R₁ is OSO₃X then R₃ is CH₂CH(CH₃)₂; and
when R₁ is OH then R₃ is an aryl or heteroaryl ring each of said aryl or heteroaryl ring being optionally substituted with one or more substituents independently selected from C₁₋₆alkyl, C₁₋₆alkoxy, halogen, C₁₋₆haloalkyl, C₁₋₆haloalkoxy, C₃₋₆cycloalkyl, OH, halogen, NH₂, NH-C₁-₆alkyl, N(C₁₋₆alkyl)₂, NH-C(=O)C₁₋₃alkyl.

Compounds of the invention are able to work as LXRs antagonists.

The present inventors found that SULT2B1b system manipulation favors the differentiation of Ly6C⁺ monocytes into Mono-DCs. This pathway is associated with lipidome reprogramming characterized by reduced levels of cellular cholesterol/cholesterol derivatives and glycerophospholipids, and increased levels of polyunsaturated fatty acid precursors of lipid mediators endowed with pro-inflammatory activity²⁰. This lipidomic profile was also detected in tumors from mice treated with the new synthetic compound PFM037, structurally and functionally related to SULT2B1b-derived products. PFM037 contributes to monocyte differentiation and induced effective antitumor responses by interfering with LXR signaling. Moreover, it increases the efficacy of ICIs and adoptive cell therapy. Finally, by interrogating available scRNA-seq datasets of tumor-infiltrating immune cells, the inventors herein report a significant enrichment of melanoma-infiltrating monocytes/macrophages over-expressing the LXR target genes *ABCA1* and *SCD1* in ICI-resistant patients. Similar results were obtained with the compound PFM046.

### DETAILED DESCRIPTION OF THE INVENTION

Lipid and cholesterol metabolism play a crucial role in tumor cell behavior and in shaping the TME. In particular, enzymatic and non-enzymatic cholesterol metabolism, and derived metabolites control DC functions, ultimately impacting tumor antigen presentation within and outside the tumor mass, dampening tumor immunity and immunotherapeutic attempts. The mechanisms accounting for such events remain largely to be defined. The present inventors perturbed (oxy)sterol metabolism genetically and pharmacologically and analyzed the tumor lipidome landscape in relation to the tumor-infiltrating immune cells. The inventors herein found that perturbing the lipidome of TME by the expression of SULT2B1b crucial in cholesterol and oxysterol sulfate synthesis, favores intratumoral representation of monocyte-derived antigen presenting cells, including Mono-DCs. They also found that treating mice with the newly developed antagonists of LXRs, promotes intratumoral Mono-DC differentiation, delayed tumor growth and synergized with anti-PD-1 immunotherapy and adoptive T cell therapy. Of note, looking at LXR/cholesterol gene signature in melanoma patients treated with anti-PD-1-based immunotherapy predicted diverse clinical outcomes. Indeed, patients whose tumors were poorly infiltrated by monocytes/macrophages expressing LXR target genes showed improved survival over the course of therapy. Thus, the data provided in the present invention support a role for (oxy)sterol metabolism in shaping monocyte-to-DC differentiation, and in tumor antigen presentation critical for responsiveness to immunotherapy. The identification of a new LXR antagonist opens new treatment avenues for cancer patients.

The LXR antagonists of the present invention can be used alone or in combination with the ICIs or with cell adoptive cell therapy, such as T cell adoptive cell therapy, to treat different cancers including melanoma, Hodgkin lymphoma, renal, lung, bladder and head and neck cancers. The compounds of the invention might be also used in combination with LXR agonists, preferably LXR agonists disclosed in WO2019/021122, particularly those activating LXRβ isoform, which are endowed with the capacity to decrease the intratumor number of myeloid-derived suppressor cells (Cell. 2018 Feb 8;172(4):825-840.e18. doi: 10.1016/j.cell.2017.12.026).

It is therefore an object of the invention a compound of formula (I) : 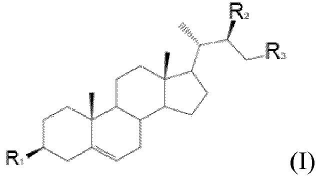 wherein:
R₁ is OH or OSO₃X;
R₂ is OH or OSO₃X;
X at each occurrence is independently selected from H⁺, Na⁺, K⁺, NH₄⁺ , pyridinium;
and wherein when R₁ is OSO₃X then R₃ is CH₂CH(CH₃)₂,
and when R₁ is OH then R₃ is an aryl or heteroaryl ring each of said aryl or heteroaryl ring being optionally substituted with one or more substituents independently selected from C₁-₆alkyl, C₁₋₆alkoxy, halogen, C₁₋₆haloalkyl, C₁₋₆haloalkoxy, C₃₋₆cycloalkyl, OH, halogen, NH₂, NH-C₁₋₆alkyl, N(C₁₋₆alkyl)₂, NH-C(=O)C₁₋₃alkyl.

Preferably the compound of formula (I) is selected from:

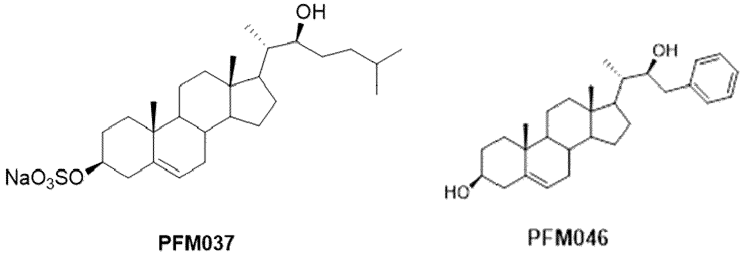

In a preferred embodiment the compound of formula (I) as defined above is for use as Liver X Receptor (LXR) antagonist; in a further preferred embodiment said compound of formula (I) is for medical use; preferably for use in the treatment and/or prevention of diseases linked or associated to LXR, preferably for use in the treatment and/or prevention of cancer, inflammatory and/or immunological diseases, preferably for use in the treatment of melanoma, skin carcinoma, Hodgkin lymphoma, renal, lung, bladder and head and neck cancers.

Still in a preferred embodiment said compound of formula (I) is for the treatment of the above defined diseases in combination with at least one further therapeutic agent, preferably said at least one further therapeutic agent is selected from the group consisting of LXR agonists, chemotherapeutics and/or in combination with immunotherapies, such as immune checkpoint inhibitors (ICIs), preferably PD1 inhibitors or PDL1 inhibitors and/or adoptive cell transfer therapy, such as adoptive T-cell transfer therapy or adoptive NK-cell transfer therapy. Preferably said LXR agonist is selected from the group comprising the following compounds:

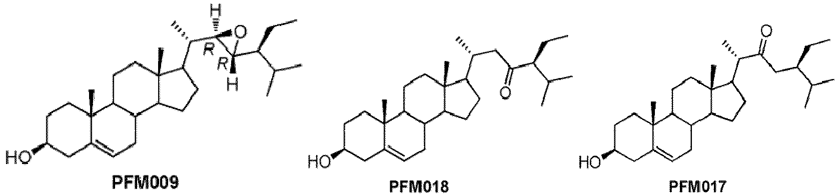

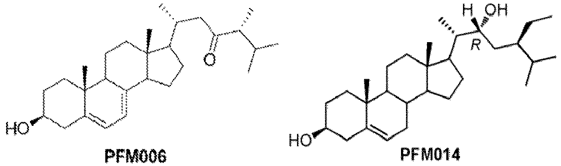

It is further object of the invention a pharmaceutical composition comprising a compound of formula (I) as defined above, or combinations thereof, alone or in combination with at least one further therapeutic agent, preferably said further therapeutic agent is selected from the group consisting of LXR agonists, chemotherapeutics, and/or in combination with immunotherapies, such as immune checkpoint inhibitors (ICIs), preferably PD1 inhibitors or PDL1 inhibitors, and/or such as adoptive cell transfer therapy, preferably adoptive T-cell transfer therapy or NK-cell transfer therapy, and at least a pharmaceutically acceptable excipient.

In a preferred embodiment said pharmaceutical composition further comprises an LXR agonist, preferably wherein said LXR agonist is selected from:

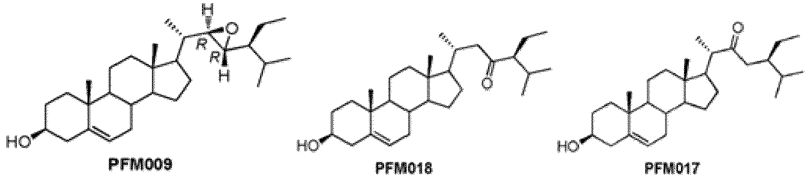

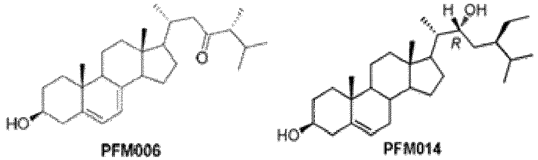

Preferably, said pharmaceutical composition is for medical use, preferably for use in the treatment and or prevention of diseases linked or associated to LXR, preferably for use in the treatment and/or prevention of cancer, inflammatory and/or immunological diseases, preferably for use in the treatment of melanoma, skin carcinoma, Hodgkin lymphoma, renal, lung, bladder and head and neck cancers.

The present invention also provides a method for obtaining a T cell or a T cell population comprising the steps of:
a) isolating a T cell or a T cell population from a biological sample of a subject;
b) activating said T cell or said T cell population, preferably by stimulating CD3 and CD28;
c) contacting and/or expanding said activated T cell or T cell population in the presence of a compound of formula (I) as herein defined to obtain a T cell or T cell population.

In step c) the T cell or T cell population is preferably contacted with IL-7 and/or IL-15, preferably with IL-7 and IL-15.

Preferably the obtained T cell or T cell population express at least one marker associated to T central memory and/or T stem cell memory.

It is a further object of the invention a method for obtaining a T cell or a T cell population expressing at least one marker associated to T central memory and/or T stem cell memory comprising the steps of:
a) isolating a T cell or a T cell population from a biological sample of a subject;
b) activating said T cell or said T cell population, preferably by stimulating CD3 and CD28;
c) contacting said activated T cell or T cell population with IL-7 and/or IL-15, preferably with IL-7 and IL-15, in the presence of a compound of formula (I) as herein defined to obtain a T cell or T cell population expressing at least one marker associated to T central memory and/or T stem cell memory.

Preferably in step b), the stimulation of CD3 and CD28 is carried out by a CD3 agonist and a CD28 agonist, preferably the stimulation is carried out by an antibody specific for CD3 and an antibody specific for CD28.

Still preferably the compound of formula (I) in step c) is PFM037 and/or PFM046.

It is a further object of the invention a T cell or T cell population obtainable by the method for obtaining a T cell or a T cell population expressing at least one marker associated to T central memory and/or T stem cell memory, comprising the steps above or herein defined.

It is a further object of the invention the use of a compound of formula (I) for augmenting T cell or NK cell memory differentiation *in vitro* and/or to decrease T cell or NK cell exhaustion *ex vivo* and/or to *in vitro or ex vivo* obtain a T or NK cell with a phenotype associated to T central memory and/or T stem cell memory (TSCM) and/or with antitumor potential, preferably for adoptive cell transfer purposes; preferably said compound is PFM037 and/or PFM046.

As used herein, the term "aryl" or "aromatic ring" means a monocyclic or polycyclic aromatic ring comprising carbon atoms and hydrogen atoms. If indicated, such aromatic ring may include one or more heteroatoms, then also referred to as "heteroaryl" or "heteroaromatic ring", preferably, 1 to 3 heteroatoms, independently selected from nitrogen, oxygen, and sulfur, preferably nitrogen. Preferably, the ring component of aryl or heteroaryl groups comprises 5 or 6 members (i.e. atoms). Still preferably, aryl or heteroaryl groups are polycyclic aromatic rings. Illustrative examples of aryl groups are optionally substituted phenyls. Illustrative examples of heteroaryl groups according to the invention include optionally substituted thiophene, oxazole, thiazole, thiadiazole, imidazole, pyrazole, pyrimidine, pyrazine, pyridine and pyridine N-oxide. An optionally substituted aryl or optionally substituted heteroaryl" generically refer to aryl or heteroaryl groups wherein the aromatic or heteroaromatic ring may be substituted with one or more substituents. Examples of said substituents include alkyl, alkoxy, amino, trifluoromethyl, aryl, heteroaryl, hydroxyl, carboxyalkyl and the like.

As used herein, LXR antagonists are compounds having antagonistic activity against LXRα and/or LXRβ activation. The LXR blockage results in an antitumor activity and in the increase in the anti-tumor immune response possibly due to the increased number and differentiation of tumor-infiltrating Mono-DCs, and/or increased number of tumor-infiltrating CD3+CD8+ T-cells. Therefore, the antitumor activity does not depend on a direct effect of the LXR antagonists on tumor cells, but on the modulation of the immune response.

As used herein, LXR activators or agonists are compounds able to induce the expression of target genes (i.e. ABCA1), which are involved in cholesterol homeostasis and fatty acids metabolism. Indeed, in the liver, LXR activation promotes the biosynthesis of fatty acids, by inducing the expression of SREBP-1c, as well as several downstream genes in the SREBP-1c pathway, including SCD1 and FASN.

Exemplary LXR agonists are disclosed in WO2019/021122, which is herein incorporated by reference. Said exemplary LXR agonists include compounds reported below:

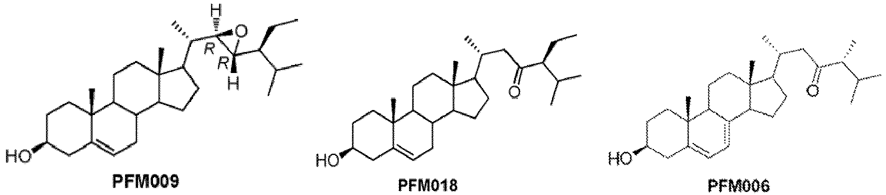

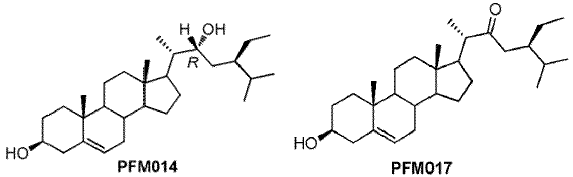

Preferably compounds of the invention are used in combination with LXR agonists, preferably said LXR agonists are compounds disclosed in WO2019/021122, more preferably are the specific compounds listed above.

Preferably, PFM037 is used in combination with PFM009 ((3β,22R,23R)-22,23-Epoxystigmast-5-en-3-ol).

In an embodiment of the invention, the T cell population isolated from the biological sample of a subject is a population of CD45RA+/CD62L+/CD95- T cells and CD45RA+/CD62L+/CD95+ T cells, as disclosed in WO2021219758 herein incorporated by reference.

Therefore, in the method of the invention step a) may consists of:
a) isolating a population of CD45RA+/CD62L+/CD95- T cells and CD45RA+/CD62L+/CD95+ T cells from a biological sample of a subject.

In a preferred embodiment the population of CD45RA⁺/CD62L⁺/CD95⁻ T cells and CD45RA⁺/CD62L⁺/CD95⁺ T cells comprise about 60 to 80 % of CD45RA⁺/CD62L⁺/CD95⁻ T cells and 40% to 20% of CD45RA⁺/CD62L⁺/CD95⁺ T cells.

Preferably the method further comprises a step d) of expanding the T cells or population of T cells in culture with IL-7 and IL-15 and in the presence of the compound(s) of the invention.

Preferably said produced or obtained T cell (or T cell population) has at least one of the following properties: expression of markers specific for T central memory and T stem cell memory, such as CD62L, CXCR3, Tcf1, CD122, Sca-1. Moreover, it proliferates much less than T-cell effectors, and are endowed with metabolic features based upon fatty acid oxidation and oxidative phosphorylation.

In a preferred embodiment said biological sample is: blood and other liquid samples of biological origin, solid tissue samples, tissue cultures of cells derived therefrom and the progeny thereof, isolated cells from biological samples as i.e. PBMCs, bone marrow, cord blood, iPSC-derived T cells.

Preferably the stimulation of CD3 and CD28 is carried out by a CD3 agonist and a CD28 agonist, preferably the stimulation is carried out by an antibody specific for CD3 and an antibody specific for CD28. Said antibodies being activating antibodies. The stimulation of CD3 and CD 28 may be performed according to any known method in the art for instance beads, matrix or cell-free matrix.

In the activation step, one or more cytokines, such as IL-2 or IL-27, and/or the peptide SIINFEKL, may be used.

Preferably, the activation of the T cell or T cell population is carried out with IL-2 and the peptide SIINFEKL or IL-27 and the peptide SIINFEKL.

Preferably, the activation of the T cell or T cell population is carried out by stimulating of CD3 and CD28, e.g. with anti-CD3/CD28 coated beads, and with IL-2.

Preferably the stimulated or activated population of T cells is contacted with IL-7 in an amount of 5-10 ng/ml.

Preferably the stimulated or activated population of T cells is contacted with IL-15 in an amount of 5-10 ng/ml.

In the method of the invention, preferably the compound of the invention is used in an amount of 5-10 µM.

In the contacting step the cells may also be expanded. The contacting step may be performed by contacting the cells with a medium comprising IL-7 and IL-15.

Preferably the stimulated or activated T cell or population of T cells is contacted for 10, 12 or 14 or 15 days with the compound(s) of the invention. Preferably during these days the cells are expanded in culture with IL-7 and IL-15. Preferably this step of expansion is carried out after the above step b) or c).

The above steps may also be sequential or performed in any order or performed at the same time. In a preferred embodiment the method further comprises introducing in said T cell or population of T cells a nucleic acid sequence encoding an exogenous gene, thereby producing an engineered T cell or T cell population. Preferably said introduction is performed before the expansion of the cells and/or after step c).

The term "contact", "incubate", "treat", "expose" may be used intercheangably.

Preferably, the marker associated to T central memory and/or T stem cell memory is CD62L, CD44, TCF 1 or CXCR3.

Preferably, the T cell or T cell population expressing at least one marker associated to T central memory and/or T stem cell memory has the following marker phenotype: CD39⁻CD69⁻CD62L⁺CD44^{+/-}TCF1⁺CXCR3⁺.

The present invention also provides a method for obtaining a Natural killer (NK) cell or a NK cell population comprising the steps of:
- isolating a NK cell or a NK cell population from a biological sample of a subject;
- contacting said NK cell or a NK cell population with a compound of formula (I) as defined herein.

In a preferred embodiment said method further comprises introducing in said NK cell or population of NK cells a nucleic acid sequence encoding an exogenous gene, thereby producing an engineered NK cell or NK cell population. Preferably said introduction is performed before the expansion of the cells.

In the present invention the T cell may also be a Natural killer T (NKT) cell.

In the context of the present invention, the expressions "adoptive cell transfer therapy" or "adoptive cell therapy" or "adoptive cell immunotherapy" may be used intercheangably. Adoptive cell transfer may be intended as the transfer of ex vivo grown cells, most commonly immune-derived cells, into a host with the goal of transferring the immunologic functionality and characteristics of the transplant. Adoptive cell transfer therapy can be autologous (e.g., isolated by leukapheresis, transduced and selected approximately 4 weeks immediately prior to administration), as is common in adoptive T-cell therapies, allogeneic, or xenogeneic. Adoptive cell transfer also comprise transfer of autologous tumor infiltrating lymphocytes (TILs), or of allogeneic lymphocytes isolated, prepared, and stored (e.g., frozen) "off-the-shelf from a healthy donor. In the context of the present invention, the adoptive cell therapy may comprise administration of cells expressing a chimeric antigen receptor (CAR), or a T-cell receptor (TCR), or may include tumor-infiltrating lymphocytes (TIL). The population of cells expressing the CAR/TCR, which recognize an antigen, may comprise a population of activated T-cells or natural killer (NK) cells or dendritic cells. Therefore the adoptive cell transfer therapy may include T-cell transfer therapy and/or NK-cell transfer therapy. The population of cells expressing the CAR/TCR may comprise a population of gene-edited cells.

The adoptive cell transfer therapy may use T-cells, natural killer (NK) cells, delta-gamma T-cells, regulatory T-cells, dendritic cells, and peripheral blood mononuclear cells. The adoptive cell therapy is preferably a CAR T-cell therapy or CAR NK-cell therapy. The CAR T-cell or CAR NK-cell can be engineered to target a tumour antigen of interest by way of engineering a desired antigen binding domain that specifically binds to an antigen on a tumour cell. According to certain aspects of the present invention, the antigen may be one that is expressed only on cancer cells or one that is preferentially expressed on cancer cells, such as a neo-antigen or lineage-specific antigen (such as CD 19 or CD20).

In the present invention, the population of cells, whether autologous or allogeneic, may be engineered using gene editing technology such as by CRISPR/cas9 (clustered regularly interspaced short palindromic repeats/ CRISPR associated protein 9), Zinc Finger Nucleases (ZFN), or transcription activator-like effector nuclease (TALEN). Accordingly, isolated autologous or allogeneic cells for adoptive transfer may be edited to delete or replace a known gene or sequence. For example, the T cell receptor (TCR) in an allogeneic T cell population may be deleted or replaced prior to or after CAR-T transduction as a means to eliminate graft-versus-host disease in recipient patients.

The population of cells administered as the adoptive cell transfer immunotherapy may express T-cell receptors (TCRs).

The adoptive cell transfer immunotherapy may comprise administration of regulatory T-cells. In the context of the present invention, preferably the exogenous gene encodes an antigen-recognizing receptor, an ortho-receptor, an immunomodulatory cytokine, a chemokine receptor, a dominant negative receptor (for instance PD1 DDR as disclosed in Cherkassky L JCI 2016, PMID: 27454297), a transcription factor able to prevent exhaustion (such as c-june as disclosed in Lynn Nature 2019, PMID: 31802004), preferably the antigen is a tumor antigen, a self-antigen or a pathogen antigen.

Preferably said antigen recognizing receptor is a T cell receptor (TCR) or a chimeric antigen receptor (CAR). Preferably it is CD 19, CD28, 41 bb. The expert in the art knows the antigens relevant for the disease herein mentioned to which the recombinant receptor as above defined may specifically bind.

In one embodiment, an endogenous gene encoding a TCR α chain and/or an endogenous gene encoding a TCR β chain in the cell is disrupted, preferably such that the endogenous gene encoding a TCR α chain and/or the endogenous gene encoding a TCR β chain is not expressed. In one embodiment, the endogenous gene encoding a TCR α chain and/or the endogenous gene encoding a TCR β chain is disrupted by insertion of an expression cassette comprising a polynucleotide sequence encoding a TCR of the present invention. In one embodiment, one or more endogenous genes encoding an MHC in the cell is disrupted, preferably wherein the cell is a non-alloreactive universal T-cell. In one embodiment, an endogenous gene involved in persistence, expansion, activity, resistance to exhaustion/senescence/inhibitory signals, homing capacity, or other T-cell functions in the cell is disrupted, preferably wherein the endogenous gene involved in persistence, expansion, activity, resistance to exhaustion/senescence/inhibitory signals, homing capacity, or other T-cell functions is selected from the group consisting of PD1, TIM3, LAG3, 2B4, KLRG1, TGFbR, CD160 and CTLA4. In one embodiment, the endogenous gene involved in persistence, expansion, activity, resistance to exhaustion/senescence/inhibitory signals, homing capacity, or other T-cell functions is disrupted by integration of an expression cassette, wherein the expression cassette comprises a polynucleotide sequence encoding a TCR of the present invention.

Preferably said antigen recognizing receptor is exogenous.

Preferably said nucleic acid sequence is introduced by a vector, preferably a lentiviral vector. A vector is a tool that allows or facilitates the transfer of an entity from one environment to another.

Preferably said nucleic acid sequence is placed at an endogenous gene locus of the T cell. Preferably said introduction of the nucleic acid sequence disrupts or abolishes the endogenous expression of a TCR.

The invention also provides a T cell or an engineered T cell produced or obtainable by the method of the invention. Preferably said produced or obtained T cell or an engineered T cell is isolated. The invention also provides a CAR T cell obtainable by the method of the invention or a TCR-engineered T cell obtainable by the method of the invention.

The invention also provides a NK cell or an engineered NK cell produced or obtainable by the method of the invention. Preferably said produced or obtained NK cell or an engineered NK cell is isolated. The invention also provides a CAR NK cell obtainable by the method of the invention.

In the present invention, preferably the exogenous gene encodes an antigen-recognizing receptor, an ortho-receptor, an immunomodulatory cytokine, a chemokine receptor, a dominant negative receptor, a transcription factor able to prevent exhaustion, preferably the antigen is a tumor antigen, a self-antigen or a pathogen antigen.

Another object of the invention is a pharmaceutical composition comprising at least one T cell or T cell population or the engineered T cell as defined above or the isolated engineered T cell population as defined above.

Preferably said T cell or population of T cells or engineered T cell or isolated engineered T cell population or a pharmaceutical composition comprising the same is for use in a therapy, preferably adoptive cell therapy and/or for use in the herein disclosed pathologies or conditions. More preferably the engineered T cell is CAR T cell.

Another object of the invention is a pharmaceutical composition comprising at least one NK cell or NK cell population or the engineered NK cell as defined above or the isolated engineered NK cell population as defined above.

Preferably said NK cell or population of NK cells or engineered NK cell or isolated engineered NK cell population or a pharmaceutical composition comprising the same is for use in a therapy, preferably adoptive cell therapy and/or for use in the herein disclosed pathologies or conditions. More preferably the engineered NK cell is CAR NK cell.

A variety of diseases may be ameliorated by introducing the cells of the invention to a subject suitable for adoptive cell therapy. Examples of diseases including various autoimmune disorders, including but not limited to, alopecia areata, autoimmune hemolytic anemia, autoimmune hepatitis, dermatomyositis, diabetes (type 1), some forms of juvenile idiopathic arthritis, glomerulonephritis, Graves' disease, Guillain-Barre syndrome, idiopathic thrombocytopenic purpura, myasthenia gravis, some forms of myocarditis, multiple sclerosis, pemphigus/pemphigoid, pernicious anemia, polyarteritis nodosa, polymyositis, primary biliary cirrhosis, psoriasis, rheumatoid arthritis, scleroderma/systemic sclerosis, Sjogren's syndrome, systemic lupus, erythematosus, some forms of thyroiditis, some forms of uveitis, vitiligo, granulomatosis with poly angiitis (Wegener's); hematological malignancies, including but not limited to, acute and chronic leukemias, lymphomas, multiple myeloma and myelodysplastic syndromes; solid tumors, including but not limited to, tumor of the brain, prostate, breast, lung, colon, uterus, skin, liver, bone, pancreas, ovary, testes, bladder, kidney, head, neck, stomach, cervix, rectum, larynx, or esophagus; and infections, including but not limited to, HIV- (human immunodeficiency virus), RSV- (Respiratory Syncytial Virus), EBV- (Epstein-Barr virus), CMV- (cytomegalovirus), HBV, HCV, adenovirus- and BK polyomavirus- associated disorders.

"Chimeric antigen receptor" or "CAR" or "CARs" refers to engineered receptors which can confer an antigen specificity onto cells (for example T cells). CARs are also known as artificial T-cell receptors, chimeric T-cell receptors or chimeric immunoreceptors. Preferably the CARs of the invention comprise an antigen-specific targeting region, an extracellular domain, a transmembrane domain, optionally one or more co-stimulatory domains, and an intracellular signaling domain. The antigen-specific targeting domain provides the CAR with the ability to bind to the target antigen of interest. The antigen-specific targeting domain preferably targets an antigen of clinical interest against which it would be desirable to trigger an effector immune response that results in tumor killing. The antigen-specific targeting domain may be any protein or peptide that possesses the ability to specifically recognize and bind to a biological molecule (e.g., a cell surface receptor or tumor protein, or a component thereof). The antigen-specific targeting domain includes any naturally occurring, synthetic, semi-synthetic, or recombinantly produced binding partner for a biological molecule of interest. Illustrative antigen-specific targeting domains include antibodies or antibody fragments or derivatives, extracellular domains of receptors, ligands for cell surface molecules/receptors, or receptor binding domains thereof, and tumor binding proteins. In a preferred embodiment, the antigen-specific targeting domain is, or is derived from, an antibody. Examples of antigens which may be targeted by the CAR of the invention include but are not limited to antigens expressed on cancer cells and antigens expressed on cells associated with various hematologic diseases, autoimmune diseases, inflammatory diseases and infectious diseases. With respect to targeting domains that target cancer antigens, the selection of the targeting domain will depend on the type of cancer to be treated, and may target tumor antigens. A tumor sample from a subject may be characterized for the presence of certain biomarkers or cell surface markers. For example, breast cancer cells from a subject may be positive or negative for each of Her2Neu, Estrogen receptor, and/or the Progesterone receptor. A tumor antigen or cell surface molecule is selected that is found on the individual subject's tumor cells. Preferably the antigen-specific targeting domain targets a cell surface molecule that is found on tumor cells and is not substantially found on normal tissues, or restricted in its expression to non-vital normal tissues. Further antigens specific for cancer which may be targeted by a CAR include but are not limited to any one or more of mesothelin, EGFRvIII, TSHR, CD19, CD123, CD22, CD30, CD171, CS-1, CLL-1, CD33, GD2, GD3, BCMA, Tn Ag, prostate specific membrane antigen (PSMA), ROR1, FLT3, FAP, TAG72, CD38, CD44v6, CEA, EPCAM, B7H3, KIT, IL-13Ra2, interleukin-11 receptor a (IL-11Ra), PSCA, PRSS21, VEGFR2, LewisY, CD24, platelet-derived growth factor receptor- beta (PDGFR-beta), SSEA-4, CD20, Folate receptor alpha (FRa), ERBB2 (Her2/neu), MUCl, epidermal growth factor receptor (EGFR), NCAM, Prostase, PAP, ELF2M, Ephrin B2, IGF-I receptor, CAIX, LMP2, gplOO, bcr-abl, tyrosinase, EphA2, Fucosyl GM1, sLe, GM3, TGS5, HMWMAA, o-acetyl-GD2, Folate receptor beta, TEM1/CD248, TEM7R, CLDN6, GPRC5D, CXORF61, CD97, CD 179a, ALK, Polysialic acid, PLAC1, GloboH, NY-BR-1, UPK2, HAVCR1, ADRB3, PANX3, GPR20, LY6K, OR51E2, TARP, WT1, NY-ESO-1, LAGE-la, MAGE-A1, legumain, HPV E6,E7, MAGE Al, ETV6-AML, sperm protein 17, XAGE1, Tie 2, MAD-CT-1, MAD-CT-2, Fos-related antigen 1, p53, p53 mutant, prostein, survivin and telomerase, PCTA-l/Galectin 8, MelanA/MARTl, Ras mutant, hTERT, sarcoma translocation breakpoints, ML-IAP, ERG (TMPRSS2 ETS fusion gene), NA17, PAX3, Androgen receptor, Cyclin B l, MYCN, RhoC, TRP-2, CYP1B 1, BORIS, SART3, PAX5, OY-TES 1, LCK, AKAP-4, SSX2, RAGE-1, human telomerase reverse transcriptase, RU1, RU2, intestinal carboxyl esterase, mut hsp70-2, CD79a, CD79b, CD72, LAIR1, FCAR, LILRA2, CD300LF, CLEC12A, BST2, EMR2, LY75, GPC3, FCRL5, and IGLL1. Antigens specific for inflammatory diseases which may be targeted by the CAR of the invention include but are not limited to any one or more of AOC3 (VAP-1), CAM-3001, CCL11 (eotaxin-1), CD125, CD147 (basigin), CD154 (CD40L), CD2, CD20, CD23 (IgE receptor), CD25 (a chain of IL-2 receptor), CD3, CD4, CD5, IFN-α, IFN-γ, IgE, IgE Fc region, IL-1, IL-12, IL-23, IL-13, IL-17, IL-17A, IL-22, IL-4, IL-5, IL-5, IL-6, IL-6 receptor, integrin α4, integrin α4β7, Lama glama, LFA-1 (CD11a), MEDI-528, myostatin, OX-40, rhuMAb β7, scleroscin, SOST, TGF β1, TNF-a or VEGF-A.

A T cell receptor (TCR) is a molecule which can be found on the surface of T-cells that is responsible for recognizing antigens bound to MHC molecules. The naturally-occurring TCR heterodimer consists of an alpha (α) and beta (β) chain in around 95% of T-cells, whereas around 5% of T-cells have TCRs consisting of gamma (γ) and delta (δ) chains.

An α chain of a TCR of the present invention may have a constant domain encoded by a TRAC gene. A β chain of a TCR of the present invention may have a constant domain encoded by a TRBC1 or a TRBC2 gene.

The present invention includes within its scope prodrugs of the compounds of formula (I). In general, such prodrugs will be functional derivatives of the compounds of formula (I), which are readily convertible *in vivo* into the required compound of formula (I). Conventional procedures for the selection and preparation of suitable prodrug derivatives are described, for example, in "Design of Prodrugs", ed. H. Bundgaard, Elsevier, 1985.

A prodrug may be a pharmacologically inactive derivative of a biologically active substance (the "parent drug" or "parent molecule") that requires transformation within the body in order to release the active drug, and that has improved delivery properties over the parent drug molecule. The transformation *in vivo* may be, for example, as the result of some metabolic process, such as chemical or enzymatic hydrolysis of a carboxylic, phosphoric or sulphate ester, or reduction or oxidation of a susceptible functionality.

The invention also includes all suitable isotopic variations of a compound of the disclosure. Examples of isotopes that can be incorporated into compounds of the disclosure include isotopes such as ²H, ³H, ¹³C, ¹⁴C, ¹⁵N, ¹⁷O, ¹⁸O, ³¹P, ³²P, ³⁵S, ¹⁸F and ³⁶Cl, respectively. Certain isotopic variations of the disclosure, for example, those in which a radioactive isotope such as ³H or ¹⁴C is incorporated, are useful in drug and/or substrate tissue distribution studies. Further, substitution with isotopes such as deuterium ²H, may afford certain therapeutic advantages resulting from greater metabolic stability. Isotopic variations of the compounds of the disclosure can generally be prepared by conventional procedures such as by the illustrative methods or by the preparations described in the examples hereafter using appropriate isotopic variations of suitable reagents.

The present invention includes within its scope solvates of the compounds of formula (I) or of the relative salts, for example, hydrates, alcoholates and the like.

Pure stereoisomeric forms of the compounds and intermediates of this invention may be obtained by the application of art-known procedures and are intended to be encompassed by the scope of the invention. In particular, "pure stereoisomeric form" or "stereoisomerically pure" indicate a compound having stereoisomeric excess of at least 80%, preferably of at least 85%. It is understood that substituents and substitution patterns on the compounds of the instant invention can be selected by one of ordinary skill in the art to provide compounds that are chemically stable and that can be readily synthesized by techniques known in the art, as well as those methods set forth below, from readily available starting materials. If a substituent is itself substituted with more than one group, it is understood that these multiple groups may be on the same carbon or on different carbons, so long as a stable structure results. The phrase "optionally substituted" should be taken to be equivalent to the phrase "unsubstituted or substituted with one or more substituents" and in such cases the preferred embodiment will have from zero to three substituents. More particularly, there are zero to two substituents.

As used herein, the term "aryl" means a monocyclic or polycyclic aromatic ring comprising carbon atoms and hydrogen atoms. If indicated, such aromatic ring may include one or more heteroatoms, then also referred to as "heteroaryl", preferably, 1 to 3 heteroatoms, independently selected from nitrogen, oxygen, and sulfur, preferably nitrogen. Illustrative examples of aryl groups are optionally substituted phenyl. Illustrative examples of heteroaryl groups according to the invention include optionally substituted thiophene, oxazole, 1,3,4-oxadiazole, 1,2,4-oxadiazole, thiazole, thiadiazole, imidazole, pyrazole, pyrimidine, pyrazine and pyridine.

The pharmaceutically acceptable salts of the instant compounds can be synthesized from the compounds of this invention which contain a basic or acidic moiety by conventional chemical methods. Generally, the salts of the basic compounds are prepared either by ion exchange chromatography or by reacting the free base with stoichiometric amounts or with an excess of the desired salt-forming inorganic or organic acid in a suitable solvent or various combinations of solvents. Similarly, the salts of the acidic compounds are formed by reactions with the appropriate inorganic or organic base. In a preferred embodiment, the compounds of the invention have at least one acidic proton and the corresponding sodium or potassium salt can be formed, for example, by reaction with the appropriate base.

Suitable "pharmaceutically acceptable salts" refers to salts prepared from pharmaceutically acceptable non-toxic bases including inorganic bases and organic bases. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, magnesium, potassium and sodium salts. Salts derived from pharmaceutically acceptable organic non-toxic bases include salts pyridine, salts of primary, secondary and tertiary amines, substituted amines including naturally occurring substituted amines, cyclic amines and basic ion exchange resins, such as arginine, betaine caffeine, choline, N,N¹-dibenzylethylenediamine, diethylamin, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine tripropylamine, tromethamine and the like.

The preparation of the pharmaceutically acceptable salts described above and other typical pharmaceutically acceptable salts is more fully described by Berg et al., "Pharmaceutical Salts," J. Pharm. Sci., 1977:66:1-19.

The compounds of the present invention find use in a variety of applications for human and animal health. The compounds of the present invention are inhibitors of LXR receptors useful for the treatment of diseases linked or associated to LXR. In the context of the present invention, diseases linked or associated to LXR are diseases as diverse as lipid disorders, atherosclerosis, chronic inflammation, autoimmunity, cancer and neurodegenerative diseases wherein LXRs are involved (for reviews see for example Trends Pharmacol Sci. 2012 Jul;33(7):394-404, doi: 10.1016/j.tips.2012.03.013; Nature Reviews Cancer volume 15, pages216-224 (2015), doi: 10.1038/nrc3912).

The compounds of this invention may be administered to mammals, preferably humans, either alone or in combination with pharmaceutically acceptable carriers, excipients or diluents, in a pharmaceutical composition, according to standard pharmaceutical practice. In one embodiment, the compounds of this invention may be administered to animals. The compounds can be administered orally or parenterally, including the intravenous, intramuscular, intraperitoneal, subcutaneous, rectal and topical routes of administration.

The invention also provides pharmaceutical compositions comprising one or more compounds of this invention and a pharmaceutically acceptable carrier. The pharmaceutical compositions containing the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, microcrystalline cellulose, sodium crosscarmellose, corn starch, or alginic acid; binding agents, for example starch, gelatin, polyvinyl-pyrrolidone or acacia, and lubricating agents, for example, magnesium stearate, stearic acid or talc. The tablets may be uncoated or they may be coated by known techniques to mask the unpleasant taste of the drug or delay disintegration and absorption in the gastrointestinal tract and thereby provide a sustained action over a longer period. For example, a water soluble taste masking material such as hydroxypropyl-methylcellulose or hydroxypropylcellulose, or a time delay material such as ethyl cellulose, cellulose acetate butyrate may be employed.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water soluble carrier such as polyethyleneglycol or an oil medium, for example peanut oil, liquid paraffin, or olive oil.

Aqueous suspensions contain the active material in admixture with excipients suitable for the manufacture of aqueous suspensions. Such excipients are suspending agents, for example sodium carboxymethylcellulose, methylcellulose, hydroxypropylmethyl-cellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl p-hydroxybenzoate, one or more coloring agents, one or more flavoring agents, and one or more sweetening agents, such as sucrose, saccharin or aspartame.

Oily suspensions may be formulated by suspending the active ingredient in a vegetable oil, for example arachis oil, olive oil, sesame oil or coconut oil, or in mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as butylated hydroxyanisol or alpha-tocopherol.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present. These compositions may be preserved by the addition of an anti-oxidant such as ascorbic acid.

The pharmaceutical compositions of the invention may also be in the form of an oil-in-water emulsions. The oily phase may be a vegetable oil, for example olive oil or arachis oil, or a mineral oil, for example liquid paraffin or mixtures of these. Suitable emulsifying agents may be naturally occurring phosphatides, for example soy bean lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening, flavoring agents, preservatives and antioxidants.

Syrups and elixirs may be formulated with sweetening agents, for example glycerol, propylene glycol, sorbitol or sucrose. Such formulations may also contain a demulcent, a preservative, flavoring and coloring agents and antioxidant.

The pharmaceutical compositions may be in the form of a sterile injectable aqueous solutions. Among the acceptable vehicles and solvents that may be employed are water, Ringer's solution and isotonic sodium chloride solution.

The sterile injectable preparation may also be a sterile injectable oil-in-water microemulsion where the active ingredient is dissolved in the oily phase. For example, the active ingredient may be first dissolved in a mixture of soybean oil and lecithin. The oil solution then introduced into a water and glycerol mixture and processed to form a microemulstion.

The injectable solutions or microemulsions may be introduced into a patient's blood stream by local bolus injection. Alternatively, it may be advantageous to administer the solution or microemulsion in such a way as to maintain a constant circulating concentration of the instant compound. In order to maintain such a constant concentration, a continuous intravenous delivery device may be utilized. An example of such a device is the Deltec CADD-PLUS^{™} model 5400 intravenous pump.

The pharmaceutical compositions may be in the form of a sterile injectable aqueous or oleagenous suspension for intramuscular and subcutaneous administration. This suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, for example as a solution in 1,3-butanediol. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Compounds of formula (I) may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials include cocoa butter, glycerinated gelatin, hydrogenated vegetable oils, mixtures of polyethylene glycols of various molecular weights and fatty acid esters of polyethylene glycol.

For topical use, creams, ointments, jellies, solutions or suspensions, etc., containing the compound of formula (I) are employed.

The compounds for the present invention can be administered in intranasal form via topical use of suitable intranasal vehicles and delivery devices, or via transdermal routes, using those forms of transdermal skin patches well known to those of ordinary skill in the art. To be administered in the form of a transdermal delivery system, the dosage administration will, of course, be continuous rather than intermittent throughout the dosage regimen. Compounds of the present invention may also be delivered as a suppository employing bases such as cocoa butter, glycerinated gelatin, hydrogenated vegetable oils, mixtures of polyethylene glycols of various molecular weights and fatty acid esters of polyethylene glycol.

The compounds of the invention may be presented in a liposome or other micro particulate or other nanoparticle designed to target the compound. Acceptable liposomes can be neutral, negatively, or positively charged, the charge being a function of the charge of the liposome components and pH of the liposome solution. Liposomes can be normally prepared using a mixture of phospholipids and cholesterol. Suitable phospholipids include phosphatidylcholine, phosphatidylethanolamine, phosphatidic acid, phosphotidylglycerol, phosphatidylinositol. Polyethylene glycol can be added to improve the blood circulation time of liposomes. Acceptable nanoparticles include albumin nanoparticles and gold nanoparticles.

When a compound according to this invention is administered into a human subject, the daily dosage will normally be determined by the prescribing physician with the dosage generally varying according to the age, weight, sex and response of the individual patient, as well as the severity of the patient's symptoms.

In one exemplary application, a suitable amount of compound is administered to a mammal. Administration generally occurs in an amount between about: 0.01 mg/kg of body weight to about 100 mg/kg of body weight per day, preferably between about 0.01 mg/kg of body weight to about 60 mg/kg of body weight per day, preferably between about 0.1 mg/kg of body weight to about 50 mg/kg of body weight per day, preferably between about 0.5 mg/kg of body weight to about 40 mg/kg of body weight per day.

The instant compounds are also useful in combination with known therapeutic agents for simultaneous, separate or sequential administration.

In an embodiment, the compounds of the present invention may be used in combination with at least one or more additional therapeutic agents, preferably said at least one further therapeutic agent is selected from the group consisting of LXR agonists, chemotherapeutics, or the compounds of the present invention may be used in combination with immunotherapies, such as immune checkpoint inhibitors (ICIs), preferably PD1 inhibitors or PDL1 inhibitors, or such as adoptive cell transfer therapy, such as adoptive T-cell transfer therapy or NK-cell transfer therapy. Preferably the ICI is an antibody, such as anti-PD1-mAb.

The present invention will be described by means of the following non-limiting examples and biological data are presented.

### Brief description of the figures

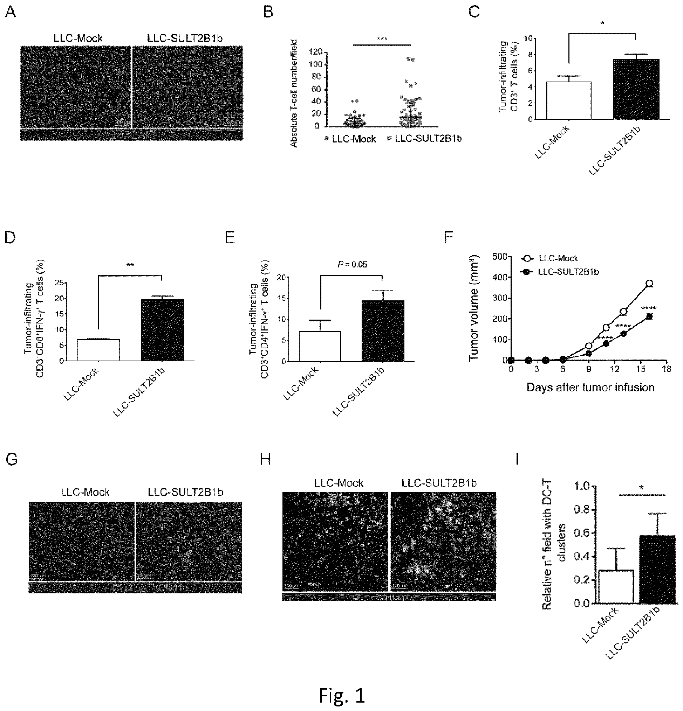

**Figure 1****. Analysis of the LLC tumor microenvironment under SULT2B1b perturbation.** (A) One representative confocal microscopy imaging of CD3⁺ T cells infiltrating LLC-Mock and LLC-SULT2B1b tumors. Red, CD3⁺ T cells; Blue, DAPI (bars = 200 µm). **(B)** Absolute T cell number/field evaluated by counting 4 different experiments with FIJI software (extension of ImageJ) and classical cell counter. DAPI⁺ events (blue) displaying CD3 staining (red) were considered as positive T cells. ^{∗∗∗}*P* < 0.001 (Student's t-test). **(C)** Flow cytometry analysis of the percentage of CD45⁺CD3⁺ T cells infiltrating LLC-Mock and LLC-SLTLT2B1b tumors. ^{∗}*P* < 0.05 (Student's t-test). Mean and s.d. of four experiments. **(D)** Flow cytometry analysis of the percentage of CD3⁺CD8⁺INF-γ⁺ T cells infiltrating LLC-Mock and LLC-SULT2B1b tumors. ^{∗∗}*P* < 0.01 (Student's t-test). Mean and s.d. of two experiments. **(E)** Flow cytometry analysis of the percentage of CD3⁺CD4⁺INF-γ⁺ T cells infiltrating LLC-Mock and LLC-SULT2B1b tumors. *P* = 0.01 (Student's t-test). Mean and s.d. of two experiments. **(F)** LLC-Mock and LLC-SULT2B1b tumor growth. Mean and s.d. of one experiment (*n* = 10 mice/group). ^{∗∗∗∗}*P* < 0.0001 (Student's t-test). **(G** and **H)** Representative confocal microscopy imaging of CD3⁺ T cells and CD11c⁺ cells **(G)** and CD3⁺ T cells, CD11b⁺ and CD11c⁺ cells **(H)** infiltrating LLC-Mock and LLC-SULT2B1b tumors. Red, CD3⁺ T cells; Blue, DAPI, Green, CD11c; Gray, CD11b (bars = 200 µm). (I) Quantification of the fields of DC/T cell clusters evaluated by counting 4 different experiments with FIJI software (extension of ImageJ) and classical cell counter. The number of sections in each image displaying the presence of both DCs (CD11c⁺CD11b⁺) and T cells (CD3⁺) was considered as a positive DC/T cell cluster. ^{∗}*P* < 0.05 (Student's t-test).

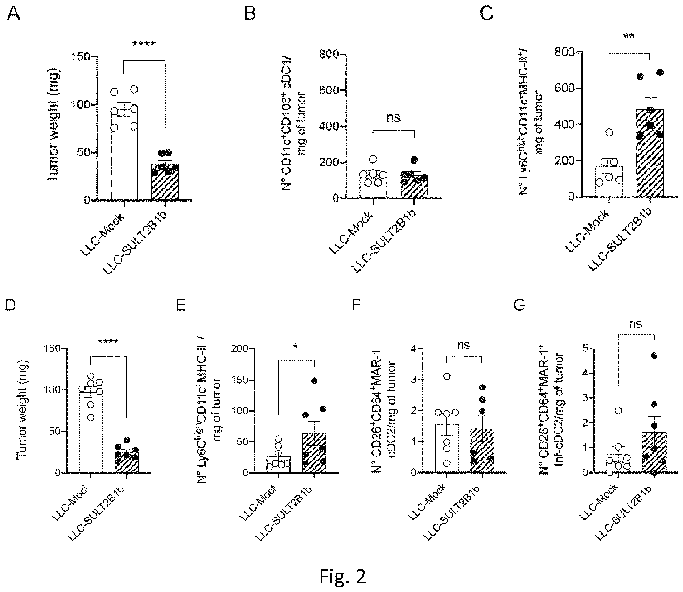

**Figure 2****. Analysis of the DC subsets infiltrating LLC tumor microenvironment under SULT2B1b perturbation. (A)** Mice challenged with LLC-Mock and LLC-SULT2B1b tumors were sacrificed 12 days after tumor challenge and evaluated for tumor weight. Mean and s.d. of 6 mice/group. ^{∗∗∗∗}*P* < 0.0001 (Student's t-test). The same tumors were analyzed for the number of CD11c⁺CD103⁺ cDC1 **(B)** and monocyte-DCs/mg of tumors **(C).** Mean and s.d. of 6 mice/group. ^{∗∗}*P* < 0.01; ns, not significant (Student's t-test). **(D)** Mice challenged with LLC-Mock and LLC-SULT2B1b tumors were sacrificed 10 days after tumor challenge and evaluated for tumor weight. Mean and s.d. of 7 mice/group. ^{∗∗∗∗}*P* < 0.0001 (Student's t-test). **(E-G)** Numbers of monocyte-DCs **(E)** CD26⁺CD64⁺MAR-1⁻ cDC2 **(F)** and CD26⁺CD64⁺MAR-1⁺ Inf-cDC2/mg of tumors **(G).** Mean and s.d. of 6-7 mice/group. ^{∗}*P* < 0.05 (Student's t-test).

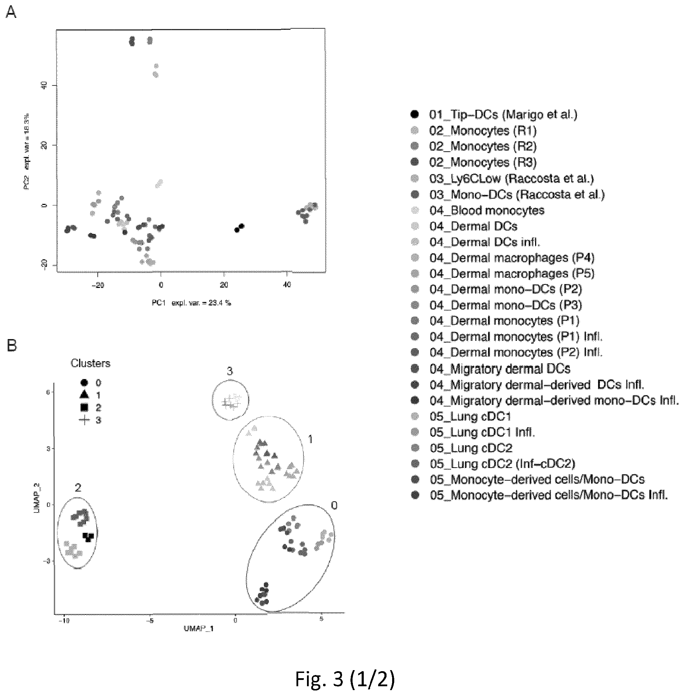

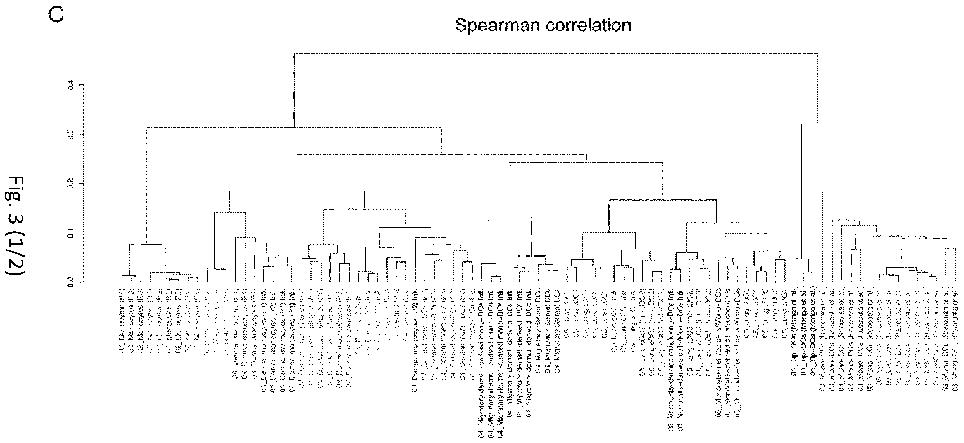

**Figure 3****. Transcriptomic features of monocyte-derived cells under SULT2B1b perturbation. (A)** Principal Component Analysis (PCA) of the transcriptomes of mono-DCs and Ly6C^{low} cells isolated from LLC-Mock and LLC-SULT2B1b tumors, integrated with 4 datasets present in the GEO (Gene Expression Omnibus) Database. PCA based on the expression values of 10080 genes detected in all the five experimental datasets. The first two principal components are shown (PC1 accounted for 23.4 % and PC2 for 18.3% of the variance among samples). **(B)** Uniform Manifold Approximation and Projection analysis (UMAP) of the transcriptomes as reported in (A). The figure shows bidimensional UMAP representation of the integrated dataset. Four different clusters have been identified using graph-based techniques. **(C)** Hierarchical clustering analysis. Hierarchical clustering of the expression values of 10080 genes detected with average linkage was performed on the specified subsets to assess their transcriptional relatedness. Spearman correlation-based distance was used.

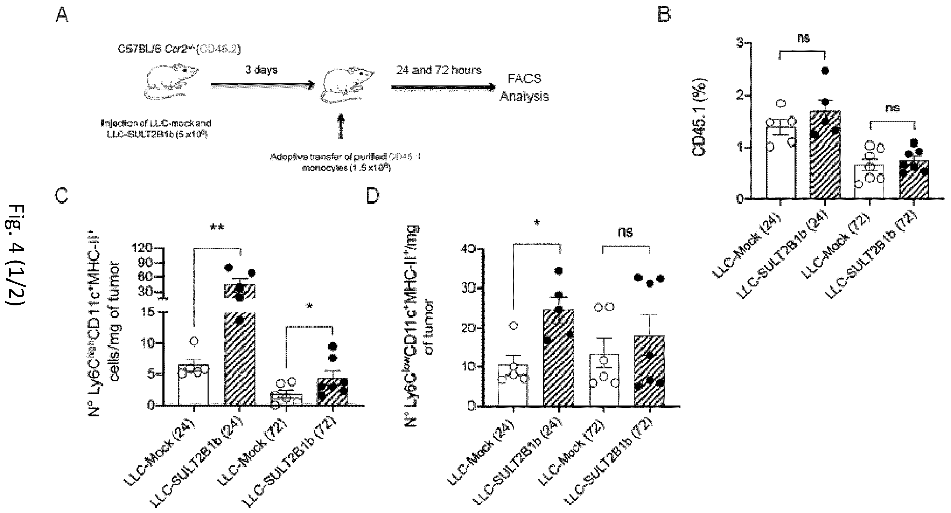

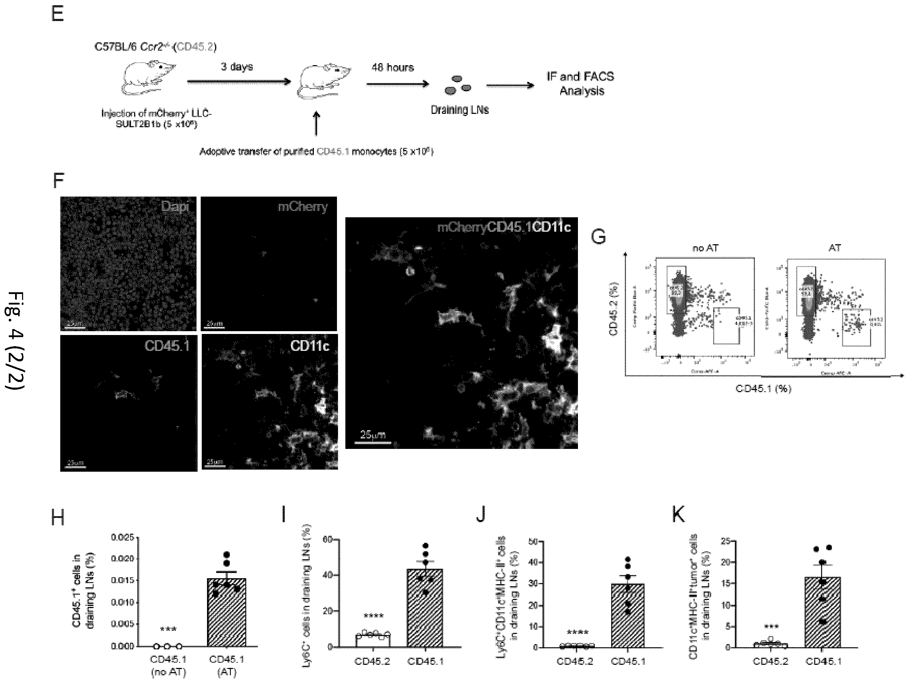

**Figure 4****. SULT2B1b expression promotes intratumor differentiation of monocyte-DCs migrating to draining lymph nodes. (A)** Schematic for assessing intratumor monocyte-DC differentiation. CD45.2⁺*Ccr2*^{-/-} mice bearing LLC-Mock and LLC-SULT2B1b tumors were administered with CD45. 1⁺bone-marrow purified monocytes (Ly6C⁺). Twenty-four hours later tumors were collected and analyzed by flow cytometry. **(B)** Percentage of CD45.1⁺ cells infiltrating LLC-Mock and LLC-SULT2B1b tumors 24 and 72 hours after the adoptive transfer of pure CD45.1⁺Ly6C⁺ monocytes. Mean and s.d. of 5 (24 hrs) and 7 (72 hrs) mice/group. No difference was observed between the two groups. ns, not significant. **(C-D)** Numbers of mono-DCs (C) and Ly6C^{low}CD11c⁺MHC-II⁺ cells **(D)** infiltrating LLC-Mock and LLC-SULT2B1b tumors 24 and 72 hours after the adoptive transfer of pure CD45.1⁺Ly6C⁺ monocytes. Mean and s.d. of 5 (24 hrs) and 6-7 (72 hrs) mice/group. ns, not significant; ^{∗}*P* < 0.05; ^{∗∗}*P* < 0.01 (Student's t-test). **(E)** Schematic for assessing tumor-engulfed monocyte-DCs in the draining lymph nodes. CD45.2⁺*Ccr2*^{*-*/*-*} mice bearing LLC-SULT2B1b tumors expressing mCherry were administered with CD45.1⁺bone-marrow purified monocytes (Ly6C⁺). Forty-eight hours later draining lymph nodes were collected and analyzed by flow cytometry. As control, draining lymph nodes were collected from not adoptively transferred tumor-bearing mice. **(F)** Representative confocal microscopy imaging of LLC-SULT2B1b tumors expressing mCherry⁺, CD45.1⁺, CD11c⁺ cells in the draining lymph nodes. Red, mCherry⁺; Blue, DAPI, Green, CD45.1; Gray, CD11c (bars = 25 µm). **(G)** Representative flow cytometry image of CD45.1⁺ and CD45.2⁺ cells in the draining lymph nodes of tumor-bearing mice. **(H)** Quantification of CD45.1⁺ cells in the draining lymph nodes of tumor-bearing mice with or without adoptive transfer of CD45.1⁺ monocytes. Mean and s.d. of 3 (no AT) and 6 (AT) mice/group. ^{∗∗∗}*P* < 0.001 (Student's t-test). **(I-K)** Quantification of CD45.1⁺Ly6C⁺ or CD45.2⁺Ly6C⁺ cells **(I),** Ly6C⁺CD11c⁺MHC-II⁺ cells **(J)** and CD11c⁺MHC-II⁺mCherry⁺ cells **(K)** in the draining lymph nodes of tumor-bearing mice adoptively transferred with bone marrow-derived CD45.1⁺ monocytes. The CD1 1c⁺MHC-II⁺mCherry⁺ cells are tumor-engulfed mono-DCs migrated to draining lymph nodes. Mean and s.d. of 6 mice/group. ^{∗∗∗}*P* < 0.001; ^{∗∗∗∗}*P* < 0.0001 (Student's t-test).

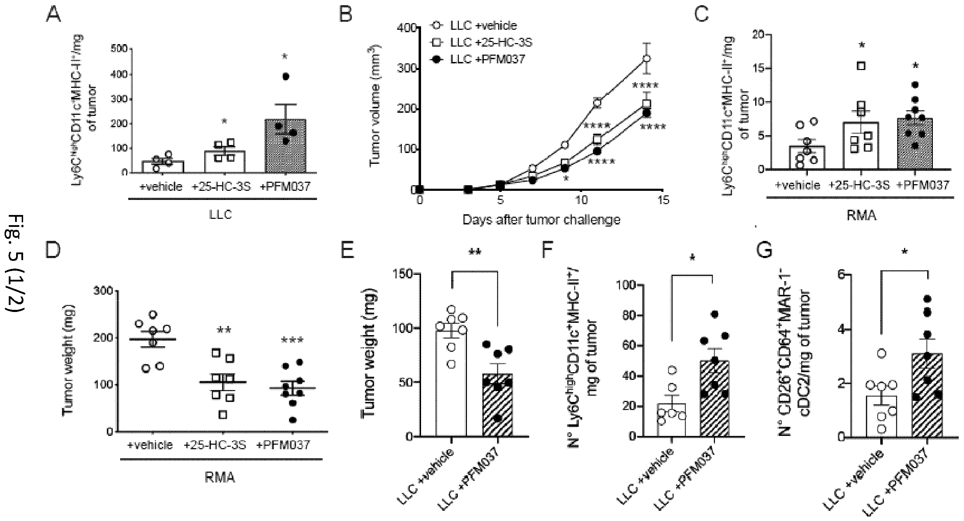

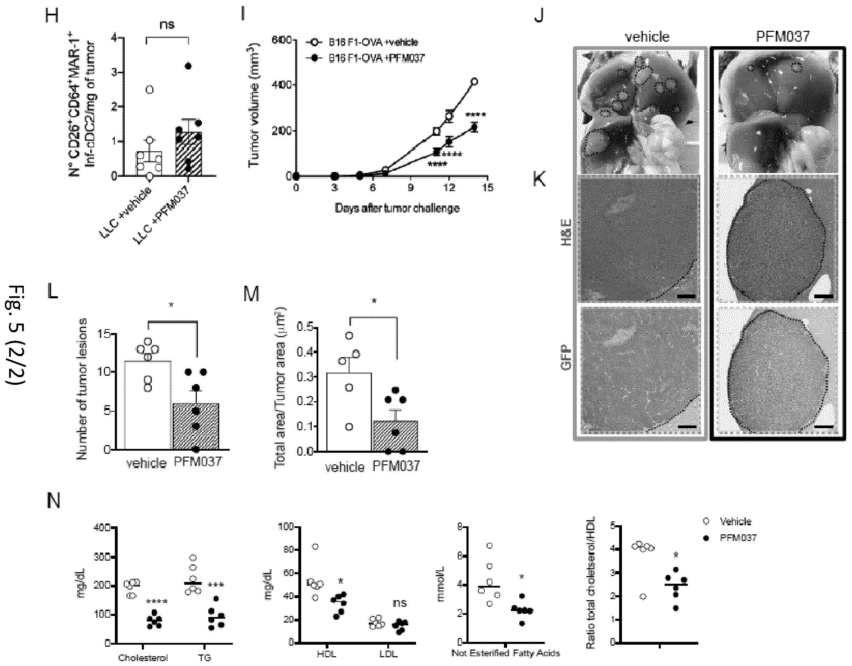

**Figure 5****. Sulfated oxysterols promote monocyte-DC differentiation and antitumor activity. (A)** Mice bearing established LLC tumors were treated with the sulfated oxysterols 25-HC-3S, PFM037 or vehicle. At sacrifice, absolute numbers of Ly6C^{high}CD11c⁺MHC-II⁺ monocyte-DCs/mg of LLC tumors, were evaluated. Mean and s.d. of 4 mice/group. ^{∗}*P* < 0.05 (Anova). **(B)** LLC-bearing mice were treated with the sulfated oxysterols 25-HC-3S, PFM037 or vehicle and monitored for tumor growth. Mean and s.d. of one experiment (n = 6 mice/group). ^{∗}*P* < 0.05; ^{∗∗∗∗}*P* < 0.0001 (Anova). **(C)** Mice bearing established RMA tumors were treated with the sulfated oxysterol 25-HC-3S, PFM037 or vehicle. At sacrifice, absolute numbers of Ly6C^{high}CD11c⁺MHC-II⁺ monocyte-DCs/mg of RMA tumors, were evaluated. Mean and s.d. of 7-8 mice/group. ^{∗}*P* < 0.05 (Anova). **(D)** RMA-bearing mice were treated with the sulfated oxysterols 25-HC-3S, PFM037 or vehicle and evaluated for tumor weight at mice sacrifice. Mean and s.d. of 7-8 mice/group. ^{∗∗}*P* < 0.01; ^{∗∗∗}*P* < 0.001 (Anova). **(E-H)** Tumor weights **(E)** and numbers of mono-DCs **(F),** cDC2 identified by the expression of CD26 and CD64 markers **(G),** and Inf-DC2 identified by the expression of CD26, CD64 and MAR-1 markers **(H)** infiltraing tumors from mice treated with vehicle or PFM037. Mean and s.d. of 6-7 mice/group. ns, not significant; ^{∗}*P* < 0.05; ^{∗∗}*P* < 0.01 (Student's t-test). The difference of the numbers of mono-DCs between the experiments reported in **A** and **F** is due to the different days of analysis after tumor challenge and treatment, 12 and 10 days in A and F, respectively. (I) Melanoma B16F1-OVA growth delay following the treatment with PFM037. B16F1-OVA-bearing mice were treated with vehicle or with PFM037. Mean and s.d. of one experiment (n = 8 mice/group). ^{∗∗}*P* < 0.01; ^{∗∗∗}*P* < 0.001; ^{∗∗∗∗}*P* < 0.0001 (Student's t-test). **(J)** Representative images of liver metastases 21 days after injection of CRC in mice treated with vehicle (gray frame) or PFM037 (black frame). The black dashed line indicates tumor lesions. **(K)** Representative H&E and GFP immunohistochemical micrographs showing liver tumor features of metastases found in vehicle- (gray frame) or PFM037-treated (black frame) mice 21 days after intramesenteric injection of MC38-GFP cells. Scale bar = 300µm. **(L, M)** Quantification of the number of macroscopic liver tumor lesions **(L),** as well as the percentage of tumor area **(M)** determined by IHC in the mice described in **J-K.** Mean values ± s.d. are shown; ^{∗}*P* < 0.05 (Mann-Whitney test). (N) Plasma levels of total cholesterol, triglycerides (TG), high and low-density lipoproteins (HDL, LDL), and high-density lipoproteins (HDL), Not Esterified Fatty Acids, and in vehicle and ratio between total cholesterol and HDL in vehicle and PFM037 (0.4 µg/day)-treated mice. ^{∗}*P* < 0.05; ^{∗∗∗}*P* < 0.001; ^{∗∗∗∗}*P* < 0.0001 (Anova).

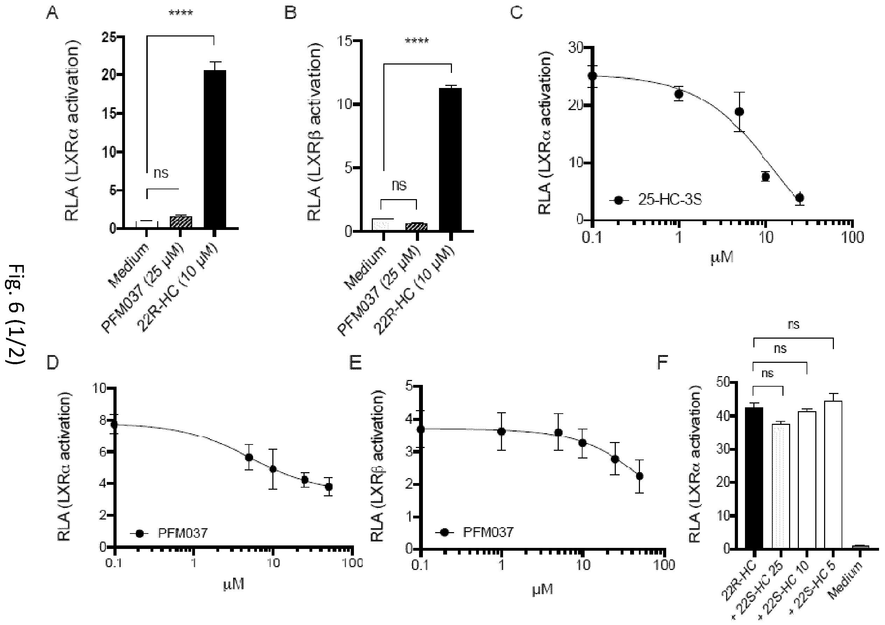

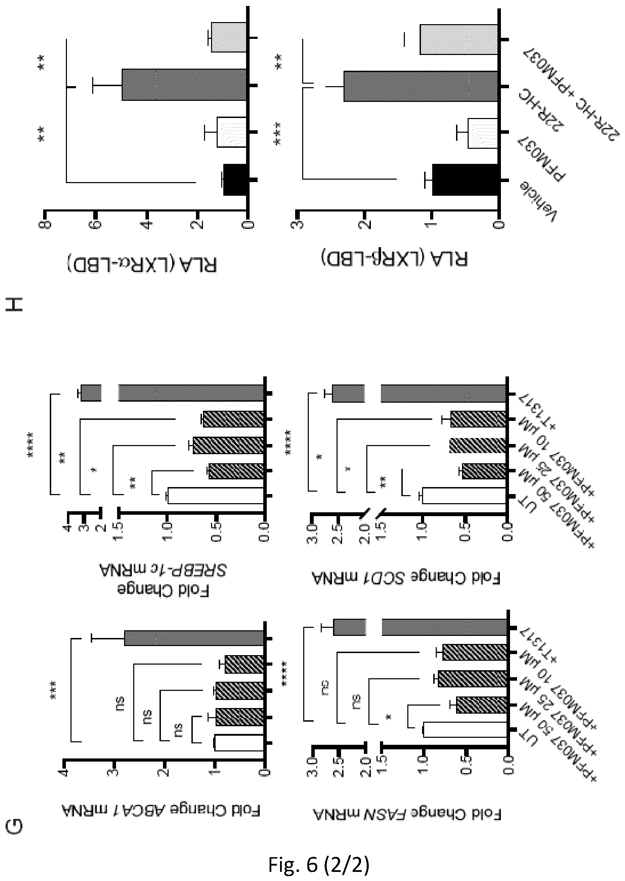

**Figure 6****. PFM037 interferes with LXR activity. (A-B)** Analysis of luciferase-based LXRα **(A)** and LXRβ **(B)** activation induced by PFM037, or 22R-HC used as control. Mean and s.d. of three independent experiments. ^{∗∗∗∗}*P* < 0.0001 (Anova). **(C-D)** Luciferase-based LXRα assays displaying the antagonistic activity of 25-HC-3S **(C)** and PFM037 **(D).** Mean and s.d. of 2 independent experiments. LXRα activation was induced by 5 **(C)** or 10 µM **(D)** of 22R-HC, 25-HC-3S was used at 25, 10, 5 and 1 µM, whereas PFM037 at 50, 25, 10, 5 and 1 µM. RLA, Relative Luciferase Activity. **(E)** Luciferase-based LXRβ assays displaying the antagonistic activity of PFM037. Mean and s.d. of 2 experiments. LXRβ activation was induced by 10 µM of 22R-HC, PFM037 was used at 50, 25, 10, 5 and 1 µM. RLA, Relative Luciferase Activity. **(F)** Luciferase-based LXRα assays to test the antagonistic activity of the inactive oxysterol 22S-HC. 22S-HC does not antagonize LXR activation induced by 5 µM of 22R-HC. 22S-HC was used at 25, 10, 5 and 1 µM. RLA, Relative Luciferase Activity. **(G)** Analysis of the expression of the LXR target genes ABCA1, SREBP1c, FASN and SCD1 following T0901317 (T1317; CAS Registry Number: 293754-55-9) or PFM037 treatment. Mean and s.d. of three independent experiments. ^{∗}*P* < 0.05; ^{∗∗}*P* < 0.01; ^{∗∗∗}*P* < 0.001; ^{∗∗∗∗}*P* < 0.0001 (Anova). **(H)** Mammalian two-hybrid assay in Huh7 cells using Gal4-DBD-tagged RAP250-Del4 (containing NR-box1), together with pVP16-LXRα-LBD or pVP16-LXRβ-LBD, and the reporter plasmid pUAS-tk-Luc. Values shown are the mean and s.d. of two experiments performed in triplicate with luciferase activity normalized to protein content and with the activity of respective pVP16-fusion without ligand set to 1.0. The ligands 22R-HC and PFM037 was used at 10 µM when indicated. ^{∗∗}*P* < 0.01; ^{∗∗∗}*P* < 0.001 (Anova).

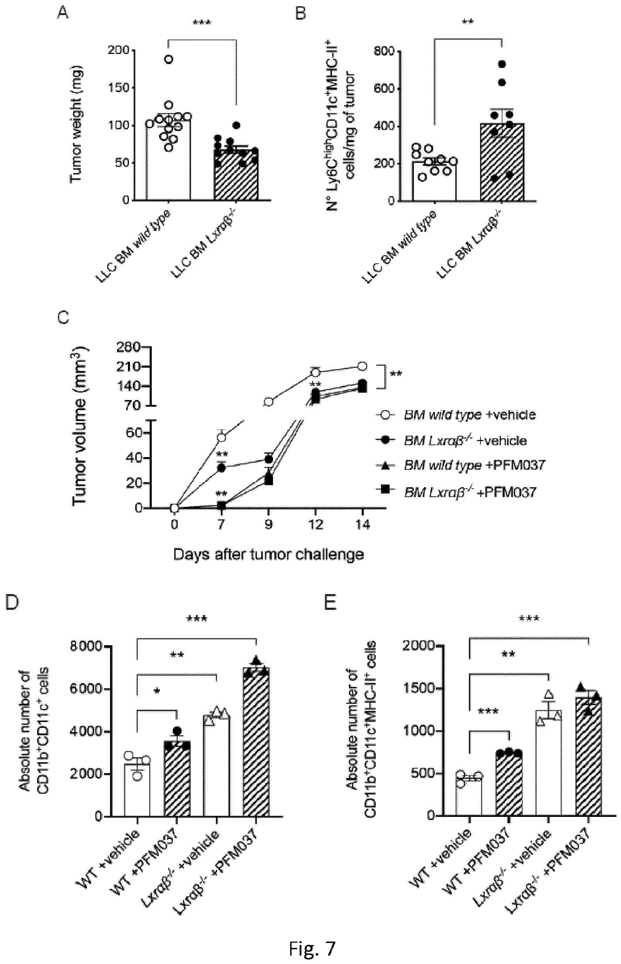

**Figure 7****. LXR signaling contributes to *in vitro* and *in vivo* PFM037-induced monocyte-to-DC differentiation. (A** and **B)** Tumor weight **(A)** and numbers **(B)** of Ly6C^{high}CD11c⁺MHC-II⁺ mono-DCs/mg of LLC-Mock tumors grown in *Lxrαβ*^{*-*/*-*} or wild type bone-marrow chimera mice. Mean and s.d. of 2 experiments (*n* = 11-12 mice/group). ^{∗∗∗}*P* < 0.001 (Student's t-test) for tumor weight. Mean and s.d. of 2 experiments (*n* = 8 mice/group). ***P* < 0.01 (Student's t-test) for Ly6C^{high}CD11c⁺MHC-II⁺ mono-DC content. **(C)** LLC tumor growth in in *Lxrαβ*^{*-*/*-*} or wild type bone-marrow chimera mice treated with vehicle or PFM037. Mean and s.d. of one experiment (*n* = 5-7 mice/group). ^{∗∗}*P* < 0.01 (Student's t-test). **(D** and **E)** Absolute number of *wild type* or *Lxrαβ*^{*-*/*-*} monocytes expressing CD11b⁺CD11c⁺ **(D)** and CD11b⁺CD11c⁺MHC-II⁺ **(E)** after 48 hours of culture in the presence of vehicle or PFM037. Mean and s.d. of 3 independent experiments. ^{∗}*P* < 0.05; ^{∗∗}*P* < 0.01; ^{∗∗∗}*P* < 0.001 (Anova).

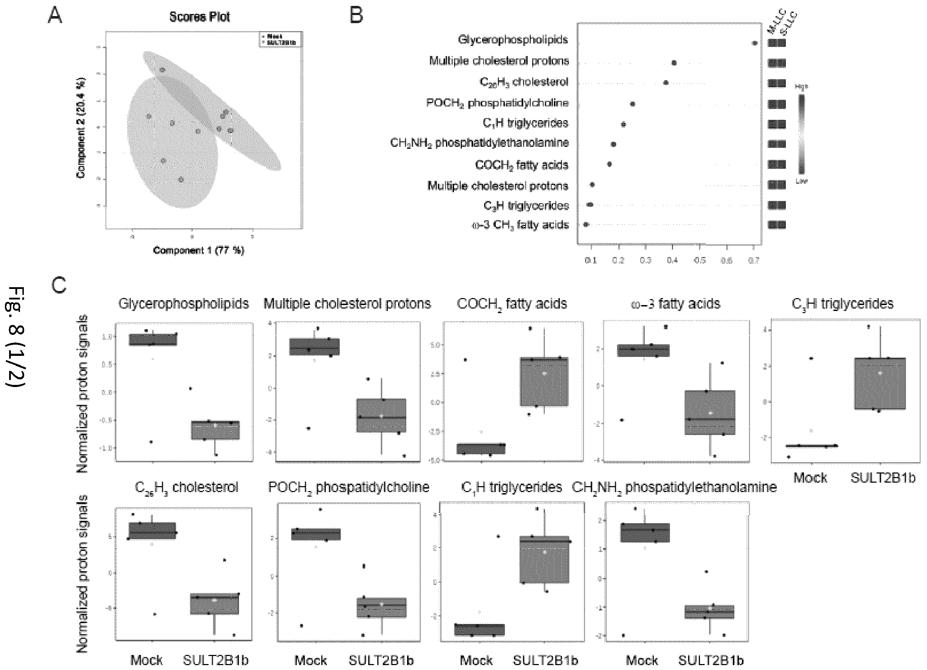

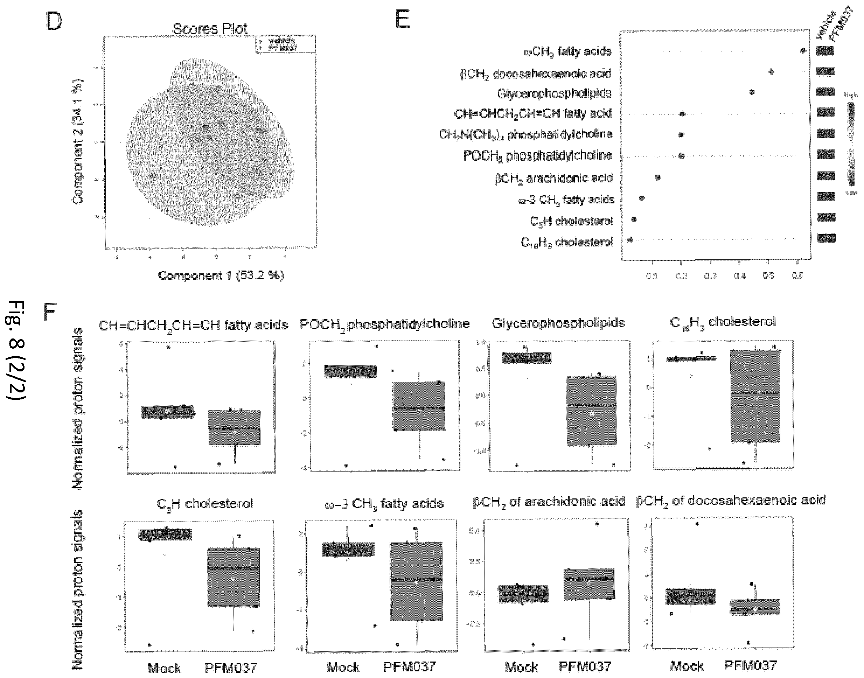

**Figure 8****. Lipidomic landscape of LLC tumors under SULT2B1b or PFM037 perturbation. (A)** Score plot by partial least squares-discriminant analysis (PLS-DA) related to the lipidic fraction of Mock and SULT2B1b-LLC tumors (*n* = 5 tumors/group). **(B)** Variable importance in projection (VIP) plot showing the top 10 NMR signals resulted more statistically different in the lipidic fractions of Mock- and SULT2B1b-LLC tumors in C57Bl/6 mice (n = 5 tumors/group). **(C)** Box plots showing the levels of lipid species, evaluated as normalized proton signals, differentially expressed by Mock- and SLTLT2B1b-LLC tumors (*n* = 5 tumors/group). **(D)** Score plot by partial least squares-discriminant analysis (PLS-DA) related to the lipidic fraction of vehicle- and PFM037-treated-LLC tumors (*n* = 5 tumors/group). **(E)** Variable importance in projection (VIP) plot showing the top 10 NMR signals resulted more statistically different in the lipidic fractions of vehicle- and PFM037-treated-LLC tumors in C57Bl/6 mice (n = 5 tumors/group). **(F)** Box plots showing the levels of lipid species, evaluated as normalized proton signals, differentially expressed by LLC tumors from mice treated with vehicle of PFM037 (*n* = 5 tumors/group).

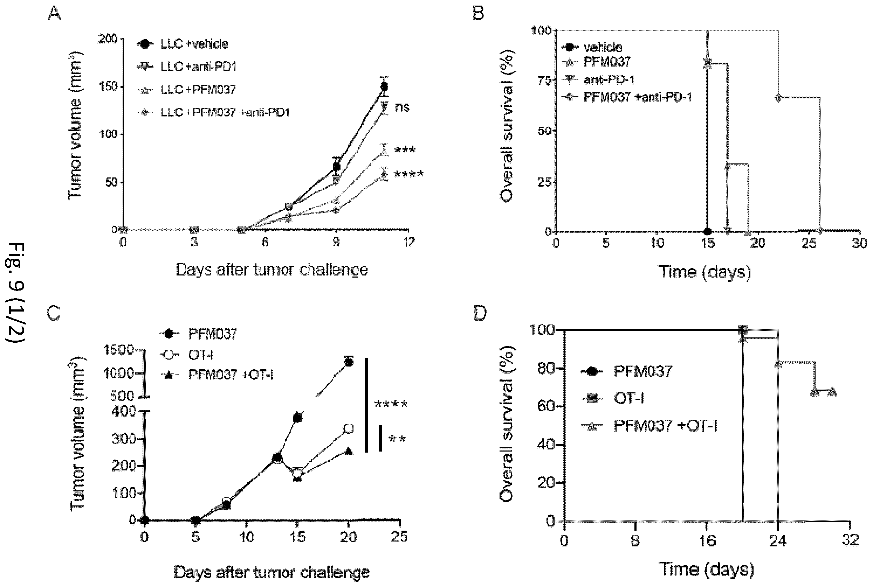

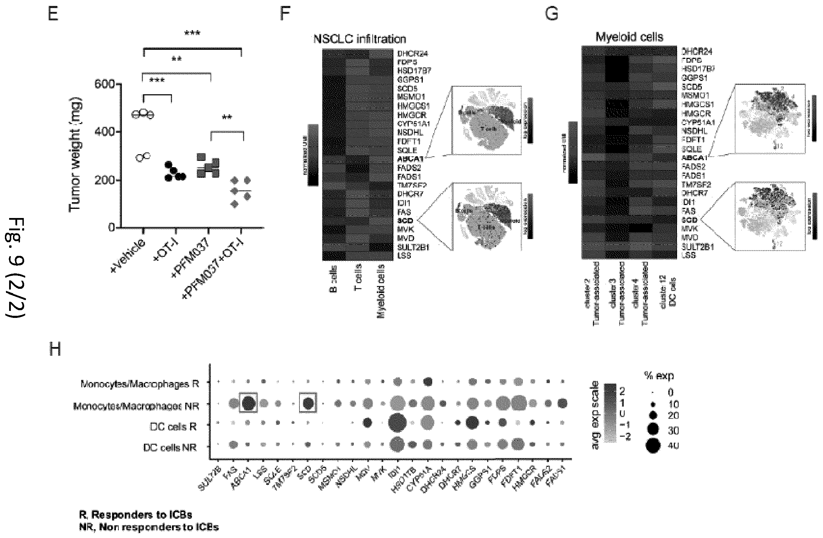

**Figure 9****. Synergizing effects of PFM037 and immunotherapy in controlling lung and melanoma tumor growth and analysis of myeloid cells infiltrating human tumors. (A)** LLC tumor growth in mice treated with PFM037, anti-PD-1 mAb, the combination therapy, or vehicle plus ctrl mAb (*n* = 5 mice/group). ^{∗∗∗}*P* < 0.001; ^{∗∗∗∗}*P* < 0.0001; ns, not significant (Student's t-test). **(B)** Overall survival of LLC-bearing mice treated with PFM037, anti-PD-1 mAb, the combination therapy, or vehicle plus ctrl mAb (*n* = 6 mice/group). Statistical comparison was performed by log-rank test (vehicle vs anti-PD1, *P* = 0.0051; vehicle vs PFM037, *P* = 0.0051; vehicle vs PFM037+anti-PD1, *P* = 0.0009). **(C, D)** Tumor volume **(C)** and overall survival **(D)** of NOD-SCID mice challenged with LLC-OVA and treated with PFM037 and/or adoptive cell therapy with in vitro activated OT-I T cells (n = 5 mice/group). For tumor volume ^{∗∗}*P* = 0.0018; ^{∗∗∗∗}*P* = 0.0001 (Student's t-test). For overall survival statistical comparison was performed by log-rank test (PFM037 vs OT-I, *P* = 0.0027; PFM037 vs PFM037+OT-I, *P* < 0.014; OT-I vs PFM037+OT-I, not significant). **(E)** B16F1-OVA tumor weight following the treatment with vehicle, PFM037, in vitro activated OT-I alone, or in combination with PFM037 (n = 5 mice/group). ^{∗∗}*P* < 0.01; ^{∗∗∗}*P* < 0.001 (Anova). **(F)** Average expression heatmap (normalized-UMI) of reverse cholesterol transport (RCT)/cholesterol synthesis genes in NSCLC immune-infiltrating cell populations⁴³. Insert displays t-SNE plots of all cells profiled, color-coded according to the expression of *ABCA1* and *SCD* transcripts in the same populations. **(G)** Average expression heatmap (normalized-UMI) of LXR/cholesterol synthesis genes in tumor-associated monocyte/macrophage and DC subsets of myeloid cells⁴³. Insert displays t-SNE plots of myeloid cells profiled, color-coded according to the expression of *ABCA1* and *SCD* transcripts in the same populations. **(H)** Dot plots of LXR/cholesterol synthesis genes in melanoma-infiltrating monocytes and DCs profiled, divided in Responder and Non-Responder patient-cells⁴⁴. Each dot is colored according to the expression value and the size is related to the percentage of cells expressing the gene in the population of interest.

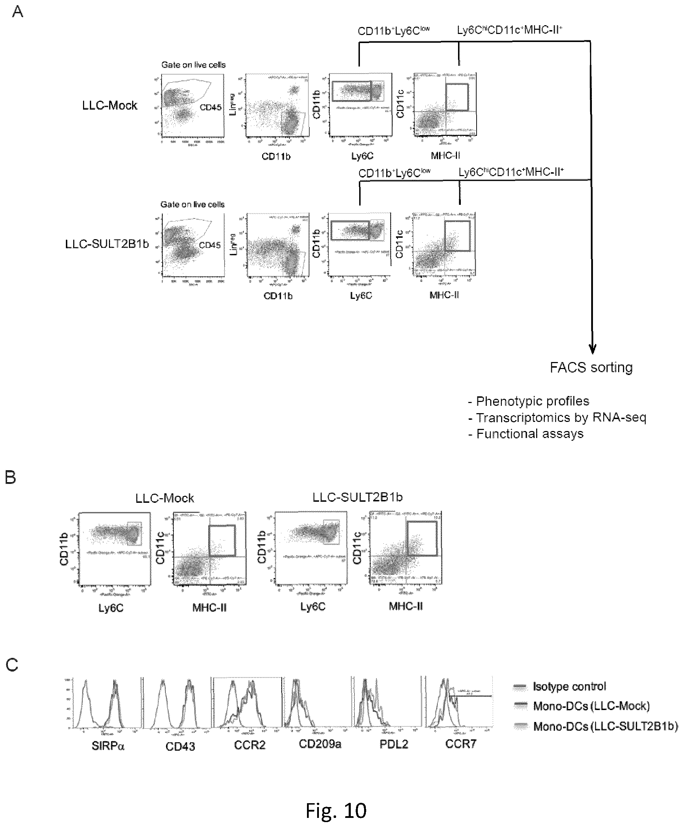

**Figure 10****. Gating strategy applied to characterize the tumor-infiltrating subsets of Ly6C+ cells. (A, B)** LLC-Mock and LLC-SULT2B1b tumors were digested and stained with a panel of mAbs against CD45, CD11b, Ly6C, CD11c and MHC-11 molecules. Dead cells were excluded. Lineage negative cells were identified by the following mAbs: CD3, NK1.1, CD19, CD49b, Ly6G and Ter119. (C) Cells in the red box as in B were stained with mAbs against SIRPα, CD43, CCR2, CD209a, PDL2 and CCR7, which identify the mono-DCs. Ly6ChighCD11c⁺MHC-II⁺ and CD11b+Ly6Clow cells were purified by FACS and analyzed by RNA-seq and functional assays.

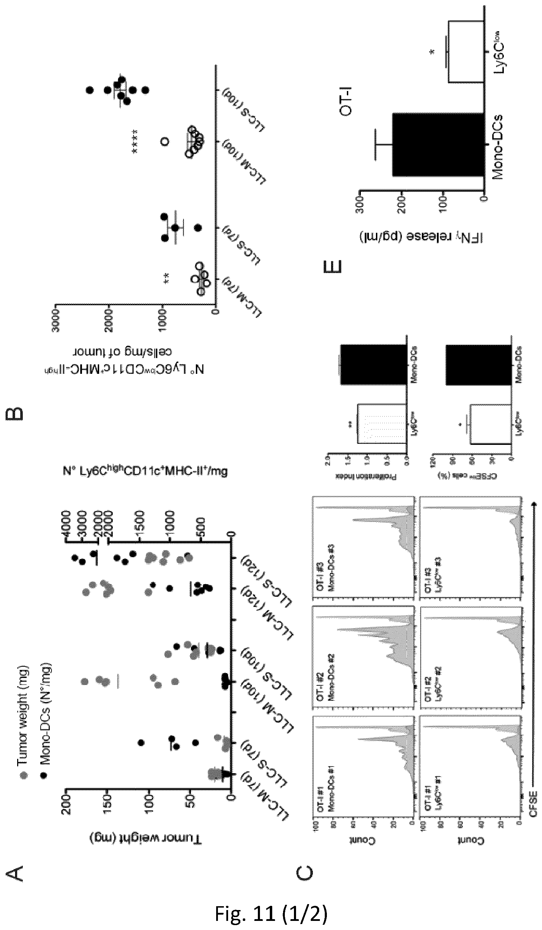

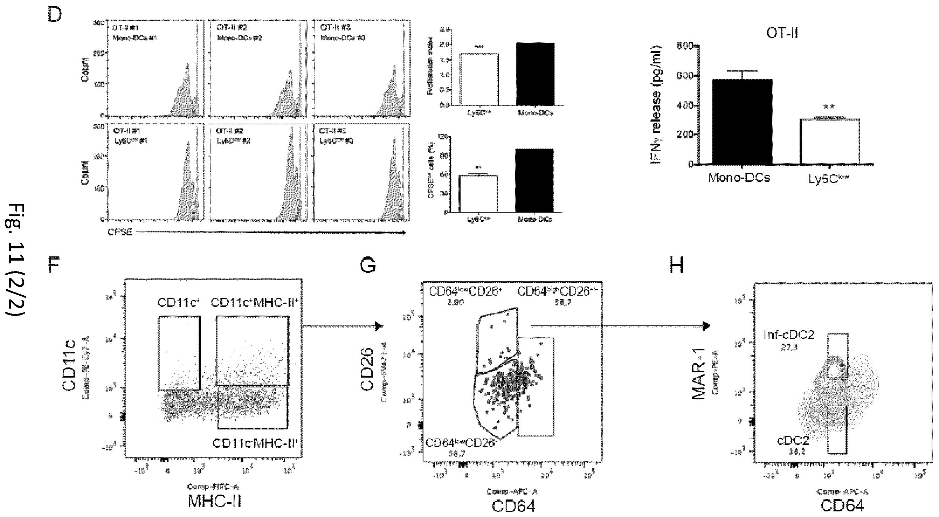

**Figure 11****. (A)** Tumor weight (mg) and absolute numbers of mono-DCs (Ly6C^{high}CD11c⁺MHC-II⁺ cells)/mg of LLC-Mock (LLC-M) and LLC-SLTLT2B1b (LLC-S) tumors collected at different times. Day 7, mean and s.d. of 4-5 mice/group. Day 10, mean and s.d. of 8 mice/group. Day 12, mean and s.d. of 7 mice/group. **(B)** Number of Ly6C^{low}CD11c⁺MHC-II⁺ cells/mg of LLC-Mock (LLC-M) and LLC-SULT2B1b (LLC-S) tumors collected at different different times. Day 7, mean and s.d. of 4-5 mice/group. Day 10, mean and s.d. of 8 mice/group. ^{∗∗}P < 0.01; ^{∗∗∗∗}P < 0.0001 (Student's t-test). **(C-D)** FACS analysis of OT-I (C) and OT-II (D) proliferation following in vivo priming with mono-DC or Ly6Clow cells isolated from LLC-SULT2B1b tumors and pulsed with MHC-I- and -II-restricted OVA peptides. Histograms represent the proliferation index of the percentage of OT-I (C) and OT-II (D) proliferation, evaluated as percentage of CFSE-diluted cells. Mean and s.d. of 3 experiments/group. ^{∗}P < 0.05; ^{∗∗}P < 0.01; ^{∗∗∗}P < 0.001 (Student's t-test). (E) Supernatants from the experiments described in C and D were collected after 48 hours of co-culture and tested for IFN-γ release. Mean and s.d. of 3 experiments per group. ^{∗}P < 0.05; ^{∗∗}P < 0.01 (Student's t-test). **(F-H)** Tumors digested and stained with mAbs against CD45, CD11b, Ly6C, CD11c, MHC-II, CD26, CD64 and MAR-1 molecules. (G) Analysis for the expression of CD26 and CD64 molecules was performed on gated CD11c+Ly6C^{high}MHC-II⁺ cells. (H) Analysis for the expression of MAR-1 marker was performed on gated CD64^{low}CD26⁺ cells.

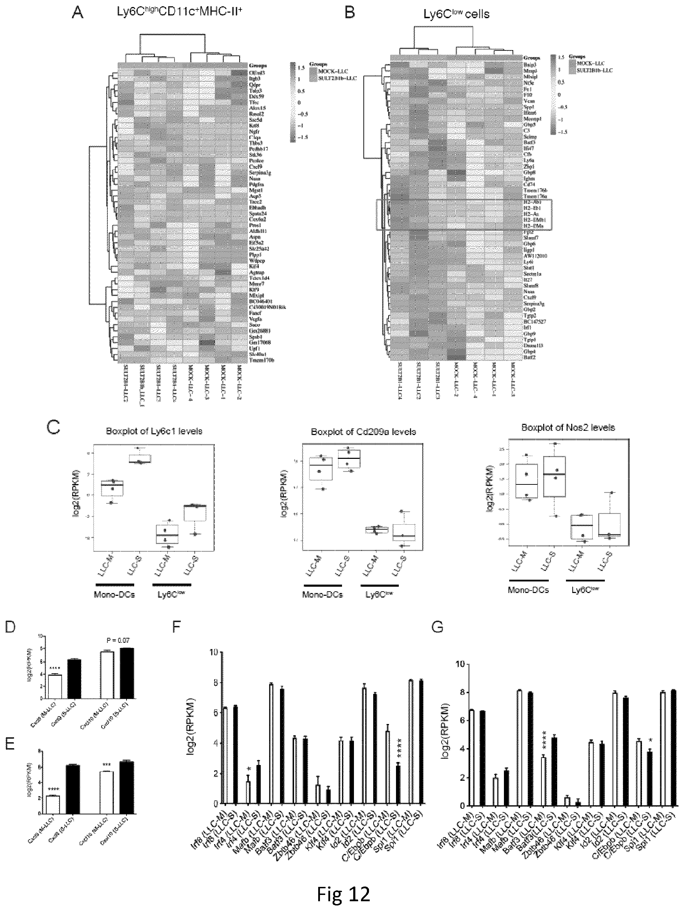

**Figure 12****. Transcriptomic analysis of monocyte-DCs and Ly6Clow cells under SULT2B1b modulation. (A, B)** Heatmap of the 50 genes differentially expressed between monocyte-DCs (A) and Ly6C^{low} cells (B) isolated from LLC-Mock and LLC-SULT2B1b tumors. Expression levels are represented in the heatmap as log2(RPKM) transformed to zero mean and unit variance. Genes encoding MHC-II molecules differentially expressed by Ly6C^{low} cells from LLC-Mock and LLC-SULT2B1b tumors are highlighted (red square). (C) Boxplots showing the expression of *Ly6Cl, Cd209a* and *Nos2* transcripts in mono-DCs and Ly6C^{low} cells isolated from LLC-M and LLC-S. Results are from RNA-seq data and are expressed as log2(RPKM). RPKM, Reads per Kilobase per Million. (D, E) Barplots representing the expression levels of *Cxcl9* and *Cxcl10* genes in mono-DCs (D) and Ly6C^{low} cells (D) isolated from Mock-LLC (M-LLC) and SLTLT2B1b-LLC (S-LLC). Results are from RNA-seq data and are expressed as log2(RPKM). RPKM, Reads per Kilobase per Million. P values refer to differential expression evaluated by limma. ^{∗∗∗}p < 0.001; ^{∗∗∗∗}p < 0.0001 (limma). (F, G) Barplots representing the expression levels of genes encoding distinct transcription factors in monocyte-DCs (F) and Ly6C^{low} cells (G), isolated from Mock-LLC (M-LLC) and SULT2B1b-LLC (S-LLC). Results are from RNA-seq data and are expressed as log2(RPKM). RPKM, Reads per Kilobase per Million. P values refer to differential expression evaluated by limma. ^{∗}p < 0.05; ^{∗∗∗∗}p < 0.0001 (limma).

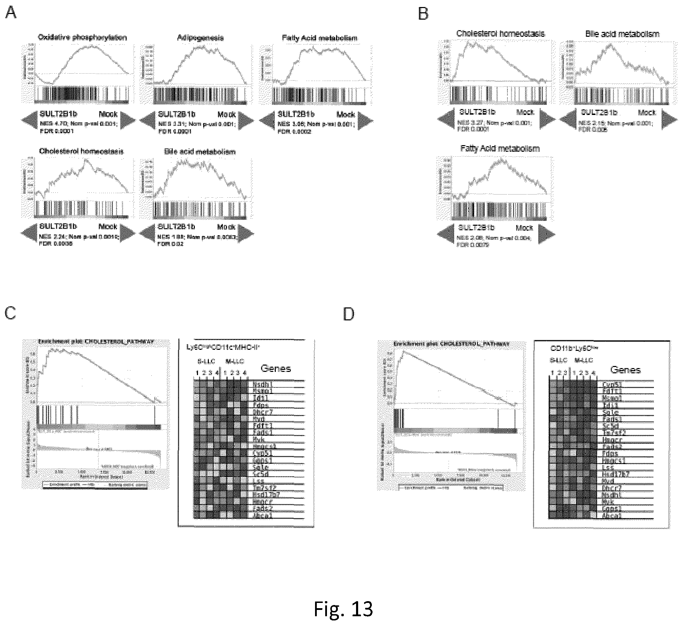

**Figure 13****. GSEA of tumor-infiltrating Ly6C^{high}CD11c⁺MHC-II⁺ and Ly6C^{low} cells under SULT2B1b modulation.** (A) Gene Set Enrichment Analysis (GSEA) tracing for oxidative phosphorylation, adipogenesis, fatty acid metabolism, cholesterol homeostasis and bile acid metabolism of tumor infiltrating Ly6C^{high}CD11c⁺MHC-II⁺ mono-DCs rom SULT2B1b- and Mock-LLC tumors. NES, normalized enrichment score; Nom p-val, Nominal p value; FDR, false discovery ratio. (B) GSEA tracing for cholesterol homeostasis, bile acid metabolism and fatty acid metabolism of tumor-infiltrating Ly6C^{low} cells from SULT2B1b- and Mock-LLC tumors. NES, normalized enrichment score; Nom p-val, Nominal p value; FDR, false discovery ratio. (C, D) GSEA using a signature of genes involved in LXR/cholesterol synthesis pathways, as reported in³². The gene set is significantly up-regulated in Ly6C^{high}CD11c⁺MHC-II⁺ mono-DCs (C) and Ly6C^{low} cells (D) isolated from LLC-SULT2B1b tumors. Nominal p value < 1 x 10⁻⁴. Abbreviations are as follows: S-LLC, LLC-SLTLT2B1b; M-LLC, LLC-Mock.

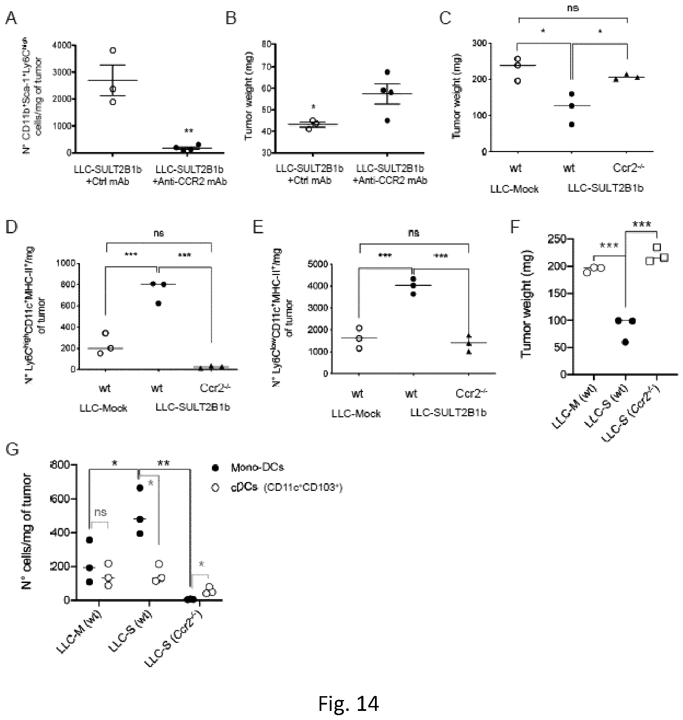

**Figure 14****. Monocyte-DCs control tumor growth under SULT2B1b perturbation.** (A, B) Mice bearing LLC-SULT2B1b tumors were treated with anti-CCR2 or ctrl mAbs for five consecutive days. At sacrifice, tumors were collected and analyzed for the content of tumor-infiltrating mono-DCs and for tumor weight. Absolute numbers of mono-DCs, evaluated as Ly6C^{high}CD11b⁺Sca-1⁺ cells/mg of tumor (A) and tumor weight (B) between the two groups of treatment. Results are presented as mean ± s.d. of 3-4 mice/group; ^{∗}P < 0.05; ^{∗∗}P < 0.01 (Student's t-test). (C-E) *Ccr2*^{*-*/*-*} and wild type mice were infused with LLC-Mock and LLC-SULT2B1b tumors. At sacrifice, tumors were collected and analyzed for the content of monocyte-DCs, Ly6C^{low}CD11c⁺MHC-II⁺ cells and tumor weight. Tumor weights (C), absolute numbers of monocyte-DCs (D) and Ly6C^{low}CD11c⁺MHC-II⁺ cells/mg of tumor (E) between the groups of treatment. Mean and s.d. of 3 mice/group. ns, not significant; ^{∗}P < 0.05; ^{∗∗∗}P < 0.001 (ANOVA). (F-G) *Ccr2*^{*-*/*-*} and wild type mice were infused with LLC-Mock and LLC-SULT2B1b tumors. At sacrifice, tumors were collected and analyzed as described above. (F) Tumor weights between the groups of treatment. Mean and s.d. of 3 mice/group. ^{∗∗∗}p < 0.001 (ANOVA). (G) Absolute numbers of monocyte-DCs and (CD103₊CD11c₊) cDCs/mg of tumor. Mean and s.d. of 3 mice/group. ns, not significant; ^{∗}P < 0.05; ^{∗∗}P < 0.01 (ANOVA). cDCs were not significantly different among the three.

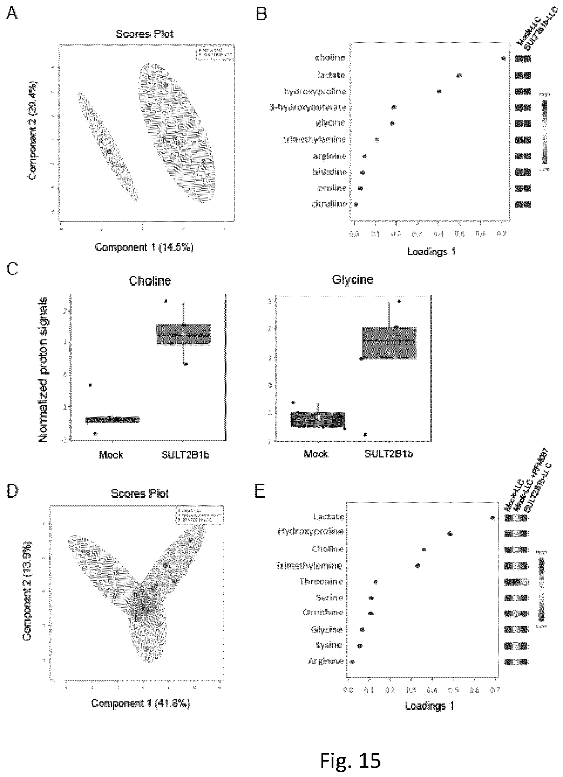

**Figure 15****. Un-targeted metabolomics analysis of LLC-Mock and LLC-SULT2B1b, LLC from mice treated with vehicle or PFM037.** (A) Score plot by Partial least squares-discriminant analysis (PLS-DA) related to the polar fraction of Mock and SULT2B1b-LLC tumors (n = 5 tumors/group). (B) Variable importance in projection (VIP) plot showing the top 10 NMR signals resulted more statistically different in the polar fractions of Mock and SULT2B1b-LLC tumors (n = 5tumors/group). (C) Box plots showing the levels of choline and glycine, evaluated as normalized proton signals, differentially expressed by Mock and SULT2B1b-LLC tumors (n = 5 tumors/group). (D) Score plot by Partial least squares-discriminant analysis (PLS-DA) related to the polar fractions of Mock-LLC, SLTLT2B1b-LLC and Mock-LLC treated with PFM037 tumors (n = 5). (E) Variable importance in projection (VIP) plot showing the top 10 NMR signals resulted more statistically different in the polar fractions of Mock-LLC, SLTLT2B1b-LLC and Mock-LLC treated with PFM037 tumors (n = 5). groups of tumors (ANOVA).

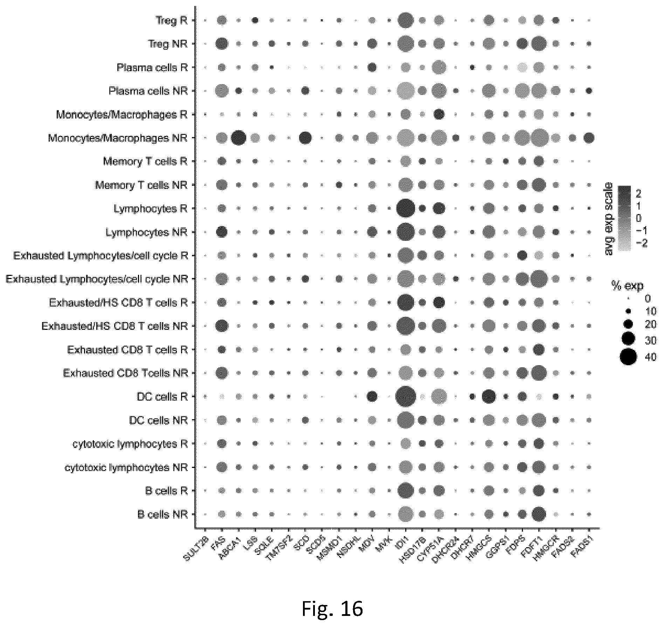

**Figure 16****.** Dot Plots of LXR/cholesterol synthesis signature genes in all melanoma-infiltrating CD45⁺ cells profiled, divided in Responder (R) and Non Responder (NR) patient-cells (21). The intensity of color in each dot represents the expression value. The size of each dot is related to the percentage of cells expressing the gene in the population of interest.

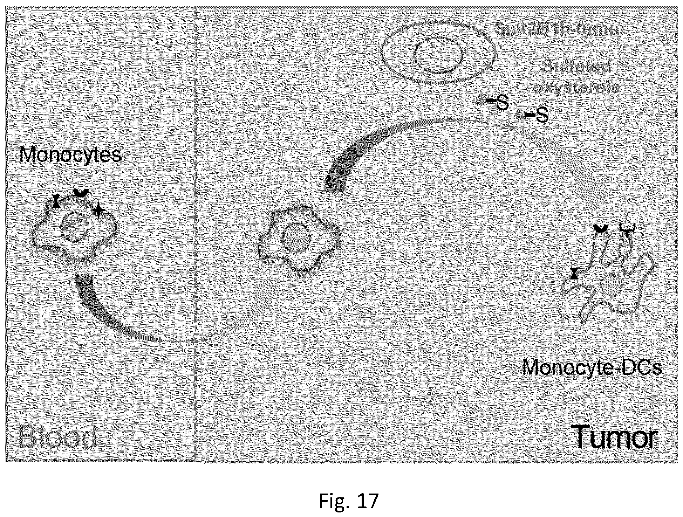

**Figure 17****.** Cartoon summarizing the activity of sulphated tumours and sulphated oxysterols on monocyte-to-DC differentiation. Monocytes recruited to tumors expressing the sulfotransferase 2B1b (Sult2B1b) or in the presence of exogenously administered sulphated oxysterols, differentiate to monocyte-DCs, which exert antitumor effects by activating/stimulating tumor antigen specific T cells.

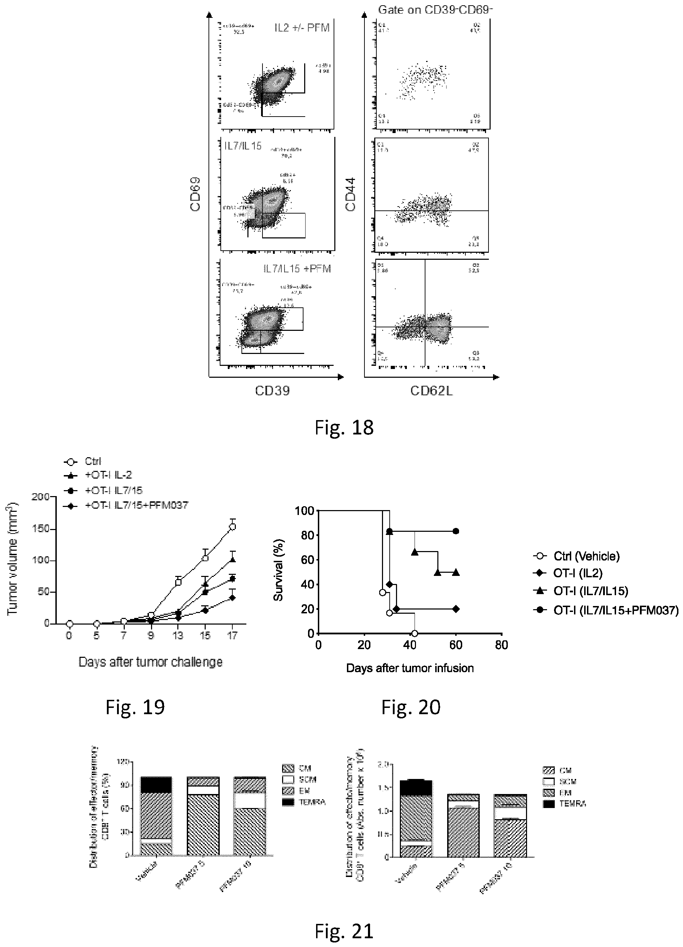

**Figure 18****.** Evaluation whether PFM037 induces functional and phenotypic changes *of in vitro* activated OT-T cells. OT-I T cells were activated in the presence of anti-CD3/CD28 coated beads and IL-2. Forty-eight hours later the cells were differentiated and expanded by IL-7/IL15 with or without PFM037. FACS analysis was performed 10, 12 and 15 days later, to evaluate their differentiation status. OT-I T cells expanded with PFM037 progressively lost CD39 and CD69 markers, while progressively acquiring CD62L and CD44^{low} markers, a phenotype associated to T Stem Cell Memory (T_{SCM}) cells.
**Figure 19****.** Antitumor activity of OT-I T cells expanded by IL-7/IL15 with PFM037 *in vivo.* OT-I T cells expanded with IL-2 (effectors) or IL-7/IL15 with or without PFM037 (memory T cells) were injected in mice bearing 7-day established B16F1 melanoma expressing OVA.
**Figure 20****.** Percentage of the overall survival of tumor-bearing mice injected with vehicle or with OT-I T cells expanded with IL-2 or with IL-7/IL15 with or without PFM037.
**Figure 21****.** *In vitro* experiments by using human T cells activated in the presence of anti-CD3/CD28 coated beads and then expanded by IL-7/IL15 with or without PFM037 (5 and 10 µM).

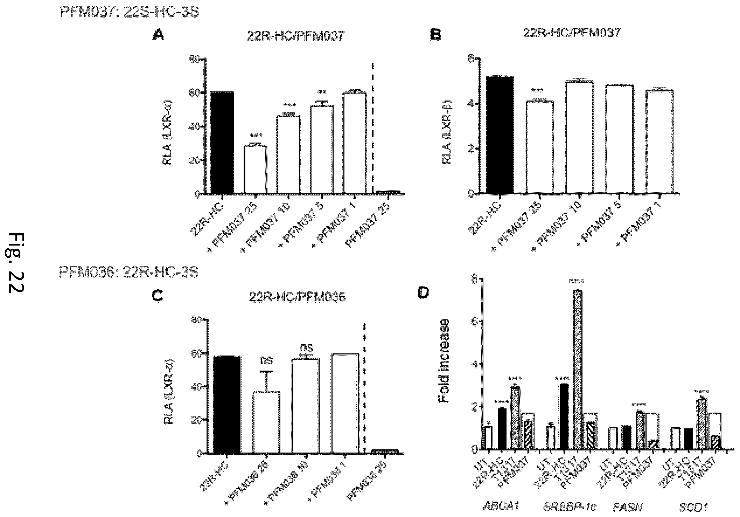

**Figure 22****.** (A) Luciferase-based LXRα assays displaying the antagonistic activity of the sulfated oxysterol 22S-HC-3S (PFM037). Mean and s.d. of 2 independent experiments. LXRα activation was induced by 10 µM of 22R-HC, while PFM037 was used at 25, 10, 5 and 1 µM. RLA, Relative Luciferase Activity. **(B)** Luciferase-based LXRβ assays displaying the antagonistic activity of PFM037. Mean and s.d. of 2 experiments. LXRβ activation was induced by 10 µM of 22R-HC, PFM037 was used at 25, 10, 5 and 1 µM. RLA, Relative Luciferase Activity. **(C)** Luciferase-based LXRα assays to test the antagonistic activity of the sulfated oxysterol 22R-HC-3S (PFM036). PFM036 does not antagonize LXR activation induced by 5 µM of 22R-HC. PFM036 was used at 25, 10, and 1 µM. RLA, Relative Luciferase Activity. **(D)** Analysis of the expression of the LXR target genes ABCA1, SREBP1c, FASN and SCD1 following T0901317 (T1317) or PFM037 treatment. Mean and s.d. of three independent experiments. ^{∗}*P* < 0.05; ^{∗∗}*P* < 0.01; ^{∗∗∗}*P* < 0.001; ^{∗∗∗∗}*P* < 0.0001 (Anova).

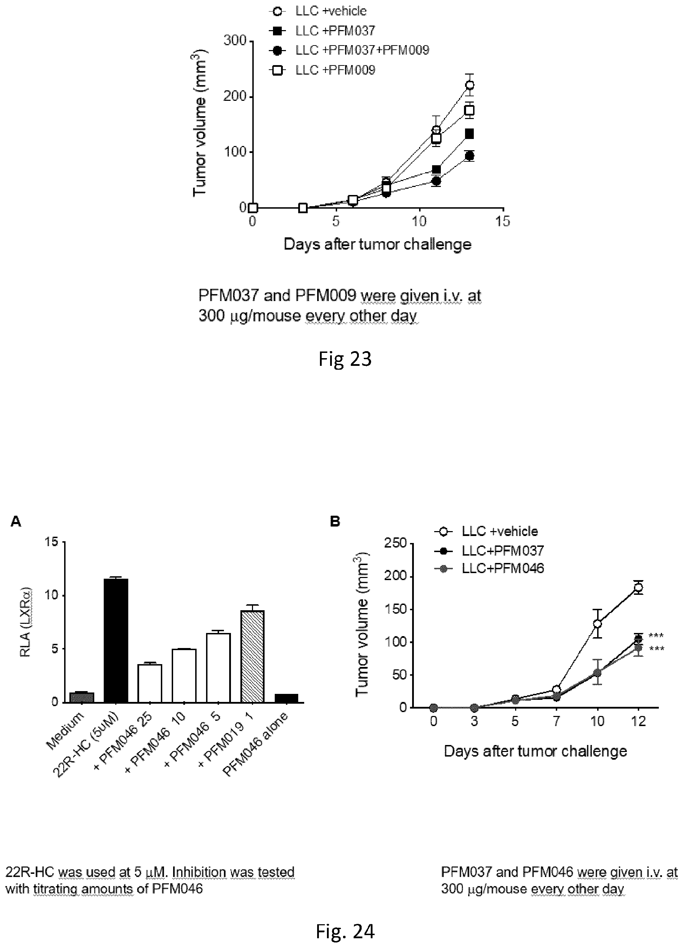

**Figure 23****.** Mice bearing the established Lewis Lung Carcinoma (LLC) were treated with vehicle, PFM037, PFM009 and the combination of PFM037 and PFM009 (300 µg/mouse every other day) and monitored for tumor growth. Mean and s.d. of one experiment (n = 4 mice/group). The combo treatment resulted to be more effective than single compounds in controlling LLC tumor growth.
**Figure 24****. (A)** Luciferase-based LXRα assays displaying the antagonistic activity of the synthetic PFM046 compound. Mean and s.d. of 2 independent experiments. LXRα activation was induced by 5 µM of 22R-HC, while PFM046 was used at 25, 10, 5 and 1 µM. RLA, Relative Luciferase Activity. **(B)** Mice bearing established LLC tumors were treated with vehicle, PFM037 or PFM046 (300 µg/mouse every other day) and monitored for tumor growth. Mean and s.d. of one experiment (n = 5 mice/group). ^{∗∗∗}*P* < 0.001 (Student's t-test). Both compounds were able to control LLC tumor growth.

### MATERIALS AND METHODS

**Animal studies and reagents.** C57BL/6 CD45.1, CD45.2, transgenic OT-I and OT-II mice were from Charles River (Calco, Italy). *Ccr2*^{*-*/*-*} mice were from Jackson Laboratory. Mice were maintained in the pathogen-free facility of San Raffaele Scientific Institute. Experiments were conducted in compliance with the Institutional Animal Care and Use Committee programme (IACUC n° 656, 657 and 835) and in accordance with ARRIVE guidelines. Lewis Lung Carcinoma (LLC) cell line, LLC-Mock, LLC-SULT2B1b, LLC-OVA and LLC-mCherry were maintained in DMEM complete medium (10% FBS supplemented with penicillin, streptomycin, glutamine and Hepes). Tumor cell lines were from ATCC and were tested for mycoplasma contamination. RMA was in RPMI complete medium and B16F1-OVA in DMEM complete medium. 25-Hydroxycholesterol-3-sulfate (25-HC-3S) was from Avanti Polar Lipids. Carboxyfluorescein succinimidyl ester (CFSE) was used at 5 µM (Molecular Probes). MHC-I-restricted SIINFEKL (257-264) (SEQ ID NO: 11) and MHC-II-restricted ISQAVHAAHAEINEAGR (323-339) (SEQ ID NO: 12) OVA peptides were from InvivoGen. Monocyte Isolation Kit (Miltenyi). Click-it EdU flow cytometry assay kit (Thermofisher). PFM009 was prepared according to Marinozzi et al. (2017).⁵⁸

### PFM037 synthesis

PFM037 (22(*S*)-hydroxycholesterol-3-sulfate sodium salt) was synthesized starting from 6β-methoxy-3α,5-cyclo-23,24-dinor-5α-cholan-22-al **(1)⁶¹** in four steps and 26.8 % overall yield.

### Scheme 1. Synthesis of 22(S)-Hydroxycholesterol-3-sulfate sodium salt (PFM037)^{a}

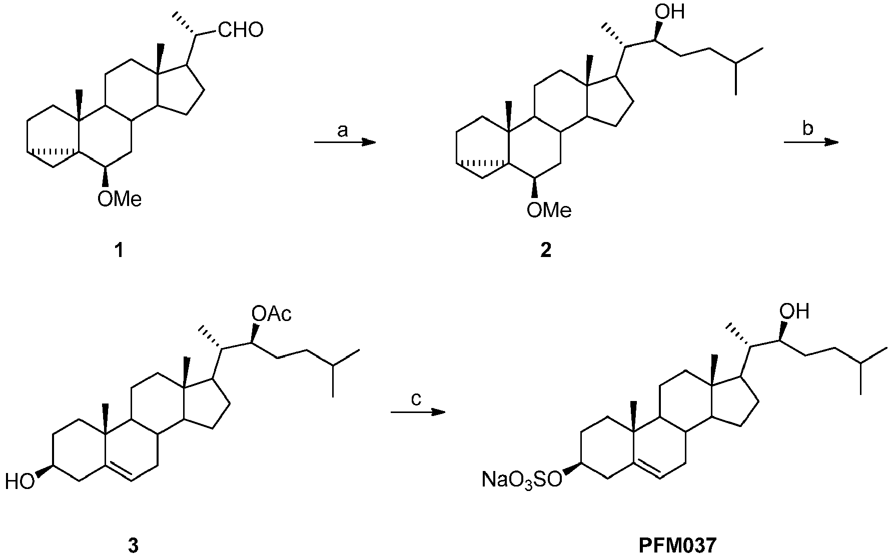

^{a}Reagents and conditions: (a) isoamyl bromide, Mg, 0 °C to rt, 5 h; (b) i. AczO, 4-DMAP, CH₂Cl₂, pyridine, rt, 20 h; ii. pTsOH, dioxane/H₂O, 80 °C, 1 h; (c) i. SO₃-pyridine complex, pyridine, rt, 4 h; ii. 1.25 M NaOH in MeOH, reflux for 1 h, then rt overnight.

***22(S)-6β-Methoxy-3α,5-cyclo-5α-cholest-22-ol (2).*** A solution of isoamyl bromide (0.65 g, 4.31 mmol) in THF (1.5 mL) was added dropwise to a stirred suspension of magnesium turnings (0.11 g, 4.83 mmol) in THF (3 mL) and the resulting mixture was kept at 0 °C under argon atmosphere. After 1 h, a solution of 1 (0.50 g, 1.45 mmol) in THF (3 mL) was added dropwise and the mixture was allowed to reach rt. After 4 h, a saturated solution of NH₄Cl (15 mL) was added and the mixture extracted with EtOAc (2 × 15 mL). The combined organic layers were washed with brine (10 mL) and dried over anhydrous Na₂SO₄. After the removal of the solvent, the crude product, thus obtained, was purified by flash chromatography (light petroleum/EtOAc, 95:5) affording **2** in 66% yield. ¹H NMR (400 MHz, CDCl₃) δ 0.43 (s, 1H), 0.65 (s, 1H), 0.73 (s, 3H), 1.87-1.99 (m, 3H), 2.77 (s, 1H), 3.32 (s, 3H), 3.63 (s, 1H). ¹³C NMR (100 MHz, CDCl₃) δ 11.43, 12.17, 13.04, 19.26, 21.43, 22.54, 22.69, 22.76, 24.09, 24.92, 27.79, 28.17, 30.52, 33.19, 33.30, 35.03, 35.18, 35.63, 40.12, 40.24, 42.66, 43.33, 47.93, 52.67, 56.36, 56.54, 73.88, 82.35.

***22(S)-Acetoxycholest-5-en-3β-ol* (3).** Acetic anhydride (0.08 g, 0.83 mmol) and 4-DMAP (0.01 g, 0.10 mmol) were added to a stirred solution **of 2** (0.16 g, 0.40 mmol) in CH₂Cl₂ (15 mL) and pyridine (0.06 mL) and the resulting mixture was kept at room temperature under argon atmosphere. After 20 h, a solution of 3 M HCl (10mL) was added and the organic layer was separated and washed with H2O (15 mL), brine (15 mL) and then dried over anhydrous Na2SO4. After the removal of the solvent the crude product was dissolved in dioxane/water (3:1, 8 mL) and p-toluenesulfonic acid (0.01 g, 0.04 mmol) was added to the resulting stirred solution. After heating at 85 °C for 1 h, the mixture was concentrated *in vacuo,* water (5 mL) was added and the mixture extracted with EtOAc (2 × 7 mL). The combined organic layers were washed with brine (10 mL) and dried over anhydrous Na₂SO₄. After the removal of the solvent the crude product was purified by flash chromatography. Elution with light petroleum/EtOAc, (80:20) gave **3** in 70% yield. ¹H NMR (400 MHz, CDCl₃) δ 0.59 (s, 3H), 0.77 (s, 3H), 0.79 (s, 3H), 0.91 (s, 3H), 1.95 (s, 3H), 2.10-2.25 (m, 2H), 3.44 (m, 1H), 4.84 (t, 1H, *J* = 6.7 Hz), 5.26 (bs, 1H); ¹³C NMR (100 MHz, CDCl₃) δ 11.62, 12.69, 19.36, 21.04, 21.25, 22.40, 22.66, 24.20, 28.02, 28.09, 29.96, 31.57, 31.78, 31.87, 34.89, 36.43, 37.20, 38.89, 39.67, 42.21, 50.03, 52.47, 56.57, 71.71, 76.56, 121.65, 140.65, 171.00.

***22(S)-Hydroxycholesterol-3-sulfate sodium salt (PFM037).*** Sulfur trioxide-pyridine complex (0.12 g, 0.76 mmol) was added to a stirred solution of **3** (0.16 g, 0.38 mmol) in pyridine (1.5 mL) and the resulting mixture was stirred at rt under argon atmosphere. After 4 h cold light petroleum (2 mL) was added and the precipitate, thus formed, was separated by vacuum filtration and then washed with cold light petroleum. The solid was then suspended in 1.25 M NaOH in MeOH (3 mL) and the resulting mixture heated at reflux for 1 h. After further 12 h of stirring at rt, H₂O (0.5mL) was added and the resulting precipitate isolated by vacuum filtration, washed with H₂O and acetone and finally dried to afford PFM037, as a white solid, in 58% yield: mp = 182.2-183.3 °C; ¹H NMR (400 MHz, DMSO-d₆) δ 0.56 (s, 3H), 3.31 (s, 1H), 3.76 (s, 1H), 5.20 (s, 1H); ¹³C NMR (100 MHz, DMSO-d6) δ 11.96, 12.07, 19.37, 20.94, 22.87, 23.00, 27.61, 28.00, 29.08, 31.82, 33.19, 35.81, 36.39, 37.17, 41.94, 49.87, 52.25, 56.58, 71.67, 75.68, 121.47, 140.96.

### PFM046 synthesis

PFM046 ((22S)-23-Phenyl-24-norchol-5-en-3β,22-diol) was synthesized starting from 6β-methoxy-3α,5-cyclo-23,24-dinor-5α-cholan-22-al **(1)**⁶¹ in two steps and 52 % overall yield.

### Scheme 2. Synthesis of (22S)-23-Phenyl-24-norchol-5-en-3β,22-diol (PFM046)

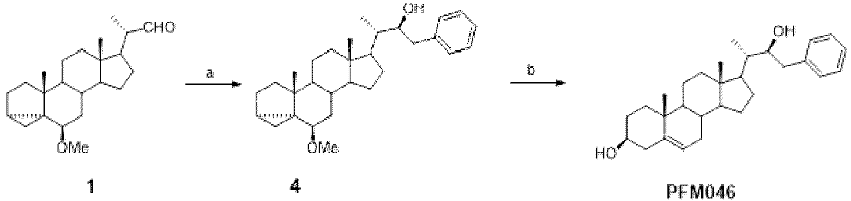

Reagents and conditions: (a) PhCH₂MgBr, THF. 0 °C to rt, (65 %); (b) pTSA, H2O/1,4-dioxane, 75 °C (80%).

***(22S)-6β-Methoxy-23-phenyl-3α,5-cyclo-5α-24-norcholan-22-ol* (4).** A solution of benzyl bromide (5 eq) in dry THF (0.5 mL/mmol of Mg) was added dropwise to a stirred suspension of magnesium turnings (5.5 eq) in dry THF (0.5 mL/mmol of Mg) and a few crystals of sublimated elemental iodine kept under argon atmosphere at room temperature. After 1-3 h the resulting silvered fine suspension was cooled to 0 °C by an ice/water bath and a solution of the aldehyde **1** (1 eq) in dry THF) (1.5 mL/mmol Mg) was slowly added dropwise. As the end of the addition the system was allowed to warm to room temperature. After 15 h the reaction was quenched by the addition of saturated solution of aqueous NH₄Cl and was extracted with EtOAc. The combined organic layers were washed with brine and dried over anhydrous Na₂SO₄. After the removal of the volatiles at reduced pressure, the crude oily product was purified by flash chromatography (eluent: light petroleum/EtOAc, 9:1, v/v). **4** was obtained in 65 % yield as a gluey wax. ¹H-NMR (400 MHz, CDCl₃): δ 0.36 (dd, 1H, *J* = 5.16 and 7.83 Hz), 0.58 (t, 1H, *J* = 4.59 Hz), 0.63 (s, 3H), 2.56 (dd, 1H, *J* = 4.27 and 13.48 Hz), 2.70-2.76 (m, 2H), 3.25 (s, 3H), 3.81 (m, 1H), 7.13-7.25 (m, 5H). ¹³C-NMR (100 MHz, CDCl₃): δ 11.87, 12.07, 12.99, 19.21, 21.36, 22.71, 24.00, 24.87, 27.68, 30.45, 33.25, 34.95, 35.11, 40.16, 40.22, 42.00, 42.59, 43.26, 47.86, 52.64, 56.25, 56.47, 74.52, 82.27, 126.17, 128.42, 129.14, 139.44. ***(22S)-23-Phenyl-24-norchol-5-en-3β,22-diol (PFM046)***

To a stirred solution of **4** in 1,4-dioxane (28 mL/mmol) and water (9 mL/mmol) *p-*toluensulfonic acid hydrate (0.3 eq) was added. The resulting mixture was warmed to 75 °C and stirred for 2.0 h. The reaction was quenched by diluting with water and extracting with EtOAc. The combined organic layers were washed with brine and dried over anhydrous Na₂SO₄. After the removal of the volatiles at reduct pressure, the crude oily product was further purified by flash chromatography chromatography (eluent: light petroleum/EtOAc, 7:3, v/v).

**PFM046** was obtained in 80% yield as a white powder. mp = 181.4-183.6 °C. ¹H-NMR (400 MHz, CDCl₃): δ 2.57 (s, 1H, 23-CHₐ), 2.73 (s, 1H, 23-CH_{b}), 3.43 (s, 1H, 3-CH), 3.82 (s, 1H, 22-CH), 5.26 (s, 1H, 6-CH), 7.13-7.22 (m, 5H, 23-C₆H₅). ¹³C-NMR (100 MHz, CDCl₃): δ 11.71, 11.91, 14.15, 19.35, 21.06, 24.14, 27.66, 31.55, 31.78, 31.88, 36.42, 37.21, 39.73, 40.21, 42.03, 42.20, 50.04, 52.56, 56.58, 60.36, 71.66, 74.60, 121.54, 126.23, 128.28 (2C), 129.17 (2C), 139.43, 140.75.

**Immunofluorescence staining and confocal analysis.** Tissues were harvested and fixed for 10 minutes in 4% (w/v) PFA (Sigma-Aldrich), then washed in PBS and dehydrated overnight in 30% sucrose (Sigma-Aldrich) at 4°C. Samples were embedded in Tissue-Tek OCT compound (Bio-optica) and frozen in ethanol dry-ice bath (using Dehyol 95; Bio-optica). 7-10 µm thick sections were placed onto glass slides (Bio-optica), fixed in cold acetone for 5 minutes, dried, and kept at -80°C until used. Slides were incubated 30 minutes with a blocking solution of PBS at 0.5% FBS and 0.05% Tween 20 (VWR) (PBS-T 0.05%), followed by primary specific antibodies or secondary reagents. Immunofluorescence images were acquired using Zeiss Axio Observer.Z1 microscope. Confocal images were acquired using Leica TCS SP8 microscope. Digital images were recorded in separately scanned channels with no overlap in detection of emissions from the respective fluorochromes. Final image processing was performed with Adobe Illustrator CS6 and Adobe Photoshop CS6. The following antibodies and detection reagents were used: CD11b-APC (M1/70 clone) and CD3-PE (17A2 clone) and CD45.1-FITC (A20 clone) from Biolegend; mCherry (NBP2-25158 Novusbio); biotinylated CD1 1c (HL3 clone, hamster) from BD Bioscience and Streptavidin 488 or 647 (Thermo Fisher, Invitrogen). For quantification analysis, a total of 64 pictures taken on 5 LLC-Mock-bearing mice and 79 pictures taken on 5 LLC-SULT2B1b-bearing mice were analyzed for T-cell numbers and T-cell-DC clusters. Draining lymph nodes were harvested, fixed and processed as described above.

**Macroscopic liver tumor quantification and Immunohistochemistry.** At time of autopsy, visible macroscopic tumors were counted, and each liver macroscopically photographed. Livers were then perfused with PBS, harvested and different pieces were sampled, fixed in zinc-formalin and embedded in paraffin for hematoxylin/eosin or immunohistochemical analysis as described⁶². Immunohistochemical staining was performed using a Bond RX Automated Immunohistochemistry system (Leica Microsystems GmbH, Wetzlar, Germany) on 3-µm-thick sections. First, tissues were deparaffinized and pre-treated with the Epitope Retrieval Solution [ER1 Citrate Buffer for GFP (dilution 1:500, GFP Polyclonal Antibody, Thermo Fisher Scientific, Cat# A11122, RRID:AB_221569). After washing steps, peroxidase blocking was carried out for 10 min using the Bond Polymer Refine Detection Kit DS9800 (Leica Microsystems GmbH). Then, tissues were washed and incubated for 1h RT with the primary antibody diluted in IHC Antibody Diluent (Novocastra, Leica RE7133). Subsequently, tissues were incubated with polymer-HRP (Biocare Medical, RT517H), developed with DAB-Chromogen for 10 minutes and counterstained with Hematoxilin for 5 minutes. For image acquisition and analysis eSlide Manager (Aperio Leica Biosystems) was used. All images were acquired using the Aperio AT2 system (Leica Biosystems). A total area of 97,6 mm² (Vehicle) and 104,98 mm² (PFM037) of liver was analyzed for each group of mice. Tumor cell quantification (MC38-GFP) was performed by automated image analysis software through the Color Deconvolution Algorithm v9.1 of the ImageScope program (version 12.4.3), following manufacturer's instructions (Leica Biosystems). The images shown were identified as representative area of interest within the total area of the specimen and exported as ImageScope snapshots at 20x magnifications as described³⁸.

**Analysis of tumor infiltrating cells by flow cytometry.** Mice were challenged with LLC, LLC-Mock, LLC-SULT2B1b and RMA tumor cells *(see **tumor challenge experiments** for details*). Tumors collected 7, 10, 12 or 14 days after injection were cut into small fragments and digested with Liberase TL (66 µg/ml, Roche) and DNAse I (150 µg/ml, Roche) for 25-30 minutes at 37 °C. Digested tissues were filtered by using a 75 µm diameter cell strainer. Single cell suspensions were stained with Live/Dead Violet kit reagents (Molecular Probes) for 30 minutes at 4°C. After washing, the cells were incubated for 5-10 minutes at RT with Fc-blocking solution (Anti-CD16/32, 10 µg/ml mouse Fc Block, BD) and then stained with various antibodies incubated at 4°C for 20 minutes. The following antibodies were used: mAbs against mouse CD45 (30F11), CD45.1 (A20), CD45.2 (104), CD11b (MI/70), Ly6C (HK1.4), Ly6G (1A8), CD11c (N418), CCR2, MHC-II (M5/114.152), CD43 (S11), CD3 (145-2c11), CD8 (53-6.7), IFNγ (XMG1.2), CD19 (6D5), CD49b (DX5) and Ter119, CD26, CD64, MAR-1 all from Biolegends. CD209a (5H10), CCR7 (4b12) and F4/80 (BM8) were from eBioscience. Rat IgG, Rat IgG2a, Rat IgG2b, Rat IgM and Hamster isotype control mAbs were used as controls (Biolegends and BD Biosciences). For intracellular staining, cells were incubated with PMA (100 ng/ml) and ionomycin (500 ng/ml) in the presence of 10 µg/ml of Brefeldin for 4 hours. Cells were then fixed using the IC Fixation buffer (eBioscience), permeabilized with 1x Permeabilization buffer (eBiosciences) and stained with anti-IFNy mAb. Samples were run by FACSCanto flow cytometer and analyzed by FlowJo software gating on live cells. FACS sorting was performed by using the FACSAria Fusion (BD)

**RNA-seq experiments.** FACS-sorted monocyte-DCs and Ly6C^{low} cells were collected in complete RPMI and pelleted. Total RNA was extracted using RNA spin columns (PureLink RNA Mini Kit, Ambion). RNA quantification was carried out using Qubit Fluorimetric Quantification (Life Technologies) and 500 ng of RNA were used to prepare the library. Sequencing libraries were prepared following SMART-Seq v4 Ultra Low Input RNA (TaKaRa) protocol and Illumina TRUSEQ stranded protocol. Briefly, 1 ng of total RNA was used for cDNA synthesis, followed by PCR amplification. Quality and quantity of cDNA was checked running samples in DNA HS chip (Bioanalyzer 2100, Agilent). 500 pg of cDNA from each sample were used for tagmentation, followed by PCR and barcoding (Nextera XT, Illumina). Sequencing was performed using an Illumina Nextseq 500 with a High Output flow cell, 75 nt read length, to obtain an average of 30M reads for each sample. Sequencing quality control and read alignment were obtained using the FastQC software to assess the quality of the fastq files [Andrews S. (2010). FastQC: a quality control tool for high throughput sequence data. Available online at: http://www.bioinformatics.babraham.ac.uk/proiects/fastqc]*.* Reads were aligned to the mouse genome, version mm10, using STAR_2.5.3⁶³ and the quality of the alignment assessed with bamtoools⁶⁴. The Rsubread package⁶⁵ was used to assign read counts to the genes of the GENCODE basic gene model, version M13 (*https:*//*www.gencodegenes.org*), with all isoforms of each gene considered together.

**Differential Expression of RNA-seq datasets.** Genes with at least 1 cpm (count per million) in at least the number of samples of the group having the minimum (e.g. 3 samples if comparing a group of 3 samples vs a group of 3 or 4 samples) were considered expressed and assessed for differential expression. Differentially expressed genes were identified using limma⁶⁶. Pairwise comparisons between the experimental groups (*e.*g. LLC-SULT2B1b and LLC-Mock conditions) were performed independently for monocyte-DCs and Ly6C^{low} cells. Genes with nominal *P* value < 0.01 and absolute value of log2 fold change > 1 were considered differentially expressed⁶⁷.

**Gene Set Enrichment Analysis (GSEA).** GSEA⁶⁸ (*http:*//*www.broad.mit.edu*/*gsea*/) was used to analyze the expression profile of a gene signature associated to cholesterol metabolism as reported in³².

**Comparing transcriptomes of monocyte-DCs and Ly6C^{low} cells with public datasets.** Gene expression profiles derived from RNA-Seq experiment were integrated with 4 datasets present in GEO (Gene Expression Omnibus) Database: GSE149619²⁴, GSE90471²³, GSE74427²⁵ and GSE49358²⁹. Probeset-level data obtained from GEO were summarized at the gene level, then the gene expression profiles of 10,080 genes evaluated in both the public datasets and expressed in the RNA-seq experiments was considered for the integrative analysis using R and Bioconductor packages (https://www.R-proiect.org/)⁶⁹. Data have been quantile normalized using limma⁶⁶ and samples clustered using hierarchical clustering algorithm with Spearman correlation based distance in R Principal Component Analysis (PCA) has been performed on scaled data using⁷⁰. Uniform Manifold Approximation and Projection (UMAP) has been done on the integrated dataset analyzed with Seurat⁷¹. Ten PCA components have been used in graph-based clustering, evaluated at different resolutions to identify stable clusters, then fixed at the value of 1 and represented in a bidimensional UMAP plot.

**Data availability.** RNA-seq data have been deposited in the GEO public repository and are accessible through the accession number GSE129746.

**Extraction of the lipidic and polar fractions from tumor tissues.** Frozen tissue of LLC-Mock, LLC-SULT2B1b and LLC+PFM037 tumors (n = 5 tumors/group) were cut to an appropriate size (mean weight: 10 mg) and placed in micro centrifuge tubes. They were resuspended in 170 µL of H₂O and 700 µL of methanol and sonicated for 60 sec. Then, 350 µL of chloroform were added and mixed the samples on an orbital shaker in ice for 10 minutes. After that, 350 µL of H₂O/chloroform (1:1, v/v) were added to each suspension and centrifuged at 4000 rpm for 10 minutes at 4°C. Thereafter, the aqueous (polar) and lipidic (non-polar) phases were collected separately, transferred to a glass vial and evaporated.

**¹H NMR spectra acquisition and data analysis.** All the lipidic and polar samples were analyzed using Nuclear Magnetic Resonance (NMR). The lipidic fractions were dissolved in 700 µL of deuterated chloroform (CDCl₃) and 0.03 volume percentage of tetramethylsilane (TMS) and inserted in NMR tubes whereas the polar fractions were dissolved in 630 µL of PBS-D₂O with the pH adjusted to 7.20, and 70 µL of trimethylsilylpropanoic acid (TSP) (1% in D₂O). TMS and TSP were used as the internal standards. A 600-MHz Bruker Avance spectrometer with a TCI cryoprobe was used to acquire ¹H spectra on all the lipidic and polar fractions at 300 K for 512 transients using the excitation sculpting pulse sequence to suppress water resonance in the case of polar fractions. All ¹H NMR spectra were manually phased and baseline-corrected and referenced to the CH₃ resonance of TSP or TMS at 0 ppm. The spectral 0.50-8.60 ppm region of ¹H-NMR spectra was integrated in buckets of 0.04 ppm by AMIX package (Bruker, Biospin, Germany). In details, inventors excluded, in the case of the polar spectra, the water resonance region (4.5-5.2 ppm) during the analysis and normalized the bucketed region to the total spectrum area using Pareto scaling by MetaboAnalyst v4.0 tool ⁷². The partial least squares-discriminant analysis (PLS-DA) algorithm was applied to explain the maximum separation between the defined class samples in the data. PLS-DA is a multivariate regression method that extracts linear relationships from two data block, X (NMR bucket variables) and Y (information data), by reducing the structured noise and highlighting the maximum correlation between the two matrices. Score, Loading and Variable Importance in Projection (VIP) Plots were used to highlight and assess the role of X-variables (NMR signals) in the classification models and, hence, to prioritize the discriminating peaks for identification. In Loading and VIP plots the top 10 more different NMR signals obtained comparing the different groups are shown. Metabolite assignments were based on the comparison of chemical shifts and spin-spin couplings with reference spectra present in the human metabolome database (HMDB)⁷³. The normalized proton signals of the statistically significant metabolites were reported by box-plot graphs.

**Tumor challenge experiments.** C57BL/6 mice were injected subcutaneously with LLC-Mock (1 ×10⁶), LLC-SULT2B1b (1 ×10⁶), RMA (2 ×10⁵), or B16F1-OVA (2 ×10⁵) tumor cells. Mock- and SULT2B1b-transduced tumor cells have been described in^{7,74}. Tumor size was evaluated by measuring perpendicular diameters by a caliper every 2-3 days. Data are reported as the average tumor volume ± SD. In selected experiments tumors were injected in *Ccr2*^{*-*/*-*}mice. Tumor challenge experiments in *Lxrαβ*^{*-*/*-*} bone marrow chimera mice. To establish bone marrow chimera mice, lethally irradiated (11 Gy) C57BL/6 mice were transplanted with the bone marrow of *Lxrαβ*^{*-*/*-*} or WT mice (5 ×10⁶ bone marrow cells/mouse). Six-eight weeks later, chimera mice were challenged with LLC and evaluated tumor size measuring perpendicular diameters by a caliper. *Lxrαβ*^{*-*/*-*} genotype was carried out by PCR on blood cells before tumor challenge. During the reconstitution phase, transplanted mice were treated with enrofloxacin for 15 days (7.5 mg/150 µl of Baytril 5% solution in 300 ml of drinking water) following the indication of the veterinary of the specific-pathogen-free animal facility.

**Treatment of tumor-bearing mice with LXR antagonists alone or with immunotherapy.** Tumor-bearing mice were treated with 25-HC-3S or PFM037 6-7 days after tumor challenge. After 6-7 days, mice were randomly assigned to either treatment or vehicle groups. Mice were injected with 300 µg of either compound given i.v. for 5 days/week. For liver metastasis studies, eight-to ten-week-old male C57BL/6 mice were injected through the superior mesenteric vein, with 7×10⁴ GFP-expressing MC38 cells (H-2b, C57BL/6-derived, MC38GFP) as previously described^{37,38}. For mesenteric vein injections, deep anesthesia was induced by isoflurane inhalation (5% induction and 2% for maintenance in 2 1/min oxygen). The indicated number of MC38-GFP cells was injected into the superior mesenteric vein using a 29G needle and to prevent excessive bleeding, vein puncture was compressed with a sterile and absorbable hemostatic gauze (TABOTAMP^{®}). The peritoneum and skin were sutured with silk 4.0 and 7 mm wound clips as described³⁸. In selected experiments, anti-PD-1 mAb (clone RMP1-14, In vivo BioXcell) 250 µg/mouse every three days were used, alone or in combination with PFM037. B16F1-OVA-bearing mice were treated with 400 µg of PFM037 for 5 days/week. For controls of mAb experiments, mice were injected with 250 µg of rat IgG2a isotype control antibody. For adoptive transfer studies, NOD-SCID mice challenged with LLC-OVA were treated with PFM037 (300 µg i.p. 3 days/week) 6 days after tumor injection. Three days later, mice were randomly assigned to either treatment or vehicle groups and infused i.v. with OT-I T cells (2×10⁶ OT-I/mouse) activated and expanded *in vitro* for 3-4 days with IL2 (20 IU/ml), IL27 (20ng/ml) and MHC-I-restricted SIINFEKL (257-264) OVA peptide (2 µg/ml) in RPMI 10% FBS + 50 µM β-ME. C57Bl/6 mice challenged with B16F1-OVA were treated with PFM037 (400 µg i.p. 3 days/week) 6 days after tumor injection. Three days after, mice were randomly assigned to either treatment or vehicle groups and infused i.v. with OT-I T cells (2×10⁶ OT-I/mouse) activated *in vitro* for 7-8 days with IL2 (20 IU/ml) and MHC-I-restricted SIINFEKL (257-264) OVA peptide (2 µg/ml) in RPMI 10% FBS + 50 µM β-ME. Tumors were collected and measured 14 days after tumor challenge. Different from LLC and RMA tumor models, 400 µg of PFM037 were used to treat B16F1-OVA-bearing mice, as 400 µg of PFM037 induced an antitumor activity much stronger than 300 µg of PFM037. For the liver metastasis model, given the complexity of the model it was started directly with 400 µg of PFM037 to minimize the number of mice to use.

**Blocking experiments with anti-CCR2 mAb.** Mice bearing 7 day-LLC-SULT2B1b tumors were treated every day i.p with 20 µg anti-CCR2 antibody³⁴ or rat IgG control antibody. Mice were sacrificed 12 days after tumor injection and analyzed for tumor weight and for the content of tumor-infiltrating monocyte-DCs

**Single cell RNAseq** data **analysis.** Two published datasets were considered in this study: E-MTAB-6149 and E-MTAB-6653 for lung tumor microenvironment and GSE120575 for CD45⁺ infiltration in melanoma. For lung dataset, tSNE projections were obtained uploading loom files in SCope focusing first on all cell types profiled and then only on myeloid clusters. Moreover, the expression of the cholesterol synthesis signature genes (*FADS1*, *FADS2, HMGCR, FDFT1, FDPS, GGPS1, HMGCS1, DHCR7, DHCR24, GYP51A1, HSD17B7, IDI1, MVK, MVD, NSDHL, MSMO1, SCD5, SCD, TM7SF2, SQLE, LSS, ABCA1, FAS, SULT2B1*) was investigated at population level using normalized-UMI counts only for tumor samples, focusing first on immune-infiltrating population (B cells, T cells and myeloid cells) and then on tumor associated clusters of myeloid cell type (clusters 2, 3, 4 and 12). The melanoma dataset was processed retrieving normalized gene expression data, cluster and patient information from GEO database. Then the expression matrix was imported and processed in Seurat package only for data visualization. Cluster and patient information (response to treatment with immune checkpoint blockers) was added to the dataset using "AddMetaData" function. Also in this case, the dataset was investigated for the expression of the cholesterol synthesis signature genes considering both the percentage and level of expression of the cells belonging to a specific cluster. Wilcoxon rank sum test has been adopted with FindMarkers function (logfc.threshold = 0.5, min.pct = 0.2, only.pos = TRUE) to verify if some cholesterol synthesis signature genes could be differentially expressed between different clusters of interest.

**Cell lineage tracing experiments and in vivo cell proliferation assays.** Monocytes were purified from the bone marrow of C57Bl/6 Ly. 1 (CD45.1) mice using the Monocyte Isolation Kit (Miltenyi). The enriched population contained >95% of Ly6C^{high} monocytes. 1.5-2.0 ×10⁶ purified Ly6C^{high} monocytes were infused i.v. into *Ccr2*^{*-*/*-*} CD45.2 mice bearing 3-day established LLC-Mock, LLC-SULT2B1b or LLC-mCherry tumors. Twenty-four and 72 hours later, mice were sacrificed, and tumors evaluated for weight and for the content of monocyte-DCs. For cell proliferation experiments, *ccr2*^{*-*/*-*} mice (CD45.2⁺) were injected with 5 ×10⁶ tumor cells. After 3 days, mice received CD45.1⁺ monocytes. Twenty-four hours later, mice were injected i.p. with 1mg/mouse of EdU. Four hours later, mice were sacrificed, tumor collected and digested as reported above. Cells were then stained, run by FACScanto (BD) and analyzed with FlowJo software. For the analysis of draining lymph nodes, *ccr2*^{*-*/*-*} mice (CD45.2⁺) were injected with 5 ×10⁶ tumor cells. After 3 days, mice received CD45.1⁺ monocytes and 48 hours later lymph nodes were collected and treated as reported in *immunofluorescence staining and confocal analysis* section.

**Antigen presentation assays.** Splenic OT-I or OT-II were purified with CD8a and CD4 (L3T4) microbeads (Miltenyi), respectively. Enriched OT-I or OT-II were labeled with CFSE (5 µM) and plated at a density of 1×10⁶/ml. Monocyte-DCs and Ly6C^{low} cells were FACS-purified from 10-day established LLC-SULT2B1b tumors and loaded with 10µg/ml of OVA-specific MHC-I and -II peptides to stimulate OT-I and OT-II T cells, respectively. Then, 1 ×10⁴ monocyte-DCs or Ly6C^{low} cells were co-cultured with 1 ×10⁵ OT-I or OT-II in RPMI complete medium. Cells were cultured at 37°C for 3 days and analyzed by flow cytometry for CFSE dilution. Supernatants collected 3 days later, were tested by ELISA to measure the content of mouse IFN-γ in agreement with manufacturer's instructions. T cell proliferation was expressed as proliferation index and CFSE-diluted cells. Proliferation index was analyzed and calculated by FlowJo software. OT-I and OT-II CFSE-diluted cells were presented as the percentage of OT-I and OT-II cells undergoing multiple rounds of proliferation. The percentage of CFSE-diluted OT-I and OT-II cells following activation with peptide-loaded Ly6C^{low} cells is relative to the percentage of CFSE-diluted OT-I and OT-II cells activated with peptide-loaded monocyte-DCs (set as 100%).

**Reporter assay for nuclear receptor transcriptional activity.** Human embryonic Kidney 293 cells (American Type Culture Collection) were cultured in Dulbecco's Modified Eagle's medium containing 10% of fetal bovine serum at 37°C in humidified atmosphere of 5% CO₂. HEK293 cells (4×10⁴ cells per well) were transiently transfected in 48 well plate with the reporter plasmids pMH100X4-TK-luc (100 ng/300µl), Renilla (22 ng/300µl) together with 100 ng/300µl of pCMX-Gal4-LXRα or pCMX-Gal4-LXRβ plasmids using X-tremeGENE 9 DNA Transfection Reagent (Roche). Six hours after transfection, the cells were treated with the appropriate compound for 24 hours. Luciferase activities were analyzed by luciferase Dual Reporter Assay Systems (Promega) according to the manufacturer's protocol. GAL4-LXRs, and TK-MHC100-luc plasmids were described in⁷. For mammalian two-hybrid assays the human liver cell line Hhu7 (American Type Culture Collection) was transiently transfected with the reporter plasmids p5xUAS-tk-Luc (100 ng/300µl) together with pGAL4-RAP250-Del4 (133 ng/300µl), described in³⁹, and either of pVP16-LXRα-LBD or pVP16-LXRβ-LBD plasmids (133 ng/300µl), described in⁴⁰, using the jetPrime transfection reagent (Polyplus). Six hours after transfection, the cells were treated with the appropriate compound for 40 hours. Luciferase activity was analyzed as described above and protein content spectrophotometrically using Coomassie Plus Protein assay reagent (Thermo Scientific). **Quantitative Real-Time-PCR.** U937 cell line was differentiated in foam macrophages with phorbol 12-myristate 13-acetate (PMA) 10 ng/ml (Sigma) for 72 hours at 37°C in RPMI 10% FBS. At day 3 T0901317 (10 µM) or different amount of PFM037 (50, 25 and 10 µM) were added for 16 hours. Total RNA was purified by TRIZOL (Invitrogen). Reverse transcription was performed by MLV-reverse transcriptase (Promega). QPCR was performed using SYBR Green Master Mix (Applied Biosystems) and real-time PCR (Viia 7 Real Time PCR System, Applied Biosystems). PCR reactions were done in triplicate. The comparative Ct method was used to quantify transcripts that were normalized for human GAPDH. The following primer pairs were used:
*GAPDH-F* 5'-ACA TCA TCC CTG CCT CTA CTG-3' (SEQ ID No. 1);
*GAPDH-R* 5'-ACC ACC TGG TGC TCA GTG TA-3'(SEQ ID No. 2);
*ABCA1-F* 5'-CCA GGC CAG TAC GGA ATT C-3' (SEQ ID No. 3);
*ABCA1-R* 5'-CCT CGC CAA ACC AGT AGG A-3' (SEQ ID No. 4);
*SREBP-1c*-*F* 5'-GGC GGG CGC AGA TC-3' (SEQ ID No. 5);
*SREBP-1c-R* 5'-TTG TTG ATA AGC TGA AGC ATG TCT-3' (SEQ ID No. 6);
*FAS F* 5'-ACA GCG GGG AAT GGG TAC T-3'(SEQ ID No. 7);
*FAS R* 5'-GAC TGG TAC AAC GAG CGG AT-3' (SEQ ID No. 8);
*SCD1-F* 5'-TTC AGA AAC ACA TGC TGA TCC TCA TAA TTC-3' (SEQ ID No. 9);
*SCD1-R* 5'-ATT AAG CAC CAC AGC ATA TCG CAA GAA AGT-3'(SEQ ID No. 10)

**Blood Plasma Chemistry Analysis.** For all in vivo experiments mouse blood samples were collected via cardiac puncture and isolated plasma was analyzed by the COBAS c311 system (Roche) for plasma lipids.

**Statistics.** Sample size and power were calculated through formal analyses capable of defining a priori the extent of the underlying effect using the G*power 3.1 software. The number of animals per experimental group considers the need to reduce the use of animals to the minimum to obtain statistically significant results for the objectives of the study. Consistently with the underlying experimental design, a type I error equal to 5% (α=0.05) was assumed and a power equal to 80% (1-β error prob=0.80). In the various comparisons a Student's t test was applied evaluating the differences between two independent groups or an ANOVA test evaluating the differences among three or more groups. Data are expressed as mean ± s.d. and were analyzed for significance by ANOVA with Dunnet's, Bonferroni's or Tukey's multiple comparison test, or by Student's t test. Statistical comparison of overall survival was performed by the log-rank test. The analysis was performed with Prism software.

### RESULTS

**The oxysterol-inactivating enzyme SULT2B1b promotes intratumor accumulation of CD3⁺CD8⁺ T-cells and monocyte-derived antigen presenting cells (APCs).** Inventors analyzed the microenvironment of the mouse Lewis Lung Carcinoma (LLC) expressing or not SULT2B1b and observed an increase in CD3⁺ T cells infiltrating LLC-SULT2B1b tumors (Fig. 1A-C). Higher percentages of CD8⁺IFN-γ⁺ and CD4⁺IFN-γ⁺ T cells (Fig. 1D, E) were detected, which paralleled a better control of tumor growth in mice bearing LLC-SULT2B1b tumors (Fig. 1F). Of note, LLC-SULT2B1b and LLC-Mock grew in a similar manner *in vitro,* and *in vivo* in NOD-SCID mice (Fig. 10A and 16). Thus, indicating that tumor growth control is mediated by the activity of SULT2B 1b on immune cells infiltrating tumors. Since the increased number of T-cells frequently associates with high numbers of tumor-infiltrating DCs^{21,22}, the presence and number of CD11c⁺ cells infiltrating SULT2B1b- and Mock-LLC tumors was evaluated by confocal microscopy analysis. Higher numbers of both CD11c⁺CD11b⁺ cells (Fig. 1G) and DC-T cell clusters were detected in LLC-SULT2B1b tumors (Fig. 1H, I). To better define the subsets of DCs infiltrating SULT2B1b- and Mock-LLC tumors, extensive FACS analysis was performed. Twelve days after tumor challenge, tumor-bearing mice were sacrificed and tumor weights (Fig. 2A) and tumor-infiltrating DCs (Fig. 2B, C) were analyzed, the latter based on the expression of CD45, CD11c, CD11b, CD103, Ly6C and MHC-II markers. Different numbers of classic CD11c⁺CD103⁺ cDC1 between SULT2B1b- and Mock-LLC tumors (Fig. 2B) were not observed. Instead, under SULT2B1b perturbation increased numbers of Ly6C^{high}CD11c⁺MHC-II+ cells were observed (Fig. 2C and Fig. 10B, C), resembling monocyte-derived DCs as suggested by the expression of CD11b, Sirp-α, Sca-1, CCR2, CD209a and PD-L2 markers (Fig. 10D)^{16,23}. The intratumor number of these monocyte-derived DCs (hereafter referred as mono-DCs) increased over time and correlated with a better control of tumor growth (Fig. 11A). The increase in a heterogeneous population of CD11b⁺Ly6C^{low} cells (hereafter referred as Ly6C^{low} cells) infiltrating LLC-SULT2B1b tumors was also observed, some of them expressing CD11c and MHC-II molecules and resembling inflammatory monocytes and/or macrophages (Fig. 11B). Functionally, FACS-purified mono-DCs elicited OVA-specific naive OT-I and OT-II T cells more efficiently than Ly6C^{low} cells (Fig. 11C-E), suggesting a primary role played by mono-DCs in eliciting effective antitumor immune responses. Because mono-DCs share similar phenotypes as the inflammatory cDC2 (inf-cDC2)²⁴, were analyzed and compared cDC2 and Inf-cDC2 in SLTLT2B1b- and Mock-LLC-bearing mice, based on the expression of CD45, CD11c, CD11b, Ly6C, CD26, CD64, MAR-1 and MHC-II markers (Fig. 11F-H). As previously shown, tumor weights (Fig. 2D) and numbers of mono-DCs (Fig. 2E) were lower and higher in LLC-SULT2B1b tumors, respectively. The numbers of tumor-infiltrating CD26⁺CD64⁺MAR-1⁻ cDC2 and CD26⁺CD64⁺MAR-1⁺ inf-cDC2²⁴ were instead very low and similar between LLC-Mock and LLC-SULT2B1b (Fig. 2F, G). These results prompted to perform RNA-seq experiments of mono-DCs and Ly6C^{low} cells isolated from SULT2B1b- and Mock-LLC tumors to obtain deeper molecular clues (Fig. 12A, B). Ly6C^{high}CD11c⁺MHC-II⁺ mono-DCs from both Mock- and SULT2B1b-tumors expressed transcripts peculiar of mono-DCs, such as *Cd209a, Nos2, C5ar1, Cx3cr1* and *FcyRI* (Fig. 12C)^{23,25}. Of note, Ly6C^{low} cells isolated from SULT2B1b-LLC tumors expressed higher levels of transcripts encoding MHC-II, CD74 and CIITA molecules (Fig. 12B). Both subsets (*i.e.* monocyte-DCs and Ly6C^{low} cells) isolated from SULT2B1b-LLC tumors, expressed high levels of *Cxcl9* and *Cxcl10* transcripts, which encode chemokines involved in the recruitment of antitumor effector T cells²⁶ (Fig. 12D, E) and lower levels of the *Cebpb* transcripts, whose increased expression has been associated with the immune suppressive abilities of bone marrow-derived, tumor-infiltrating myeloid cells²⁷ (Fig. 12F, G). The transcription factor *Irf8* was expressed at high levels by both subsets isolated from Mock- and SULT2B1b-LLC tumors, whereas *Irf4,* which regulates monocyte-DC differentiation²⁸, was expressed at higher levels in mono-DCs isolated from SULT2B1b-LLC tumors as compared to cells isolated from Mock-LLC (Fig. 12F). Then, transcriptomic results were compared with publicly available datasets, particularly TNF- and NOS-producing DCs (Tip-DCs)²⁵, bone marrow-derived and blood monocytes²³, skin-derived macrophages, cDCs and monocyte-DCs²⁹, and lung-derived cDC1, cDC2, inf-cDC2 and monocyte-derived APCs²⁴. Ly6C^{high}CD11c⁺MHC-II⁺ mono-DCs as well as Ly6C^{low} cells isolated from SULT2B1b- and Mock-LLC tumors resulted to be closer to the Tip-DCs (Fig. 3A). By UMAP analyses 4 clusters were identified grouping cells isolated from different organs or pathologic conditions sharing some program commonalities (Fig. 3B). Cluster 2 grouped mono-DCs and Ly6C^{low} cells with Tip-DCs²⁵, both groups isolated from tumors. Cluster 0, cells isolated from lung independently of their state of activation²⁴. Cluster 0 also encompassed DC and monocytes isolated from lymph nodes. Cluster 1 grouped cells isolated from skin independently of their state of activation²⁹, and Cluster 3 monocytes isolated from blood or bone marrow²³. These results, also confirmed by the hierarchical clustering analysis (Fig. 3C), indicate that cell identities account for the tissue or the pathologic condition where the cells come from (Fig. 3B), as previously demonstrated for macrophages isolated from different organs^{30,31}.

To get deeper insights into the pathways modulated by SULT2B1b-perturbed mono-DCs and Ly6C^{low} cells, gene set enrichment analyses (GSEA) of the transcriptomic data by Hallmarks were performed, which highlighted the enrichment of specific metabolic pathways (Fig. 13A, and Tables 1 and 2 below).

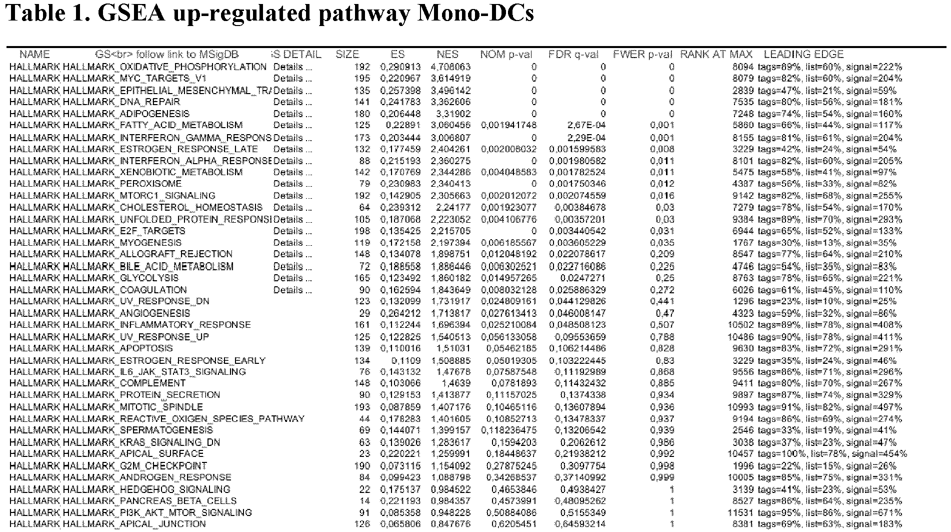

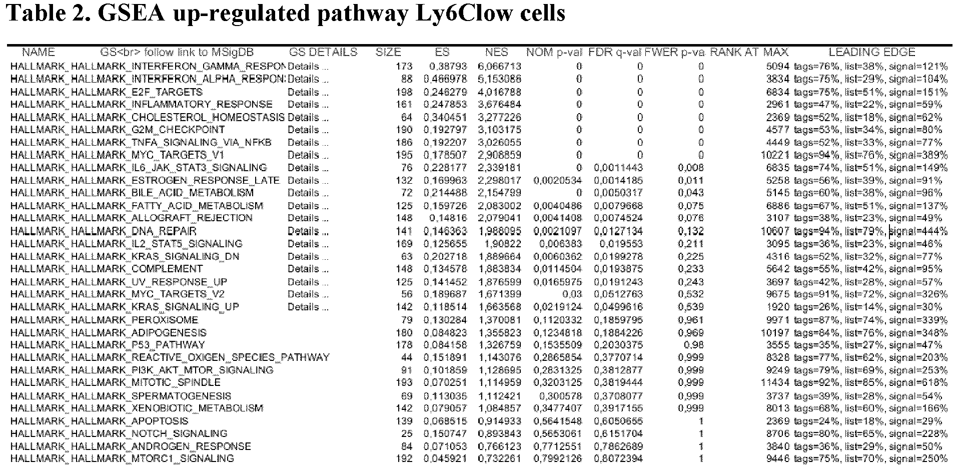

Oxidative phosphorylation, adipogenesis, fatty acid metabolism and cholesterol metabolism were significantly up-regulated in mono-DCs from SULT2B1b-LLC tumors (Fig. 13A). Cholesterol and fatty acid pathways were also up-regulated in Ly6C^{low} cells (Fig. 13B). The up-regulation of cholesterol pathway in cells from LLC-SULT2B 1b tumors was also confirmed using a previously reported cholesterol synthesis gene signature³² (Fig. 13C). It was also observed the down-regulation of the LXR target gene *Abcal*³³ in the cells isolated from LLC-SULT2B1b tumors (Fig. 13D), in accordance with previous *in vivo* data¹¹.

Altogether, these results indicate that under SULT2B1b manipulation there is an intratumor increase of activated monocyte-derived cells correlating with a better control of tumor growth. **Under SULT2B1b perturbation tumor-recruited monocytes differentiate into DCs.** The above-reported results suggest that in certain conditions, *i.e.* under SULT2B1b perturbation, the immunostimulatory ability of monocyte-derived DCs can be exploited to induce effective antitumor responses. To validate this hypothesis, tumor challenge experiments were performed by systemically depleting CCR2⁺ monocytes, using the monoclonal antibody MC-21³⁴, or by blocking the egress of monocytes from the bone marrow towards inflammatory sites taking advantage of CCR2-deficient mice³⁵, under SULT2B1b manipulation. Mice bearing SULT2B 1b-tumors treated with an anti-CCR2 depleting mAb³⁴ for five consecutive days were infiltrated by lower numbers of mono-DCs, identified by the expression of Ly6C, CD11b and Sca-1 markers, and failed to control tumor growth (Fig. 14A, B). In accordance with these results, *Ccr2*^{*-*/*-*} mice failed to control SULT2B1b-tumors as compared to *wild type* mice (Fig. 14C). Ly6C^{high}CD11c⁺MHC-II⁺ mono-DCs were virtually absent in SLTLT2B1b-tumors grown in *Ccr2*^{*-*/*-*} mice (Fig. 14D). Ly6C^{low}CD11c⁺MHC-II⁺ cells were lower in SULT2B1b-tumors grown in *Ccr2*^{-/-} mice as compared to *wild type* mice (Fig. 14E). The number of CD11c⁺CD103⁺ cDCs were not significantly different among the group of tumors grown in *Ccr2*^{*-*/*-*} and *wild type* mice (Fig. 14F, G). These experiments suggest that increasing the intratumor number of monocyte-derived antigen presenting cells improves the antitumor immune responses.

Then, inventors investigated whether the increased numbers of mono-DCs in SLTLT2B1b-tumors were due either to an enhanced recruitment of monocytes or to their intratumor differentiation. To address this issue, cell lineage tracing experiments were performed by using congenic CD45 mice (Fig. 4A). CD45.2⁺ *Ccr2*^{*-*/*-*} mice bearing 3-day-established SULT2B1b- or Mock-LLC tumors were injected with bone marrow-purified CD45.1⁺Ly6C⁺ monocytes. One and three days later, tumors were collected and analyzed for the content of mono-DCs and Ly6C^{low}CD11c⁺MHC-II⁺ cells. The percentage of CD45.1⁺ cells recruited to SULT2B1b- and Mock-LLC tumors was similar (Fig. 4B), while the number of mono-DCs was higher in LLC-SULT2B1b both at 24 and 72 hours (Fig. 4C). The number of Ly6C^{low}CD11c⁺MHC-II⁺ cells was higher only at 24 hours (Fig. 4D). At 72 hours the number of intratumor mono-DCs was greatly reduced, suggesting their possible migration to draining lymph nodes. These experiments indicate that under SULT2B1b perturbation, there is an increased rate of monocytes undergoing monocyte-to-DC differentiation. Based upon the results obtained at 72 hours, inventors analyzed whether mono-DCs generated in SULT2B1b-perturbed tumors were able to migrate to draining lymph nodes, where antitumor immune responses occur. *Ccr2*^{*-*/*-*}CD45.2⁺ mice were challenged with SULT2B1b-LLC expressing the mCherry fluorescent protein, and five days later with CD45.1⁺ purified monocytes (Fig. 4E). Two days later inventors collected and analyzed by confocal microscopy draining lymph nodes, and visualized CD11c⁺CD45.1⁺ cells phagocytosing mCherry⁺ bodies (Fig. 4F). By FACS analysis of the cell suspension of draining lymph nodes it was provided evidence and quantified the percentage of CD45.1⁺ cells (Fig. 4G, H), also expressing Ly6C, CD11c and MHC-II molecules (Fig. 4I, J). Approximately 16% of CD45.1⁺Ly6C⁺CD11c⁺MHC-II⁺ cells were mCherry⁺, indicating that these cells were able to phagocytose tumor-associated proteins and migrate to draining lymph nodes (Fig. 4K), whereby directly¹⁸ or indirectly³⁶ activate effective antitumor responses.

**Sulfated oxysterols promote LXR-dependent monocyte-DC differentiation and antitumor responses.** Hypothesis that sulfated sterols, which are generated by SULT2B1b⁸, could condition the differentiation of tumor-recruited monocytes was investigated, by evaluating whether the administration of sulfated oxysterols could induce antitumor responses. Tumor-bearing mice were treated with the commercially available 25-hydroxycholesterol-3 sulfate (25-HC-3S) or with a newly developed 3-sulfate form of the LXR-inactive oxysterol 22S-hydroxycholesterol (22S-HC), namely PFM037, obtained by chemical sulfation of the 22S-HC. Treatments started 6-7 days after tumor challenge, when tumors are well established. Higher absolute numbers of tumor-infiltrating mono-DCs (Fig. 5A) was detected, as well as the delay of tumor growth (Fig. 5B) in both 25-HC-3S- and PFM037-treated LLC-bearing mice. Of note, LLC tumor cells treated *in vitro* with 25-HC-3S, PFM037 or vehicle grew in a similar manner (Fig. 10A). Higher numbers of tumor-infiltrating mono-DCs (Fig. 5C) and tumor growth control (Fig. SD) were also observed when mice bearing the RMA lymphoma were treated with 25-HC-3S or PFM037. Yet in this experimental setting, the absolute number of cDC2 and Inf-DC2 were respectively 15 and 30 times lower than mono-DCs (Fig. 5E-H). PFM037 treatment also delayed the growth of the melanoma B16F1-OVA (Fig. 5i), indicating that different tumor types could benefit from therapy based on sulfate oxysterol administration. Finally, PFM037 was tested in a model of liver metastasis formation following intramesenteric injection of the MC38-GFP colon cancer cells^{37,38}. Seven days after tumor challenge mice were treated with PFM037 or vehicle 3 days/week for 2 weeks. Twenty-one days after tumor challenge, mice were sacrificed, and liver analyzed (Fig. SJ, K). Mice treated with PFM037 developed much less tumor nodules than vehicle treated mice (Fig. 5L). In addition, the tumor area was reduced in PFM037-treated mice as compared to vehicle-treated mice (Fig. 5M). Of note, tumor-bearing mice treated with PFM037 showed reduced levels of plasma total cholesterol, triglycerides, not esterified fatty acids and HDL, whereas LDL did not change (Fig. 5N). Altogether, these findings indicate that sulfate oxysterols exogenously provided favor *in vivo* mono-DC differentiation and contribute to antitumor responses. Furthermore, these findings suggest a shared mechanism of mono-DC differentiation by SULT2B1b, 25-HC-3S and PFM037. It has been shown that SULT2B1b/SULT2B1b-derived products antagonize LXR activity^{11,8,7}. Therefore, inventors asked whether PFM037 was interfering with LXR activation/signaling. To address this issue, inventors set up different molecular assays. First, by luciferase-based reporter assays inventors demonstrated that PFM037, the new 3-sulfate form of 22S-HC obtained by its chemical sulfation, did not activate LXRα or β (Fig. 6A, B). Then, inventors tested the antagonism of LXRα or β activation induced by 22R-HC by using titrating amounts of 25-HC-3S, 22S-HC and PFM037 (Fig. 6C-F). 25-HC-3S and PFM037 antagonized the LXRα activity in a dose-dependent manner (Fig. 6C, D) with IC₅₀ of 12.65 and 5.18 µM, respectively. PFM037 also antagonized LXRβ activity with an IC₅₀ of 82.46 µM (Fig. 6E). Of note, 22S-HC did not antagonize LXR activity (Fig. 6F). Then, inventors tested by qPCR the capacity of PFM037 to activate the expression of the LXR target genes *ABCA1, SREBP1c, FASN* and *SCD1* in the U937 myeloid cells differentiated with PMA for 72 hours and then treated with T0901317 (10 µM) or with different concentrations of PFM037. As expected, no activation of *ABCA1* transcripts was found (Fig. 6G). Unexpectedly, the inhibition of *SREBP-1c, FASN,* and *SCD1* mRNA expression were observed (Fig. 6G). These results prompted to test PFM037 by using the mammalian two-hybrid system and inventors took advantage of the Del4 construct, which is from the NR coactivator RAP250/NcoA6 containing the first NR-box that is known to bind to agonist-bound nuclear receptors³⁹. Cells were transfected with Del4 plasmid together with either LXRα or LXRβ LBDs fused to the activating domain of VP16⁴⁰. The addition of 22R-HC induced luciferase activity, while the addition of PFM037 slightly inhibited the luciferase activity for LXRβ, thus suggesting that PFM037 antagonizes endogenous LXR ligands (Fig. 6H). In accordance with the luciferase assays reported above (Fig. 6C, D), PFM037 reduced the luciferase activity induced by 22R-HC (Fig. 6H). Altogether these results demonstrate that PFM037 is endowed with an LXR antagonistic activity. Moreover, the reduction of luciferase activity in the absence of agonists (Fig. 6H), as well as the reduction of *SREBP1c, FASN* and *SCD1* transcripts, suggest that PFM037 may behave as an inverse agonist of LXRs. To investigate the involvement of LXR in monocyte activation as well as in monocyte-to-DC programming and antitumor responses, inventors took advantage of *Lxrαβ*^{*-*/*-*} mice. *Wild type* and *Lxrαβ*^{*-*/*-*} bone marrow (BM) chimera mice were challenged with LLC tumor cells. *Lxrαβ*^{*-*/*-*} BM-chimera mice controlled LLC tumor growth better than LLC-bearing wild type chimera mice (Fig. 7A) and were infiltrated by a higher number of mono-DCs (Fig. 7B), suggesting a possible role exerted by LXR signaling in the differentiation of tumor-infiltrating monocytes. Similar experiments in *Lxrαβ*^{*-*/*-*} and wild type BM-chimera mice challenged with LLC tumor cells and treated with vehicle or PFM037 were performed. A better control of LLC growth by *Lxrαβ*^{*-*/*-*} BM-chimera mice treated with vehicle or PFM037 and by wild type BM-chimera mice treated with PFM037 were observed as compared to wild type BM-chimera mice treated with vehicle (Fig. 7C). Moreover, early after tumor injection a significant difference was noticed between mice treated with PFM037 (both *wild type* and *Lxrαβ*^{*-*/*-*} BM chimera) and those treated with vehicle (Fig. 7C), suggesting the co-existence of LXR-dependent and -independent mechanisms, exerted by PFM037 at different times. To investigate whether PFM037 promotes mono-DC differentiation *in vitro,* bone marrow-purified monocytes were cultured with M-CSF in the presence or in the absence of PFM037. Monocytes cultured with PFM037 expressed CD11c marker as early as after 48 hours of culture, with a smaller fraction of cells expressing MHC-II⁺ molecules (Fig. 7D, E). The involvement of LXR signaling was demonstrated by culturing *Lxrαβ*^{*-*/*-*} monocytes with M-CSF in the presence or in the absence of PFM037 (Fig. 7C, D). In this condition, it was observed the up-regulation of CD11c (Fig. 7D) and MHC-II (Fig. 7E) molecules also in the absence of PFM037. Of note, the up-regulation of CD11c was even higher in *Lxrαβ*^{*-*/*-*}monocytes treated with PFM037 (Fig. 7D). Altogether, these experiments indicate that SULT2B1b/LXR axis contributes to monocyte differentiation towards mono-DCs, which in turn contribute to tumor growth control.

**Analysis of the lipidomic/metabolomic landscape of SULT2B1b-perturbed and PFM037-treated tumors.** GSEA of SULT2B1b-perturbed mono-DCs (Fig. 13) and the molecular analyses of PFM037 prompted us to carry out un-targeted metabolomics/lipidomics profiling of SULT2B1b- and PFM037-perturbed tumors by Nuclear Magnetic Resonance (NMR) spectroscopy, aimed to identify metabolic changes and commonalities between SULT2B1band PFM037-perturbed tumors. Mock- and SULT2B1b-LLC tumors grouped into two different clusters in the score plot, indicating the presence of significant different levels of lipidic species between the two groups. It was observed a significant reduction in SULT2B1b-LLC tumors of proton signals related to cholesterol/cholesterol products, glycerophospholipids among them phosphatidylcholine and phosphatidylethanolamine, and polyunsaturated ω-3 fatty acids (Fig. 8A-C). Saturated fatty acids and triglycerides were instead enriched in SULT2B1b-LLC tumors (Fig. 8A-C). A similar profile was also observed in PFM037-treated tumors, as compared to vehicle-treated tumors (Fig. 8D-F). Notably, PFM037-treated tumors were enriched in proton signals of ω-6 fatty acid arachidonic acid and its derivatives, indicating an enrichment of lipids endowed with pro-inflammatory functions²⁰ (Fig. 8E, F). On the other hand, lower levels of proton signals of ω-3 fatty acid docosahexaenoic acid and its derivatives were detected, which lead to pro-resolving mediators (SPMs)²⁰ endowed with antiinflammatory capabilities (Fig. 8E, F). The analysis of the polar fractions (Fig. 15A-C) highlighted high levels of the proton signals of choline in SLTLT2B1b-LLC tumors, an observation strictly correlated with the low levels of the proton signals of phospholipids (Fig. 8B, C). Indeed, choline is an essential nutrient for all the cells and plays a role in the synthesis of the phospholipid components of cell membranes⁴¹. Furthermore, higher levels of the proton signals of glycine in SULT2B1b-LLC tumors (Fig. 15C) were observed, that could be correlated to the lower levels of the proton signals of cholesterol (Fig. 8B, C), as the glycine is linked to cholesterol transport in the bile acid metabolism pathway⁴². Similar results were also detected on the polar fractions of tumors treated with PFM037 (Fig. 15D, E), indicating that SULT2B 1b and PFM037 induce similar lipidomic and metabolomic changes in tumors.

**PFM037 synergizes with immunotherapy to enhance antitumor responses.** Inventors reasoned that PFM037 by increasing the intratumor number of monocyte-derived APCs could synergize with immunotherapy. Therefore, LLC-bearing mice were treated with PFM037 and anti-PD-1 mAbs and it was observed a heightened control of tumor growth (Fig. 9A), and a prolonged overall survival with the combination therapy (Fig. 9B), suggesting that PFM037 could be used alone or in combination with immune checkpoint blockers (ICBs) (e.g. anti-PD-1 mAb) for cancer treatment. Moreover, a better control of tumor growth (Fig. 9C) was observed and a prolonged survival (Fig. 9D) of NOD-SCID mice bearing LLC-OVA tumors receiving the combination of PFM037 and the adoptive transfer of *in vitro* activated OT-I T cells. Finally, a better control of tumor growth (Fig. 9E) was observed in immunocompetent mice bearing the melanoma B16F1-OVA and treated with the combination of PFM037 and *in vitro* activated OT-I T cells. Thus, indicating that SULT2B1b/PFM037 treatments reprogram the hostile tumor microenvironment into an immune sensitive one.

Expression of LXR/cholesterol genes in myeloid cells infiltrating human tumor samples predicts tumor response to ICBs. To find molecular correlates in human tumors inventors evaluated the expression and clinical significance of LXR/cholesterol synthesis genes in myeloid cells infiltrating human tumors by taking advantage of recently published scRNA-seq datasets of non-small cell lung cancer (NSCLC)⁴³ and melanoma samples, the latter from ICB-responder and non-responder patients⁴⁴. Inventors detected an increased expression of *ABCA1* and *SCD* transcripts, as well as cholesterol synthesis genes, such as *HMGCR,* in myeloid cells infiltrating NSCLC samples (Fig. 9F). The expression of *ABCA1* and *SCD* mainly in tumor-associated macrophages (cluster 2) was observed (Fig. 8G), while *HMGCR* was mainly expressed in DCs (Fig. 9G). To correlate myeloid cell infiltration, LXR/cholesterol synthesis pathways and response to immunotherapy, it was interrogated the scRNA-seq dataset of melanoma samples of ICB-responder and non-responder patients⁴⁴ and observed a statistically significant enrichment of *ABCA1* and *SCD* transcripts in monocytes/macrophages of patients not responding to ICBs (Fig. 9H and Table 3 below) (*ABCA1* NR vs R = 1.52; *P* = 4.29 ×10⁻⁸; *SCD* NR vs R = 1.80; *P* = 2.93 ×10⁻⁵; Wilcoxon rank sum test).

**Table 3. Tumor-infiltrating monocytes/macrophages NR vs R**

| | Avg. logFC | Pct. 1 (%) | Pct. 2 (%) | P val adj |
|---|---|---|---|---|
| *ABCA1* | 1.521254 | 35.1 | 5.9 | 4.29 x10⁻⁸ |
| *SCD* | 1.803138 | 29.2 | 5.2 | 2.93 x10⁻⁵ |

Of note, the enrichment of *ABCA1* transcripts in monocytes/macrophages of non-responders turned out to be the most significant among all melanoma-infiltrating CD45⁺ cells (T lymphocytes, B lymphocytes, etc.; *ABCA1* NR vs All = 1.40; *P* = 5.79 ×10⁻²¹²; Wilcoxon rank sum test) (Fig. 16 and Table 4).

**Table 4.Tumor-infiltrating DCs R vs NR**

| | Avg. logFC | Pct. 1 (%) | Pct. 2 (%) | P val adj |
|---|---|---|---|---|
| *HMGCS1* | 2.377323 | 31.9 | 26.3 | 1 |
| *MVD* | 1.254463 | 23.4 | 17.72 | 1 |

The enrichment of some cholesterol synthesis genes, such as *HMGCS1,* was also observed in DCs of ICB responder patients, although it was not statistically significant (Fig. 9H and Table 5).

**Table 5. Tumor-infiltrating monocytes/macrophages NR vs all**

| | Avg. logFC | Pct. 1 (%) | Pct. 2 (%) | *P* val adj |
|---|---|---|---|---|
| *ABCA1* | 1.404119 | 35.1 | 7.8 | 5.79 x10⁻²¹² |
| *SCD* | 0.68751 | 29.2 | 8.8 | 1.23 x10⁻¹⁰⁵ |

These results indicate that tumor-infiltrating myeloid cells (monocytes and DCs) differentially express genes associated to LXR and cholesterol synthesis pathways and that the over-expression of transcripts associated to LXR activation in monocytes/macrophages correlates with a worse clinical outcome to ICBs. Altogether, these results indicate that SULT2B1b and PFM037 perturbation reprogram the tumor microenvironment generating an immune contexture, *i.e.* monocyte-derived APCs (Fig. 17), contributing to antitumor immune responses and synergizing with immune checkpoint inhibitors as well as adoptive cell therapy.

### PFM037 and T cell differentiation and function

To evaluate whether PFM037 induces functional and phenotypic changes of *in vitro* activated OT-T cells, OT-I T cells were activated in the presence of anti-CD3/CD28 coated beads and IL-2. Forty-eight hours later the cells were differentiated and expanded by IL-7/IL15 (5ng/ml both cytokines) with or without PFM037. Ten, 12 and 15 days later, FACS analysis was performed to evaluate their differentiation status. OT-I T cells expanded with PFM037 progressively lost CD39 and CD69 markers, while progressively acquiring CD62L and CD44^{low} markers, a phenotype associated to T Stem Cell Memory (T_{SCM}) cells (Krishna, Lowery et al. Science. 2020 11;370 (6522):1328-1334*)* (Fig. 18). T cells expanded by IL7 and II,15 in the presence of PFM037 also expressed at higher levels the sternness markers Sca1, Tcf1 and CXCR3. Based on these results, the antitumor activity of OT-I T cells expanded by IL-7/IL15 with PFM037 *in vivo* was tested. First, OT-I T cells expanded with IL-2 (effectors) or IL-7/ILIS with or without PFM037 (memory T cells) were injected in mice bearing 7-day established B16F1 melanoma expressing OVA. Tumor-bearing mice injected with OT-I T cells expanded by IL-7/IL15 with PFM037 controlled tumor growth significantly better than mice injected with OT-I T cells expanded with IL-2 or IL-7/IL15 without PFM037 (Fig. 19). Then, mice bearing MC-38-OVA-liver metastases were treated with vehicle, or OT-I T cells expanded with IL-2 (effectors), or IL-7/IL15 with or without PFM037. Tumor-bearing mice injected with vehicle were all dead 40 days after tumor injection. Twenty percent of tumor-bearing mice treated with OT-I T cells expanded with IL-2 were alive at 60 days, whereas 50% and 80% of tumor-bearing mice treated with IL-7/IL15 with or without PFM037, respectively, were alive at 60 days (Fig. 20). These experiments suggest that T cells expanded in the presence of PFM037 express higher levels of markers associated to memory/stem cell memory T cells and are endowed with a stronger antitumor potential. Finally, inventors performed *in vitro* experiments by using human T cells activated in the presence of anti-CD3/CD28 coated beads and then expanded by IL-7/IL15 with or without PFM037 (5 and 10 µM). T cells expanded in the presence of PFM037 acquired higher levels of markers associated to memory/stem cell memory T cells (Fig. 21), thus, suggesting that PFM037 can be used to augment T cell memory differentiation *in vitro* for adoptive transfer purposes.

### Analysis of the LXR antagonistic activity of PFM037.

In vitro experiments shown in Fig. 22 demonstrate that PFM037, *i.e.,* the sulfated form of the 22S-HC isomer, antagonizes LXRα and β activation induced by the 22R-HC isomer. Moreover, it demonstrates that the sulfated form of the 22R-HC (22(R)-hydroxycholesterol-3-sulfate sodium salt, herein also indicated as PFM036) is instead unable to antagonize LXRα and β activation. These results are unexpected because the sulfated form of the oxysterol 24S-HC is capable of antagonizing LXRα and β activation induced by the non-sulfated 24S-HC oxysterol. Moreover the *in vivo* analysis of mice bearing the lung tumor LLC, treated with PFM037, PFM009 or the combo treatmen shown in Fig. 23 demonstrate that the combo treatment with PFM037 and PFM009 (PFM013), the latter being a strong LXRα and β inducer, exerts a stronger antitumor effect as compared to the single treatments. PFM046 compound afforded similar in vitro and in vivo results, as demonstrated by the experiments in Fig. 24, confirming that PFM046, similar to what was observed for PFM037, antagonizes LXRα activation induced by the 22R-HC oxysterol and controls tumor growth in vivo.

### DISCUSSION

Histopathologic pictures characterized by low intratumor numbers of T cells and dendritic cells (DCs) frequently correlate with the failure of immune checkpoint blockers (ICBs) therapy in advanced cancer patients². This histopathologic condition often associates with high intratumor number of immature myeloid cells, as well as high levels of VEGF, M-CSF and IL-6, which are known to block the differentiation of cDC^{45,4}. Although the paralysis of the antigen presentation due to the blockade of cDC differentiation argues against the occurrence of antitumor immune responses in advanced cancer patients, the identification of *de novo* generated TCR clonotypes recognizing cross-presented antigens following ICB therapy⁴⁶, predicts residual activity of rare DCs not yet dysfunctional, or the presence of surrogate antigen presenting cells. Although recent studies have pointed out the key role played by resident cDCs, particularly cDC1, to inducing both CD8⁺ and CD4⁺ TAA-specific T cells^{47,36}, there are other studies demonstrating that monocyte-DCs can be a relevant source of antigen presenting cells (APCs), capable of priming antigen-specific T cells *in vivo*^{18,25,19,48}. Different mechanisms/factors, such as chemotherapeutic agents¹⁸, transcription factors¹⁹, retinoids⁴⁸, have been shown to favor the differentiation of monocyte-DCs. Here, inventors demonstrate that also the perturbation of the reverse cholesterol transport pathway (*i.e*., the LXR/oxysterol pathway), enhances intratumor monocyte-derived APC activation and differentiation (Fig. 17), which independently of their capacity to ultimately interact with antigen-specific T cells, induce effective antitumor immune responses, as shown by our experiments performed under CCR2 blockade. Circulating monocytes have been widely used in the past to obtain large numbers of human monocyte-DCs for the immunotherapy of advanced cancer patients⁴⁹. These cells, loaded with TAA and injected into patients induced suitable TAA-specific immune responses, which in some cases resulted in optimal antitumor responses^{50,49,51}. Inventors observed monocyte-DCs phagocytosing tumor-derived mCherry⁺ bodies in tumor-draining lymph nodes (Fig. 4F); however, it remains to evaluate whether these cells prime efficiently tumor-specific T cells either directly or indirectly. ICBs are standard of care for several tumor types (melanoma, NSCLC, head and neck cancers, etc.)¹, and adoptive cell therapy is rapidly emerging as a powerful immunotherapy strategy⁵². In our pre-clinical models, the concomitant administration of PFM037 and immunotherapy with either anti-PD-1 mAb or adoptive cell therapy enhanced the antitumor responses. Indeed, in both conditions inventors observed a prolonged survival of tumor-bearing mice treated with the combo therapies, indicating that the perturbation of the reverse cholesterol transport process improves the effects of immunotherapy by overcoming mechanisms of immune escape, such as the low intratumor number of APCs. These results are in accordance with bioinformatic analyses showing improved antitumor responses in melanoma patients with lower levels of LXR target gene transcripts, *i.e., ABCA1* and *SCD* (Fig. 9H). Therefore, the findings highlight the possibility to overcome resistance mechanisms related to the low number of tumor-infiltrating APCs and to augment the clinical activity of immunotherapy by manipulating LXR/SULT2B1b axis. The results of the GSEA showing a differential expression of metabolic pathways belonging to cholesterol and fatty acid metabolisms in monocyte-DCs and Ly6C^{low} cells isolated from LLC-SULT2B1b tumors, together with both the analysis of lipid levels in the blood of mice treated with PFM037 (Fig. SN) and previous results demonstrating that SULT2B1b interferes with LXR and lipid metabolism¹¹, enabled us to evaluate the lipidomic profiles of tumors perturbed by SULT2B1b or PFM037. PFM037 is a novel LXR antagonist, structurally and functionally analog to endogenous sulfate oxysterols. The comparative analysis of the effects exerted by SULT2B1b and PFM037 in the tumor microenvironment highlighted some commonalities, *i.e.,* the reprogramming of cholesterol/cholesterol products, phosphatidylcholine and phosphatidylethanolamine, as well as fatty acids endowed with inflammatory (arachidonic acid) or anti-inflammatory (docosahexaenoic acid) activities. Since arachidonic acid is the precursor of lipid mediators of the acute phase of inflammation (*i.e*., prostaglandins, leukotrienes, eoxins) while docosahexaenoic acid is an ω-3 FA with anti-inflammatory properties²⁰, our findings suggest that tilting the balance towards lipids mostly involved in the acute phase of inflammation may contribute to monocyte-derived cell activation and differentiation, including monocyte-DC differentiation. It remains to establish the type(s) of lipid mediators contributing to monocyte-derived cell activation and differentiation, given the heterogenous nature of lipids and the multitude of effects they exert depending on their nature⁵³. Authors of the present invention showed that the *in vitro* and *in vivo* effects induced by PFM037 are mainly dependent on LXR signaling, as indicated by *in vitro* differentiation experiments of *Lxrαβ*^{*-*/*-*} monocytes in the presence or in the absence of PFM037, and by tumor challenge experiments in *Lxrαβ*^{*-*/*-*} bone marrow chimera mice treated with vehicle or PFM037. However, in both experiments phenotypic and functional features were noticed predicting some LXR-independent effects by PFM037. *In vitro,* it was observed an increased number of *Lxrαβ*^{*-*/*-*}monocytes acquiring CD11c molecules when LXR-deficient monocytes were treated with PFM037 (Fig. 7D). *In vivo,* it was observed an earlier control of tumor growth when wild type and *Lxrαβ*^{*-*/*-*} chimera mice were treated with PFM037 (Fig. 7C). Further studies are needed to clearly investigate the role of LXR-dependent and -independent effects of PFM037 treatment. There is still debate on the role played by LXR on tumor growth, *i.e.* protumor vs antitumor effects. Molecular and cellular clues, such as the presence of two isoforms of the receptor, LXR-α and -β, which can be selectively expressed by tumoral and non-tumoral cell components of the tumor microenvironment, and the sensitivity of different tumor-infiltrating immune cells to LXR activation/repression, might therefore explain some contrasting results obtained by different groups. This work is for instance in accordance with studies showing that the pharmacologic inhibition of LXR by the inverse agonist SR9243, enhances dendritic cell activity that is suppressed by tumor-produced LXR ligands, and induces *in vivo* control of triple negative breast cancers (TNBC) through the stimulation of CD8⁺ T-cell cytotoxic activity^{54,55}. Interestingly, Flaveny and colleagues reported up-regulated levels of the LXR target gene *ABCA1* in myeloid cells (macrophages and dendritic cells) infiltrating human TNBC samples⁵⁵, an observation in agreement with our results in human lung and melanoma samples (Fig. 9F-H). While these studies point to the detrimental effects of LXR activation, other studies have instead shown effective antitumor responses when LXRs are activated *in vivo* by the administration of LXR agonists^{56,57}. These studies have reported the downregulation of the frequency of intratumor myeloid-derived suppressor cells (MDSCs) and increased CTL activity in response to high doses of LXR agonists⁵⁶, and the reduction of intratumoral regulatory T cells (Treg) depending on the downregulation of the Treg-attracting chemokine *Ccll7* by MHC-II^{high} tumor-associated macrophages in response to lower doses of LXR agonists⁵⁷. In the experimental settings, present inventors sought to fix some variables by transplanting tumors in *Lxrαβ*^{*-*/*-*} bone marrow chimera mice to rule out the possible interference of *Lxrαβ*^{*-*/*-*} non-hematopoietic stromal cells. Further studies are however needed to dissect in a systematic manner the complex role of LXRs in the tumor microenvironment, *i.e.* generating or taking advantage of LXR-deleted cell/tissue specific mice and by using LXR antagonists, such as PFM037, and selective LXR agonists⁵⁸. SULT2B1b perturbation induces SREBP2 activity¹² and therefore the intracellular accumulation of cholesterol in APCs. This pathway, by promoting the inflammasome-dependent IL-1β release⁵⁹, has been shown to lead to APC activation⁶⁰. Although at early times no difference was observed in IL-1β release after PFM037 treatment, the possibility that it may occur at later times cannot be rule out, further contributing to monocyte-derived cell activation and differentiation and to the generation of robust antitumor immune responses. In the microenvironment of SULT2B1b-tumors or PFM037-treated tumors higher numbers of monocyte-DCs were detected and a heterogenous population of monocyte-derived cells resembling inflammatory monocytes or activated macrophages, which expressed higher levels of transcripts associated to the antigen presentation (*H2-Ab, H2-Eb, H2-Aa, H2-DMa, H2DMb, Cd74 and Ciita*) and to T-cell recruitment (*Cxcl9* and *Cxcl10*)²⁶*.* Although these cells when pulsed with the SIINFEKL peptide, were much less stimulators than pulsed monocyte-DCs (Fig. 11C-E), it cannot be excluded that they contribute to the antitumor immune response by recruiting antitumor T cells and/or generating a stimulatory immune contexture. In conclusion, the invention indicates that SLTLT2B1b-derived products reprogram the lipidome of the tumor microenvironment and favor monocyte-derived cell activation and differentiation, including monocyte-DCs (Fig. 17), by interfering with LXR signaling. The characterization of a new compound, obtained by the chemical sulfation of the LXR-inactive oxysterol 22S-HC and promoting effective antitumor responses, may represent an important step towards the clinical translation of new compounds linking lipid metabolism and immunity, given alone or in combination with immunotherapy.

### References

1. Ribas A, Wolchok JD. Cancer immunotherapy using checkpoint blockade. Science (New York, NY 2018, 359(6382): 1350-1355.
2. Sharma P, Hu-Lieskovan S, Wargo JA, Ribas A. Primary, Adaptive, and Acquired Resistance to Cancer Immunotherapy. Cell 2017, 168(4): 707-723.
3. Wculek SK, Cueto FJ, Mujal AM, Melero I, Krummel MF, Sancho D. Dendritic cells in cancer immunology and immunotherapy. Nat Rev Immunol 2020, 20(1): 7-24.
4. Jhunjhunwala S, Hammer C, Delamarre L. Antigen presentation in cancer: insights into tumour immunogenicity and immune evasion. Nature reviews 2021, 21(5): 298-312.
5. Brendolan A, Russo V. Targeting cholesterol homeostasis in hematopoietic malignancies. Blood 2022, 139(2): 165-176.
6. Tyurin VA, Cao W, Tyurina YY, Gabrilovich DI, Kagan VE. Mass-spectrometric characterization of peroxidized and hydrolyzed lipids in plasma and dendritic cells of tumor-bearing animals. Biochem Biophys Res Commun 2011, 413(1): 149-153.
7. Villablanca EJ, Raccosta L, Zhou D, Fontana R, Maggioni D, Negro A, et al. Tumor-mediated liver X receptor-alpha activation inhibits CC chemokine receptor-7 expression on dendritic cells and dampens antitumor responses. Nature medicine 2010, 16(1): 98-105.
8. Fuda H, Javitt NB, Mitamura K, Ikegawa S, Strott CA. Oxysterols are substrates for cholesterol sulfotransferase. J Lipid Res 2007, 48(6): 1343-1352.
9. Repa JJ, Mangelsdorf DJ. The role of orphan nuclear receptors in the regulation of cholesterol homeostasis. Annu Rev Cell Dev Biol 2000, 16: 459-481.
10. Janowski BA, Willy PJ, Devi TR, Falck JR, Mangelsdorf DJ. An oxysterol signalling pathway mediated by the nuclear receptor LXR alpha. Nature 1996, 383(6602): 728-731.
11. Chen W, Chen G, Head DL, Mangelsdorf DJ, Russell DW. Enzymatic reduction of oxysterols impairs LXR signaling in cultured cells and the livers of mice. Cell Metab 2007, 5(1): 73-79.
12. Bensinger SJ, Bradley MN, Joseph SB, Zelcer N, Janssen EM, Hausner MA, et al. LXR signaling couples sterol metabolism to proliferation in the acquired immune response. Cell 2008, 134b(1): 97-111.
13. Wang F, Beck-Garcia K, Zorzin C, Schamel WW, Davis MM. Inhibition of T cell receptor signaling by cholesterol sulfate, a naturally occurring derivative of membrane cholesterol. Nature immunology 2016, 17(7): 844-850.
14. Raccosta L, Fontana R, Maggioni D, Lanterna C, Villablanca EJ, Paniccia A, et al. The oxysterol-CXCR2 axis plays a key role in the recruitment of tumor-promoting neutrophils. J Exp Med 2013, 210(9): 1711-1728.
15. Shi C, Pamer EG. Monocyte recruitment during infection and inflammation. Nat Rev Immunol 2011, 11(11): 762-774.
16. Jakubzick CV, Randolph GJ, Henson PM. Monocyte differentiation and antigen-presenting functions. Nat Rev Immunol 2017, 17(6): 349-362.
17. Noy R, Pollard JW. Tumor-associated macrophages: from mechanisms to therapy. Immunity 2014, 41(1): 49-61.
18. Ma Y, Adjemian S, Mattarollo SR, Yamazaki T, Aymeric L, Yang H, et al. Anticancer chemotherapy-induced intratumoral recruitment and differentiation of antigen-presenting cells. Immunity 2013, 38(4): 729-741.
19. Sharma MD, Rodriguez PC, Koehn BH, Baban B, Cui Y, Guo G, et al. Activation of p53 in Immature Myeloid Precursor Cells Controls Differentiation into Ly6c(+)CD103(+) Monocytic Antigen-Presenting Cells in Tumors. Immunity 2018, 48(1): 91-106 e106.
20. Serhan CN, Chiang N, Dalli J, Levy BD. Lipid mediators in the resolution of inflammation. Cold Spring Harb Perspect Biol 2014, 7(2): a016311.
21. Broz ML, Binnewies M, Boldajipour B, Nelson AE, Pollack JL, Erle DJ, et al. Dissecting the tumor myeloid compartment reveals rare activating antigen-presenting cells critical for T cell immunity. Cancer cell 2014, 26(5): 638-652.
22. Gajewski TF, Corrales L, Williams J, Horton B, Sivan A, Spranger S. Cancer Immunotherapy Targets Based on Understanding the T Cell-Inflamed Versus Non-T Cell-Inflamed Tumor Microenvironment. Advances in experimental medicine and biology 2017, 1036: 19-31.
23. Menezes S, Melandri D, Anselmi G, Perchet T, Loschko J, Dubrot J, et al. The Heterogeneity of Ly6C(hi) Monocytes Controls Their Differentiation into iNOS(+) Macrophages or Monocyte-Derived Dendritic Cells. Immunity 2016, 45(6): 1205-1218.
24. Bosteels C, Neyt K, Vanheerswynghels M, van Helden MJ, Sichien D, Debeuf N, et al. Inflammatory Type 2 cDCs Acquire Features of cDC1s and Macrophages to Orchestrate Immunity to Respiratory Virus Infection. Immunity 2020, 52(6): 1039-1056 e1039.
25. Marigo I, Zilio S, Desantis G, Mlecnik B, Agnellini AH, Ugel S, et al. T Cell Cancer Therapy Requires CD40-CD40L Activation of Tumor Necrosis Factor and Inducible Nitric-Oxide-Synthase-Producing Dendritic Cells. Cancer cell 2016, 30(4): 651.
26. Spranger S, Dai D, Horton B, Gajewski TF. Tumor-Residing Batf3 Dendritic Cells Are Required for Effector T Cell Trafficking and Adoptive T Cell Therapy. Cancer cell 2017, 31(5): 711-723 e714.
27. Marigo I, Bosio E, Solito S, Mesa C, Fernandez A, Dolcetti L, et al. Tumor-induced tolerance and immune suppression depend on the C/EBPbeta transcription factor. Immunity 2010, 32(6): 790-802.
28. Briseno CG, Haldar M, Kretzer NM, Wu X, Theisen DJ, Kc W, et al. Distinct Transcriptional Programs Control Cross-Priming in Classical and Monocyte-Derived Dendritic Cells. Cell Rep 2016, 15(11): 2462-2474.
29. Tamoutounour S, Guilliams M, Montanana Sanchis F, Liu H, Terhorst D, Malosse C, et al. Origins and functional specialization of macrophages and of conventional and monocyte-derived dendritic cells in mouse skin. Immunity 2013, 39(5): 925-938.
30. Gosselin D, Link VM, Romanoski CE, Fonseca GJ, Eichenfield DZ, Spann NJ, et al. Environment drives selection and function of enhancers controlling tissue-specific macrophage identities. Cell 2014, 159(6): 1327-1340.
31. Lavin Y, Winter D, Blecher-Gonen R, David E, Keren-Shaul H, Merad M, et al. Tissue-resident macrophage enhancer landscapes are shaped by the local microenvironment. Cell 2014, 159(6): 1312-1326.
32. Restivo G, Diener J, Cheng PF, Kiowski G, Bonalli M, Biedermann T, et al. Publisher Correction: The low affinity neurotrophin receptor CD271 regulates phenotype switching in melanoma. Nat Commun 2018, 9(1): 314.
33. Calkin AC, Tontonoz P. Transcriptional integration of metabolism by the nuclear sterol-activated receptors LXR and FXR. Nat Rev Mol Cell Biol 2012, 13(4): 213-224.
34. Bruhl H, Cihak J, Plachy J, Kunz-Schughart L, Niedermeier M, Denzel A, et al. Targeting of Gr-1+,CCR2+ monocytes in collagen-induced arthritis. Arthritis Rheum 2007, 56(9): 2975-2985.
35. Tsou CL, Peters W, Si Y, Slaymaker S, Aslanian AM, Weisberg SP, et al. Critical roles for CCR2 and MCP-3 in monocyte mobilization from bone marrow and recruitment to inflammatory sites. J Clin Invest 2007, 117(4): 902-909.
36. Ruhland MK, Roberts EW, Cai E, Mujal AM, Marchuk K, Beppler C, et al. Visualizing Synaptic Transfer of Tumor Antigens among Dendritic Cells. Cancer cell 2020, 37(6): 786-799 e785.
37. Talamini L, Picchetti P, Ferreira LM, Sitia G, Russo L, Violatto MB, et al. Organosilica Cages Target Hepatic Sinusoidal Endothelial Cells Avoiding Macrophage Filtering. ACS Nano 2021, 15(6): 9701-9716.
38. Ngoc Lan Tran GS. Continuous sensing of IFNα by hepatic endothelial cells shapes a vascular antimetastatic barrier. https://doiorg/101101/20220510491298*.*
39. Caira F, Antonson P, Pelto-Huikko M, Treuter E, Gustafsson JA. Cloning and characterization of RAP250, a novel nuclear receptor coactivator. The Journal of biological chemistry 2000, 275(8): 5308-5317.
40. Antonson P, Jakobsson T, Almlof T, Guldevall K, Steffensen KR, Gustafsson JA. RAP250 is a coactivator in the transforming growth factor beta signaling pathway that interacts with Smad2 and Smad3. The Journal of biological chemistry 2008, 283(14): 8995-9001.
41. Glunde K, Bhujwalla ZM, Ronen SM. Choline metabolism in malignant transformation. Nature reviews 2011, 11(12): 835-848.
42. Sarenac TM, Mikov M. Bile Acid Synthesis: From Nature to the Chemical Modification and Synthesis and Their Applications as Drugs and Nutrients. Front Pharmacol 2018, 9: 939.
43. Lambrechts D, Wauters E, Boeckx B, Aibar S, Nittner D, Burton O, et al. Phenotype molding of stromal cells in the lung tumor microenvironment. Nature medicine 2018, 24(8): 1277-1289.
44. Sade-Feldman M, Yizhak K, Bjorgaard SL, Ray JP, de Boer CG, Jenkins RW, et al. Defining T Cell States Associated with Response to Checkpoint Immunotherapy in Melanoma. Cell 2018, 175(4): 998-1013 e1020.
45. Gabrilovich D. Mechanisms and functional significance of tumour-induced dendritic-cell defects. Nat Rev Immunol 2004, 4(12): 941-952.
46. Yost KE, Satpathy AT, Wells DK, Qi Y, Wang C, Kageyama R, et al. Clonal replacement of tumor-specific T cells following PD-1 blockade. Nature medicine 2019, 25(8): 1251-1259.
47. Ferris ST, Durai V, Wu R, Theisen DJ, Ward JP, Bern MD, et al. cDC1 prime and are licensed by CD4(+) T cells to induce anti-tumour immunity. Nature 2020, 584(7822): 624-629.
48. Devalaraja S, To TKJ, Folkert IW, Natesan R, Alam MZ, Li M, et al. Tumor-Derived Retinoic Acid Regulates Intratumoral Monocyte Differentiation to Promote Immune Suppression. Cell 2020, 180(6): 1098-1114 e1016.
49. Schuler G. Dendritic cells: indispensable? Cancer J 2011, 17(5): 337-342.
50. Steinman RM, Banchereau J. Taking dendritic cells into medicine. Nature 2007, 449(7161): 419-426.
51. Carreno BM, Magrini V, Becker-Hapak M, Kaabinejadian S, Hundal J, Petti AA, et al. Cancer immunotherapy. A dendritic cell vaccine increases the breadth and diversity of melanoma neoantigen-specific T cells. Science (New York, NY 2015, 348(6236): 803-808.
52. June CH, O'Connor RS, Kawalekar OU, Ghassemi S, Milone MC. CAR T cell immunotherapy for human cancer. Science (New York, NY 2018, 359(6382): 1361-1365.
53. Hicks KC, Tyurina YY, Kagan VE, Gabrilovich DI. Myeloid Cell-Derived Oxidized Lipids and Regulation of the Tumor Microenvironment. Cancer Res 2022, 82(2): 187-194.
54. Flaveny CA, Griffett K, El-Gendy Bel D, Kazantzis M, Sengupta M, Amelio AL, et al. Broad Anti-tumor Activity of a Small Molecule that Selectively Targets the Warburg Effect and Lipogenesis. Cancer cell 2015, 28(1): 42-56.
55. Carpenter KJ, Valfort AC, Steinauer N, Chatterjee A, Abuirqeba S, Majidi S, et al. LXR-inverse agonism stimulates immune-mediated tumor destruction by enhancing CD8 T-cell activity in triple negative breast cancer. Sci Rep 2019, 9(1): 19530.
56. Tavazoie MF, Pollack I, Tanqueco R, Ostendorf BN, Reis BS, Gonsalves FC, et al. LXR/ApoE Activation Restricts Innate Immune Suppression in Cancer. Cell 2018, 172(4): 825-840 e818.
57. Carbo JM, Leon TE, Font-Diaz J, De la Rosa JV, Castrillo A, Picard FR, et al. Pharmacologic Activation of LXR Alters the Expression Profile of Tumor-Associated Macrophages and the Abundance of Regulatory T Cells in the Tumor Microenvironment. Cancer Res 2021, 81(4): 968-985.
58. Marinozzi M, Castro Navas FF, Maggioni D, Carosati E, Bocci G, Carloncelli M, et al. Side-Chain Modified Ergosterol and Stigmasterol Derivatives as Liver X Receptor Agonists. J Med Chem 2017, 60(15): 6548-6562.
59. Dang EV, Cyster JG. Loss of sterol metabolic homeostasis triggers inflammasomes - how and why. Curr Opin Immunol 2018, 56: 1-9.
60. Ito A, Hong C, Oka K, Salazar JV, Diehl C, Witztum JL, et al. Cholesterol Accumulation in CD11c(+) Immune Cells Is a Causal and Targetable Factor in Autoimmune Disease. Immunity 2016, 45(6): 1311-1326.
61. Castro Navas FF, Giorgi G, Maggioni D, Pacciarini M, Russo V, Marinozzi M. C24-hydroxylated stigmastane derivatives as Liver X Receptor agonists. Chem Phys Lipids 2018, 212: 44-50.
62. Sitia G, Aiolfi R, Di Lucia P, Mainetti M, Fiocchi A, Mingozzi F, et al. Antiplatelet therapy prevents hepatocellular carcinoma and improves survival in a mouse model of chronic hepatitis B. Proceedings of the National Academy of Sciences of the United States of America 2012, 109(32): E2165-2172.
63. Dobin A, Davis CA, Schlesinger F, Drenkow J, Zaleski C, Jha S, et al. STAR: ultrafast universal RNA-seq aligner. Bioinformatics 2013, 29(1): 15-21.
64. Barnett DW, Garrison EK, Quinlan AR, Stromberg MP, Marth GT. BamTools: a C++ API and toolkit for analyzing and managing BAM files. Bioinformatics 2011, 27(12): 1691-1692.
65. Liao Y, Smyth GK, Shi W. The R package Rsubread is easier, faster, cheaper and better for alignment and quantification of RNA sequencing reads. Nucleic Acids Res 2019.
66. Ritchie ME, Phipson B, Wu D, Hu Y, Law CW, Shi W, et al. limma powers differential expression analyses for RNA-sequencing and microarray studies. Nucleic Acids Res 2015, 43(7): e47.
67. Consortium SM-I. A comprehensive assessment of RNA-seq accuracy, reproducibility and information content by the Sequencing Quality Control Consortium. Nat Biotechnol 2014, 32(9): 903-914.
68. Subramanian A, Tamayo P, Mootha VK, Mukherjee S, Ebert BL, Gillette MA, et al. Gene set enrichment analysis: a knowledge-based approach for interpreting genome-wide expression profiles. Proceedings of the National Academy of Sciences of the United States of America 2005, 102(43): 15545-15550.
69. Huber W, Carey VJ, Gentleman R, Anders S, Carlson M, Carvalho BS, et al. Orchestrating high-throughput genomic analysis with Bioconductor. Nat Methods 2015, 12(2): 115-121.
70. Rohart F, Gautier B, Singh A, Le Cao KA. mixOmics: An R package for 'omics feature selection and multiple data integration. PLoS Comput Biol 2017, 13(11): e1005752.
71. Stuart T, Butler A, Hoffman P, Hafemeister C, Papalexi E, Mauck WM, 3rd, et al. Comprehensive Integration of Single-Cell Data. Cell 2019, 177(7): 1888-1902 e1821.
72. Xia J, Sinelnikov IV, Han B, Wishart DS. MetaboAnalyst 3.0--making metabolomics more meaningful. Nucleic Acids Res 2015, 43(W1): W251-257.
73. Wishart DS, Tzur D, Knox C, Eisner R, Guo AC, Young N, et al. HMDB: the Human Metabolome Database. Nucleic Acids Res 2007, 35(Database issue): D521-526.
74. Lanterna C, Musumeci A, Raccosta L, Corna G, Moresco M, Maggioni D, et al. The administration of drugs inhibiting cholesterol/oxysterol synthesis is safe and increases the efficacy of immunotherapeutic regimens in tumor-bearing mice. Cancer Immunol Immunother 2016, 65(11): 1303-1315.

## Claims

1. A compound of formula (I) : 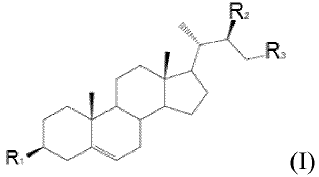 Wherein:
R₁ is OH or OSO₃X;
R₂ is OH or OSO₃X;
X at each occurrence is independently selected from H⁺, Na⁺, K⁺, NH₄⁺ , pyridinium; and wherein when R₁ is OSO₃X then R₃ is CH₂CH(CH₃)₂, and
when R₁ is OH then R₃ is an aryl or heteroaryl ring each of said aryl or heteroaryl ring being optionally substituted with one or more substituents independently selected from C₁₋₆alkyl, C₁₋₆alkoxy, halogen, C₁₋₆haloalkyl, C₁₋₆haloalkoxy, C₃₋₆cycloalkyl, OH, halogen, NH₂, NH-C₁₋₆alkyl, N(C₁₋₆alkyl)₂, NH-C(=O)C₁₋₃alkyl.

2. The compound of formula (I) according to claim 1 being selected from:

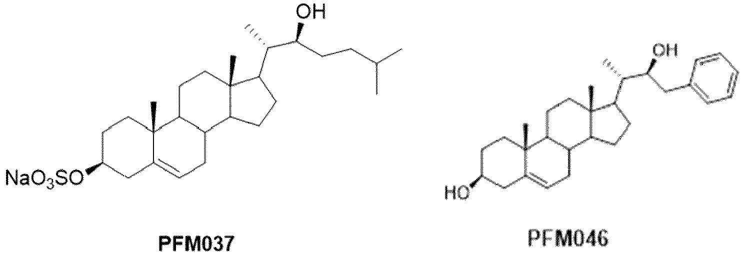

3. The compound of formula (I) according to any one of claims 1 or 2 for use as Liver X Receptor (LXR) antagonist.

4. The compound of formula (I) according to any one of claims 1 to 3 for medical use.

5. The compound of formula (I) according to any one of claims 1 to 3 for use in the treatment and/or prevention of diseases linked or associated to LXR, preferably for use in the treatment and/or prevention of cancer, inflammatory and/or immunological diseases, preferably for use in the treatment of melanoma, skin carcinoma, Hodgkin lymphoma, renal, lung, bladder and head and neck cancers.

6. The compound of formula (I) for use according to claim 5, in combination with at least one further therapeutic agent, preferably said at least one further therapeutic agent is selected from the group consisting of LXR agonists, chemotherapeutics, or in combination with immunotherapies, such as immune checkpoint inhibitors (ICIs), preferably PD1 inhibitors or PDL1 inhibitors, or such as adoptive cell transfer therapy, preferably adoptive T-cell transfer therapy or NK-cell transfer therapy.

7. The compound of formula (I) for use according to claim 5, wherein the LXR agonist is selected from the group comprising the following compounds:

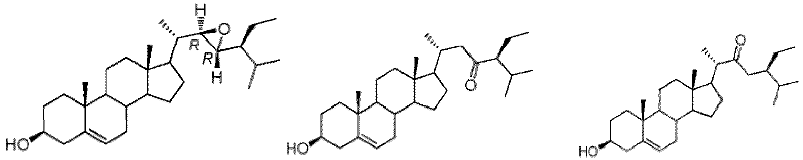

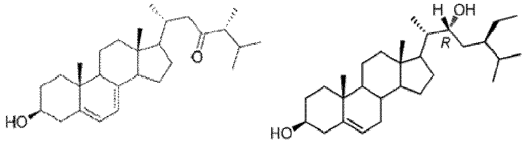

8. A pharmaceutical composition comprising a compound of formula (I) according to any one of claims 1 to 3, or combinations thereof, alone or in combination with at least one further therapeutic agent, and at least a pharmaceutically acceptable excipient.

9. The pharmaceutical composition according to claim 8 wherein said further therapeutic agent is an LXR agonist, preferably wherein said LXR agonist is selected from:

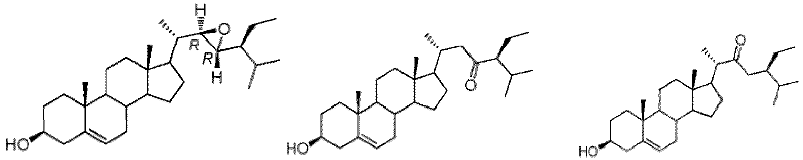

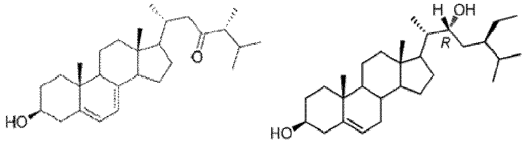

10. The pharmaceutical composition of claims 8 or 9 for medical use, preferably for use in the treatment and or prevention of diseases linked or associated to LXR, preferably for use in the treatment and/or prevention of cancer, inflammatory and/or immunological diseases, preferably for use in the treatment of melanoma, skin carcinoma, Hodgkin lymphoma, renal, lung, bladder and head and neck cancers.

11. A method for obtaining a T cell or a T cell population expressing at least one marker associated to T central memory and/or T stem cell memory comprising the steps of:
a) isolating a T cell or a T cell population from a biological sample of a subject;
b) activating said T cell or said T cell population, preferably by stimulating CD3 and CD28;
c) contacting said activated T cell or T cell population with IL-7 and IL-15 in the presence of a compound of formula (I) as defined in any one of claims 1 to 3 to obtain a T cell or a T cell population expressing at least one marker associated to T central memory and/or T stem cell memory.

12. The method according to claim 11 wherein the the stimulation of CD3 and CD28 is carried out by a CD3 agonist and a CD28 agonist, preferably the stimulation is carried out by an antibody specific for CD3 and an antibody specific for CD28.

13. The method of claim 11 or 12, wherein the compound of formula (I) in step c) is PFM037 and/or PFM046.

14. A T cell or T cell population obtainable by the method of any one of claims 11-13.

15. Use of a compound of formula (I) according to any one of claims 1 to 3 for augmenting T cell or NK cell memory differentiation *in vitro* and/or to decrease T cell or NK cell exhaustion *ex vivo* and/or to *in vitro or ex vivo* obtain a T or NK cell with a phenotype associated to T central memory and/or T Stem Cell Memory (TSCM) and/or with antitumor potential, preferably for adoptive cell transfer purposes.
